# EUROPEAN PATENT SPECIFICATION

(11) **EP 2 656 879 B1**
(45) Date of publication and mention of the grant of the patent: **28.06.2017**
(21) Application number: 13161970.2
(22) Date of filing: 10.12.2006
(51) Int. Cl.: C07D 499/00, A61K 31/43, A61K 31/545, A61P 31/04

(54) **Transdermal delivery systems of beta-lactam antibiotics**
Transdermale Abgabesysteme von Beta-Lactamantibiotika
Systèmes d'administration transdermique d'antibiotiques de beta-lactame

(43) Date of publication of application: 30.10.2013
(62) Divisional of application: 06832186.8
(73) Proprietor: Yu, Chongxi, E-7, Kensington, MD 20895 (US)
(72) Inventor: Yu, Chongxi, E-7, Kensington, MD 20895 (US)
(74) Representative: Finnegan Europe LLP

(56) References cited:
- DE-C- 858 699
- FR-A1- 2 132 447
- US-A- 2 694 031
- US-A- 2 694 063
- US-A- 3 957 764
- US-A- 5 321 020
- US-B1- 6 191 143
- "chap.46: antimicrobial agents" In: GOODMAN GILMAN A ET AL: "The Pharmacological Basis of Therapeutics, 8th ed", 1 January 1991 (1991-01-01), 19910101, XP008110226, pages 1065-1097, * the whole document *
- HATANAKA T ET AL: "Ion pair skin transport of a zwitterionic drug, cephalexin", JOURNAL OF CONTROLLED RELEASE, ELSEVIER, AMSTERDAM, NL, vol. 66, no. 1, 1 May 2000 (2000-05-01), pages 63-71, XP004202707, ISSN: 0168-3659
- J. BAGYALAKSHMI, AS WILLIAM, AW MITHUN, TK. RAVI, R.MANAVALAN, PK. MANNA: "pharmacodynamics of ampicillin sodium transdermal patches in an in vitro infection model", INDIAN JOURNAL OF PHARMACEUTICAL SCIENCES, 20 April 2006 (2006-04-20), pages 540-541, XP002569979, Retrieved from the Internet: URL:http://www.ijpsonline.com/text.asp?200 6/68/4/540/27843 [retrieved on 2010-02-23]
- JANSEN A B A ET AL: "SOME NOVEL PENICILLIN DERIVATIVES", JOURNAL OF THE CHEMICAL SOCIETY, CHEMICAL SOCIETY, LETCHWORTH; GB, vol. 65, 1 March 1965 (1965-03-01), pages 2127-2132, XP009051067, ISSN: 0368-1769, DOI: 10.1039/JR9650002127
- DAVID A. JOHNSON: JOURNAL OF THE AMERICAN CHEMICAL SOCIETY, vol. 75, no. 15, 5 August 1953 (1953-08-05), pages 3636-3637, XP055080675, ISSN: 0002-7863, DOI: 10.1021/ja01111a009
- RIE TANAKA ET AL: "Structure-activity relationships of penem antibiotics: Crystallographic structures and implications for their antimicrobial activities", BIOORGANIC & MEDICINAL CHEMISTRY, vol. 5, no. 7, 1 July 1997 (1997-07-01), pages 1389-1399, XP055080673, ISSN: 0968-0896, DOI: 10.1016/S0968-0896(97)00073-4
- ILANKUMARAN PALANICHAMY ET AL: "Prop-2-ynyl as a protective group for carboxylic acids: a mild method for the highly selective deprotection of prop-2-ynyl esters using tetrahiomolybdate", JOURNAL OF THE CHEMICAL SOCIETY, CHEMICAL COMMUNICATIONS, CHEMICAL SOCIETY. LETCHWORTH, GB, vol. 16, 1 January 1996 (1996-01-01), pages 1957-1958, XP009146007, ISSN: 0022-4936

## Description

### Technical Field

The present invention relates to the preparations of positively charged and watersoluble pro-drugs of beta-lactam antibiotics and related compounds and their medicinal use in treating any beta-lactam antibiotics and related compounds-treatable conditions in humans or animals. More specifically, the present invention is to enable quick bacterial cell wall and human skin penetration of beta-lactam antibiotics and related compounds.

### Background Art

A beta-lactam is a cyclic amide with four atoms in its ring and the chemical name for this ring system is azetidinone. Since French medical student Ernest Duchesne initially discovered penicillin in 1896 and Alexander Fleming rediscovered it in 1929 along with the subsequent purification of penicillin from it in the late 1930s and early 1940s by Florey, Chain, Abraham, and Heatley, over a hundred thousand of betalactam antibiotics have been prepared by partial or total chemical synthesis (L.A. Mitscher, et al., Antibiotic and Antimicrobial Drugs, in D.F. Smith, Ed., Handbook of Stereoisomers: Therapeutic Drugs, Boca Raton, FL, CRC Press, 1989; R.B. Morin and M. Gorman Eds., Chemistry and Biology of Beta-lactam Antibiotics, Volumes 1-3, New York, Academic Press, 1982; and A.L.Demain and N.A. Solomon, Eds., Antibiotics Containing the Beta-lactam Structure, Vols, 1 and 2, Handbook of experimental Phgarmacology, vol. 67, New York, Springer, 1983).

When penicillin became widely available during the second world war, it was a medical miracle, rapidly vanquishing the biggest wartime killer-infected wounds. Penicillin killed many types of disease-causing bacteria. But just four years after drug companies began mass-producing penicillin in 1943, microbes began appearing that could resist it. Antibiotic resistance spreads fast. Between 1979 and 1987, for example, only 0.02 % of pneumococcus strains infecting a large number of patients surveyed by the national Centers for Disease Control and Prevention were penicillin-resistant. Only 7 years late , 6.6 percent of pneumococcus strains are resistant, according to a report in the June 15, 1994, Journal of the American Medical Association by Robert F. Breiman, M.D., and colleagues at CDC (Ricki Lewis, U.S. Food and Drug Administration Home Page). Antimicrobial resistance is driving up health care costs, increasing the severity of disease, and increasing the death rates from certain infections. According to CDC statistics, nearly 2 million patients in the United States get an infection in the hospital each year and about 90,000 of those patients die each year as a result of their infection, up from 13,300 patient deaths in 1992. More than 70 percent of the bacteria that cause hospital-acquired infections are resistant to at least one of the antibiotics most commonly used to treat them. The increased prevalence of antibiotic resistance is an outcome of evolution, but overuse of antibiotics accelerates antibiotic resistance. To develop new antibiotics is a very urgent and challenging task.

Intravenous infusion, intramuscular injection, subcutaneous, buccal, oral, and rectal routes are the methods for administration of a wide variety of antimicrobial agents for the treatment of systemic bacterial diseases. Oral administration has disadvantage of poor absorption of the antibiotics from GI tract. The intravenous, subcutaneous and intramuscular routes are not only painful, but also must be performed by trained individuals and there is the risk of needle injury, infection, and other trauma. One alternative method of administering drugs is topical delivery. Topical drug delivery has several advantages. This method helps avoid inactivation of a drug caused by first pass metabolism in the liver and gastro-intestinal tract. It can provide local delivery of appropriate concentrations of a drug to the intended site of action without systemic exposure. Fishman (Fishman; Robert, U.S. Pat. No. 7,052,715) indicated that an additional problem associated with oral medications, is that the concentration levels which must be achieved in the bloodstream must be significant in order to effectively treat distal areas of pain, inflammation, or infection. These levels are often much higher than would be necessary if it were possible to accurately target the particular site of pain or injury. Watts, et al. (U.S. Pat. Nos. 6,191,143 and 5,929,086) have described topical administration of some antimicrobial agents that have special structure characteristics (we noticed that all these chemicals have one positive charge and a lipophilic portion). For most of antibiotics, topical administration cannot deliver an effective therapeutic level. Susan Milosovich, et al. designed and prepared testosteronyl-4-dimethylaminobutyrate.HCl (TSBH), which has a lipophilic portion and a tertiary amine group that exists in the protonated form at physiological pH. They found that the prodrug (TSBH) diffuses through human skin -60 times faster than does the drug (TS) itself [Susan Milosovich, et al., J. Pharm. Sci., 82, 227(1993). US2694063 discloses quaternary ammonium addition salts of penicillin esters. US 2694031 discloses hydroiodides of amino esters of penicillin.

### Disclosure of Invention

### Technical Problem

The continued efficacy is threatened by microbial resistance caused by evolutionary pressures and overuse. Antibiotic resistance spreads fast. More people are contracting infections. People on chemotherapy and transplant recipients taking drugs to suppress their immune function are at greater risk of infection. The general aging of patients who live longer, get sicker, and die slower contributes to the problem.

Developing new antibiotics is a very urgent and challenging task.

Insufficient contact of the antibiotics with pathogenic bacteria at the site of infection is a major cause of meningitis, encephslitis, myelitis, abscess, mastitis, and prostatitis treatment failure. The reason of the treatment failure is that antibiotics cannot penetrate the blood-brain barrier, blood-milk barrier, and other biological barriers efficiently,

Most antibiotics are rapidly metabolized and inactivated by various pathways. When antibiotics are taken orally, the first pass metabolism, which refers to the chemical breakdown of compounds in the liver and gastro-intestinal tract, can destroy and inactivate a large portion of them. In the case of injection, administration of antibiotics is painful and in many cases requires frequent and costly office visits to treat chronic conditions. The blood and liver can also destroy and inactivate most antibiotics before they reach the intended site of action.

### Technical Solution

The present invention provides a compound of Structure 1 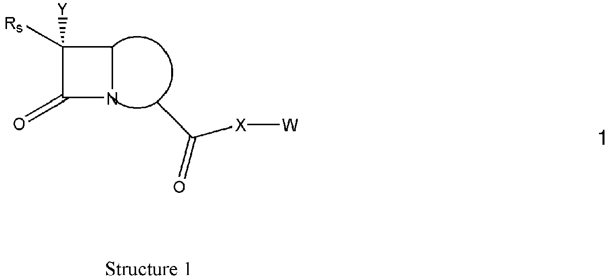 wherein
X is selected from O, S, CH₂, and NH;
Y is selected from H, OH, NHCHO, OCOCH₃, OCH₃, OC₂H₅, CH₃SO₃, NO₂, CN, CF₃, OCF₃, F, Br, I, and CI; 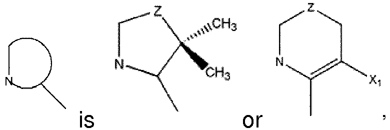
wherein
Z is S;
-X₁ is selected from -H, -OH, -OCH₃, -OC₂H₅, OC₃H₇, -CONH₂, -CH₂OCONH₂, - CH₂OCOCH₃, -OCOCH₃, -CH₂OCOCH₃, -CH₂OCH₃, -CH₃, -C₂H₅, -C₃H₇, -C₄H₉, C₅H₁₁, C₆H₁₃, - Cl, -F, -Br, -I, -HC=CHCH₃, -HC=CH₂, -CH₂OCH₃, 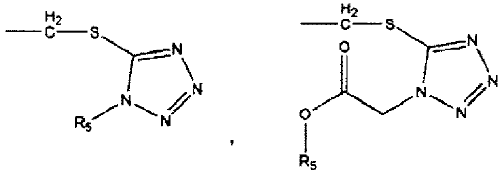
wherein
R₅ is selected from H, CONH₂, CH₂CH₂N(CH₃)₂, CH₂CH₂N(CH₂CH₃)₂, Cl, F, Br, I, alkyl residues having 1 to 12 carbon atoms, alkyloxyl residues having 1 to 12 carbon atoms, alkenyl residues having up to 12 carbon atoms, and alkynyl residues having up to 12 carbon atoms;
Rₛ- is selected from: 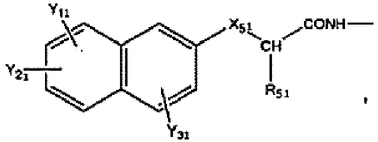 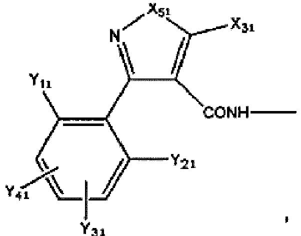 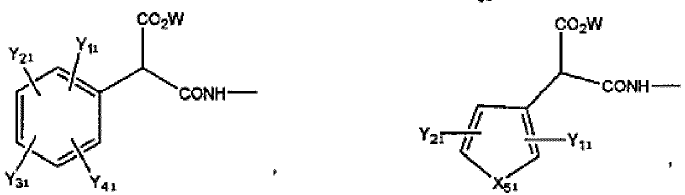 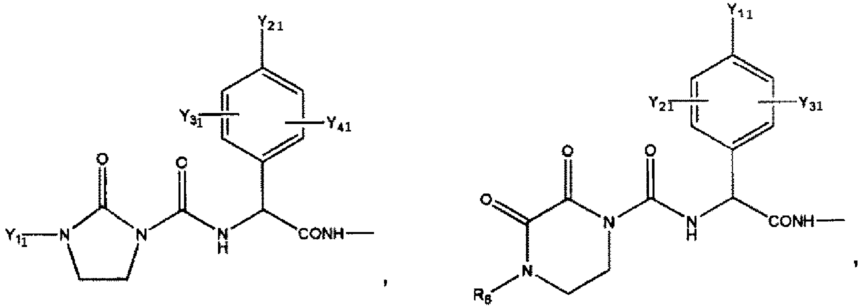 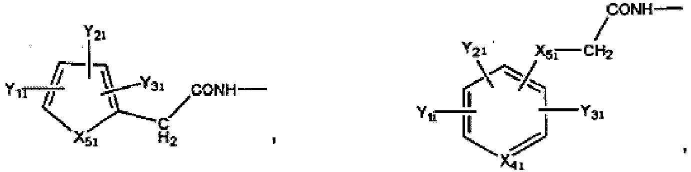 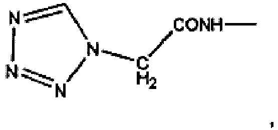 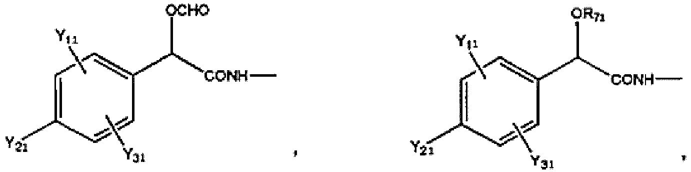 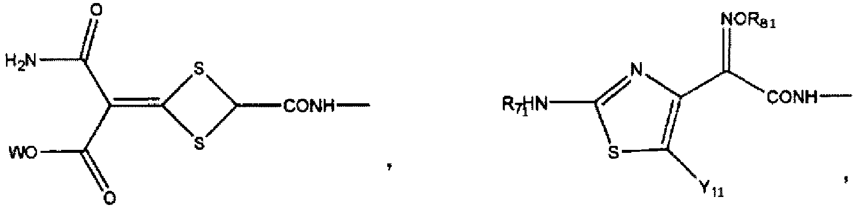 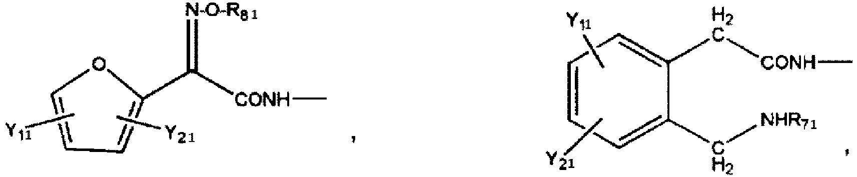 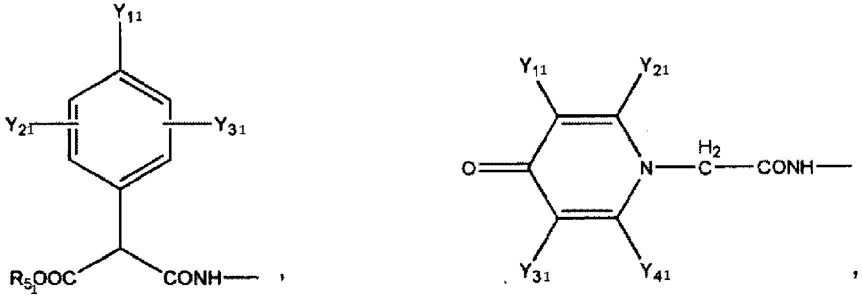 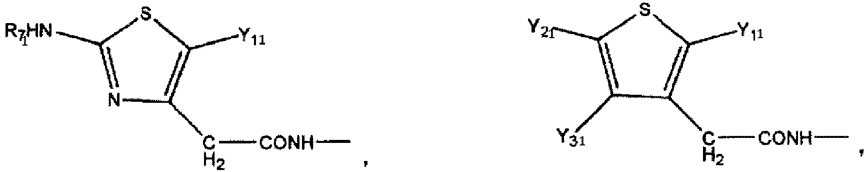 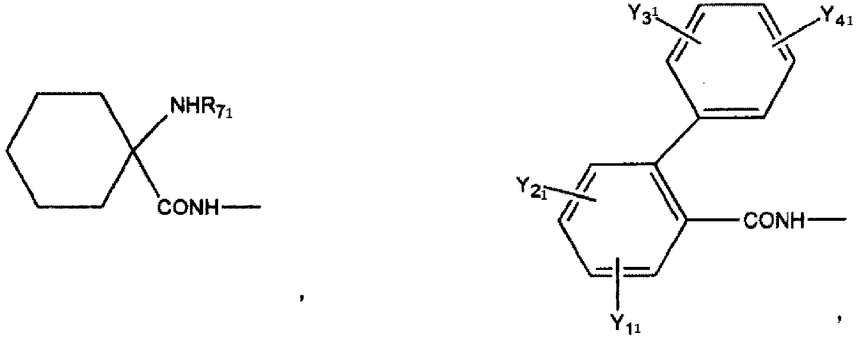 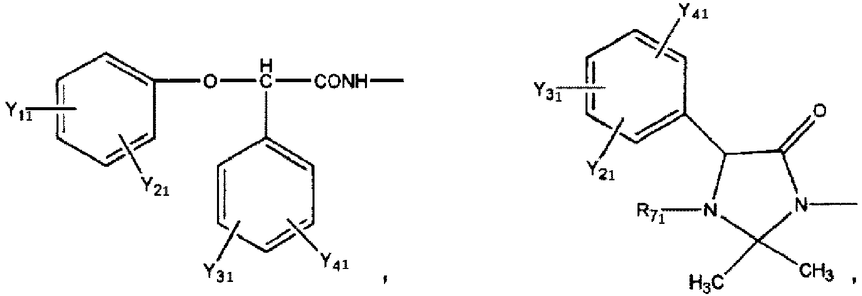 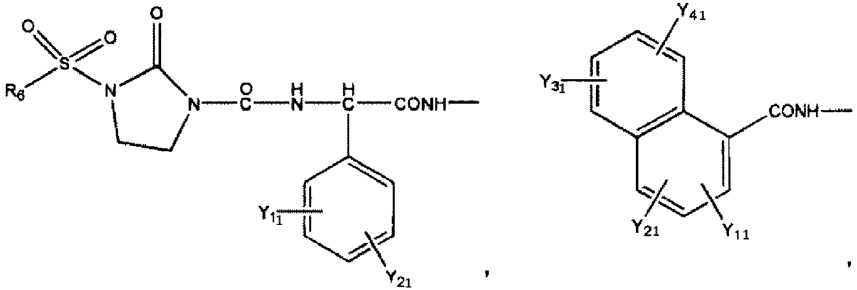 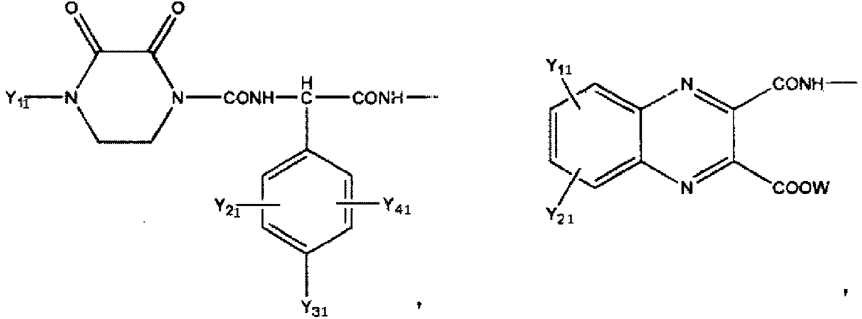 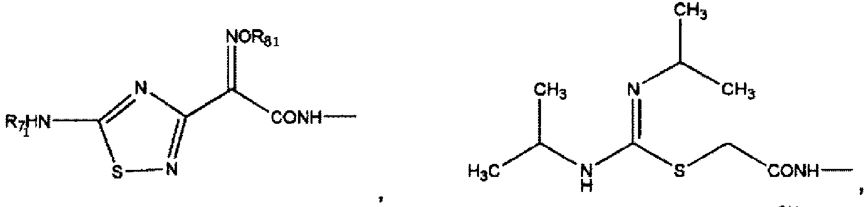 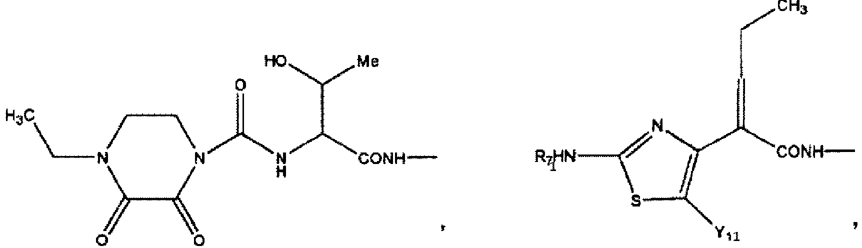 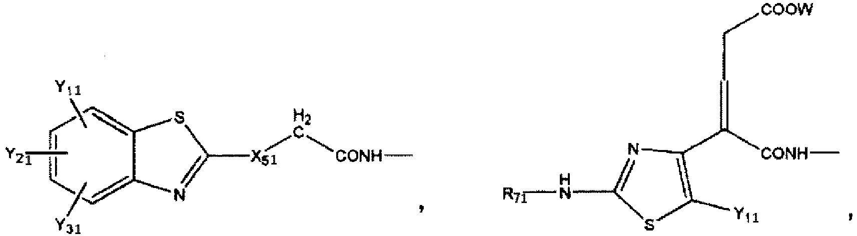 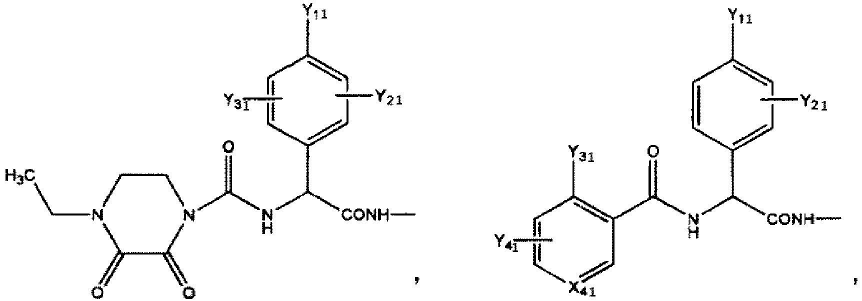 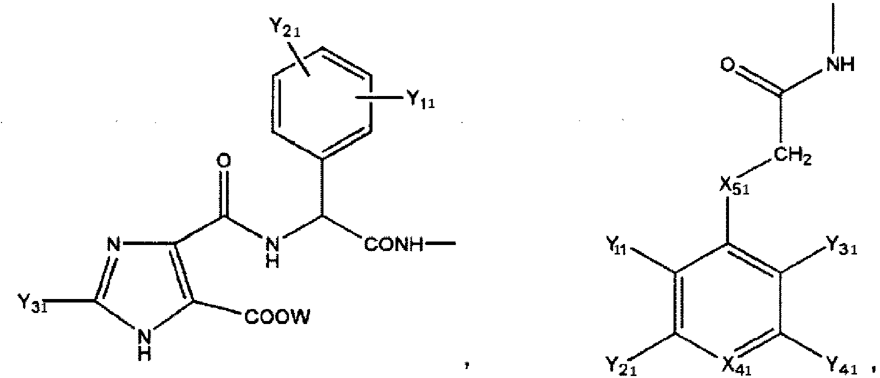 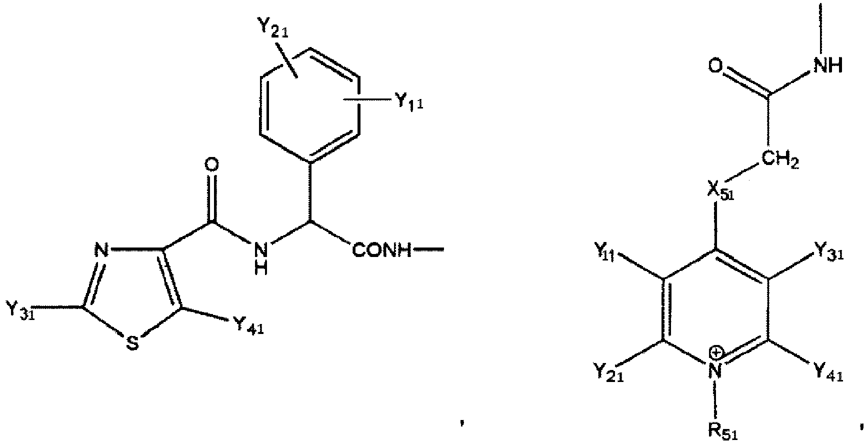 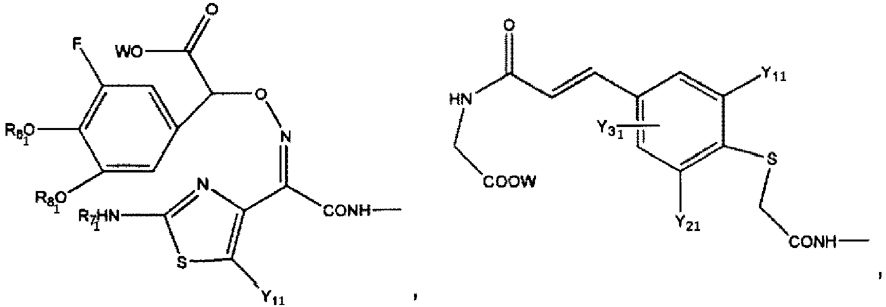 R₆₁OOCCH(NHR₇₁)(CH₂)ₙCONH-, R₆₁OOCCH(NHR₇)(CH₂)ₙSCONH-, CF₃SCH₂CONH-, CF₃CH₂CONH-, CHF₂SCH₂CONH- , CH₂FSCH₂CONH-, NH₂COCHFSCH₂CONH-, R₇₁NHCH(COOW), CH₂SCH₂CONH-, CNCH₂SCH₂CONH-, CH₃(CH₂)ₙCONH-, R₇₁N=C(NR₇₁)CH₂CH₂S-, R₇₁N=C(NHR₇₁)NHCO-, CH₃C(Cl)=CHCH₂SCH₂CONH-, (CH₃)₂C(OR₆₁)-, CNCH₂CONH-, CNCH₂CH₂S-, R₇₁N=C(NR₇₁)CH₂CH₂S-, CH₂=CHCH₂SCH₂CONH-, CH₃CH(OH)-, CH₃CH(OR₈₁)-, CH₃CH(Y₁₁)-, (CH₃)₂CH-, CH₃CH₂-, and CH₃(CH₂)ₙCH=CH(CH₂)ₘCONH-, or Rₛ and Y taken together are R₆₁OCH₂C(R₅₁)=, wherein
n and m are each independently selected from 0, 1, 2, 3, 4, 5, and 6;
X₃₁ is selected from H, N₃, SO₃W, F, Cl, Br, I, OH, OCH₃, OC₂H₅, CH₃, and C₂H₅;
X₄₁ is N or CH;
X₅₁ is selected from CH₂, S, O, and NH;
R₅₁ is selected from H, -CONH₂, CH₂CH₂N(CH₃)₂, CH₂CH₂N(CH₂CH₃)₂, CH₂CH₂OR₆₁, Cl,
   F, Br, I, alkyl residues having 1 to 12 carbon atoms, alkyloxyl residues having 1 to 12 carbon atoms, alkenyl residues having up to 12 carbon atoms, alkynyl residues having up to 12 carbon atoms, aryl residues, and heteroaryl residues;
R₆₁ is selected from H, F, Cl, Br, I, 2-oxo-1-imidazolidinyl, alkyl residues having 1 to 12 carbon atoms, alkyloxyl residues having 1 to 12 carbon atoms, alkenyl residues having up to 12 carbon atoms, alkynyl residues having up to 12 carbon atoms, aryl (phenyl, 5-indanyl, 2,3-dihydro-1H-inden-5-yl) residues and heteroaryl (4-hydroxy-1,5-naphthyridin-3-yl) residues;
R₇₁ is selected from H, F, Cl, Br, I, CH₃NHCOCH₂CH(NHR₈₁)CO, R₅₁N=C(NHR₆₁)NHCO, COCH₃, COR₆₁, PO(OR₅₁)OR₆₁, alkyl residues having 1 to 12 carbon atoms, alkyloxyl residues having 1 to 12 carbon atoms, alkenyl residues having 1 to 12 carbon atoms,alkynyl residues having 1 to 12 carbon atoms, aryl residues, and heteroaryl residues;
R₈₁ is selected from the group consisting of H, F, Cl, Br, I , CH₃, C₂H₅, CF₃, CH₂CH₂ F, CH₂CH₂Cl, CH₂CH₂Br, CH₂CH₂I, CH₂CHF₂, CH₂CF₃, CH₂F, CH₂Cl, CH₂Br, CH₂I, C₃H₇, C₄H₉, C₅H₁₁, CONH₂, and CH₂OCONH₂;
Y₁₁, Y₂₁, Y₃₁ and Y₄₁ are each independently selected from H, OH, OW, OCOW, OCOCH₃, CH₃, C₂H₅, C₃H₇, C₄H₉, SO₃R₆₁, CH₂COOR₈₁, OCH₃, OC₂H₅, CH₃SO₃, NO₂, CN, CF₃, OCF₃, CH=CHCONHCH₂COOW, CH₂(CH₂)ₙNR₅₁R₆₁, CH₂(CH₂)ₙOR₆₁, CH(CONH₂)NHR₆₁, COOR₅₁, F, Br, I, and Cl;
-W is selected from alkyl residues having 1 to 12 carbon atoms, alkyloxy residues having 1 to 12 carbon atoms, alkenyl residues having up to 12 carbon atoms, alkynyl residues having up to 12 carbon atoms, aryl residues, heteroaryl residues, 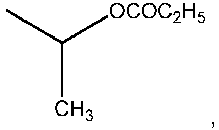 and 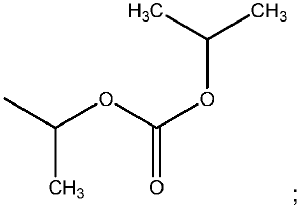
-W₁ is -RN⁺R₁R₂HA;
R₁, and R₂ are independently selected from alkyl residues having 1 to 12 carbon atoms, alkyloxyl residue having 1 to 12 carbon atoms, alkenyl residues having up to 12 carbon atoms, and alkynyl residues having up to 12 carbon atoms;
R is a branched or straight chain, -(CH₂)ₙ₁-;
n1 is selected from 0, 1, 2, 3, 4, 5, 6, 7, and 8; and
A⁻ is a negative ion selected from the group consisting of chloride, bromide, fluoride, acetate, and citrate.

Topical drug delivery for the treatment of local infections helps to avoid inactivation of a drug caused by first pass metabolism in the liver and gastro-intestinal tract and can provide local delivery of appropriate concentrations of a drug to the intended site of action without systemic exposure.

This invention relates to the designing and the preparation of novel positively charged pro-drugs ofbeta-lactam antibiotics and their medicinal use. The principles for designing these prodrugs ofbeta-lactam antibiotics are: 1. The prodrug must have a lipophilic portion and a primary, secondary, or tertiary amine group that exists in the protonated form (hydrophilic portion) at physiological pH. 2. Every prodrug of betalactam antibiotics should have only one or two (preferably one) primary, secondary, tertiary amine, or monoprotected quanidino groups that exists in the protonated form (hydrophilic portion) at physiological pH. 3. The primary, secondary, tertiary amine group, a quanidino, or monoprotected quanidino groups can be on any part of the compound. The design in which one end of the compound is the positive charge and other end is the lipophilic portion is preferable. 4. Carboxyl groups, amino groups, hydroxyl groups, guanidino groups and other hydrophilic groups can be protected with an alkyl, aryl, or heteroaryl ester or amide group to make the beta-lactam antibiotics more lipophilic.

One of beta-lactam antibiotics, Penethamate Hydriodide (Jensen et al., Ugeskrift for Laeger 112, 1043, 1075, 1950; Span. Pat. 206,653-5; Brit. Pat. 759,603), has the exact same structure characteristics of the novel prodrugs and show high penetration rate of milk-blood barrier. Surprisingly, nobody has tried to administer it transdermally.

The pro-drugs of beta-lactam antibiotics disclosed herein have the general formula (1) 'Structure 1'. 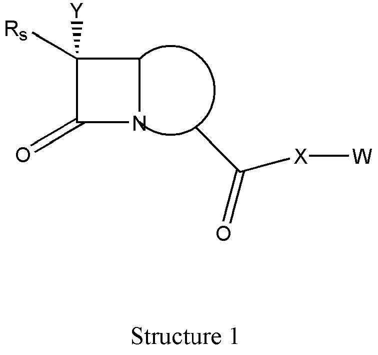 wherein, X represents O, S, or NH; Y represents H, OH, OR₅, NHCHO, CH₂CH₂ NHC(=NR₇)NHR₇, OCOCH₃, OCH₃, OC₂H₅, CH₃SO₃, NO₂, CN, CF₃, OCF₃, F, Br, I, or C1; 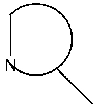 represents 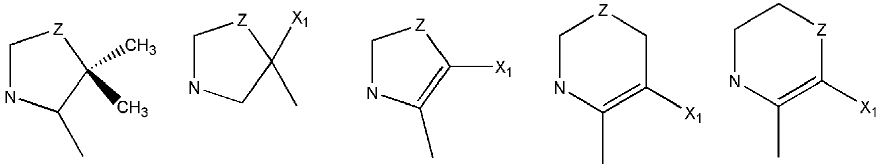 wherein, Z represents CH₂, S, SO, SO₂, NH, CHCH₃, CHCH₂CH₃, or O; -X₁ represents: -H, -OH, -OCH₃, -OC₂H₅, OC₃H₇, -CONH₂, -CH₂OCONH₂, -CH₂OCOCH₃ -OCOCH₃, -CH₂ OCOCH₃, -CH₂OCH₃, -CH₃, -C₂H_{5,} -C₃H₇ -C₄H₉, C₅H₁₁,-C₆H₁₃,-Cl,-F,-Br,-I,-HC=CHCH₃, -HC=CH₂, -CH₂OCH₃, -S(CH₂)ₙ -NHR₇, 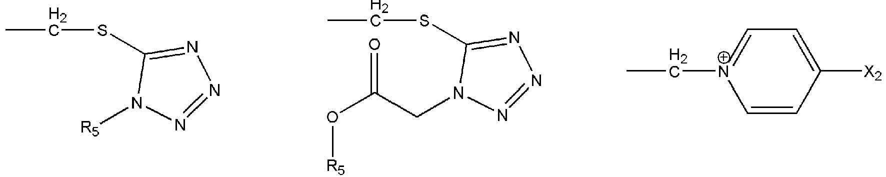 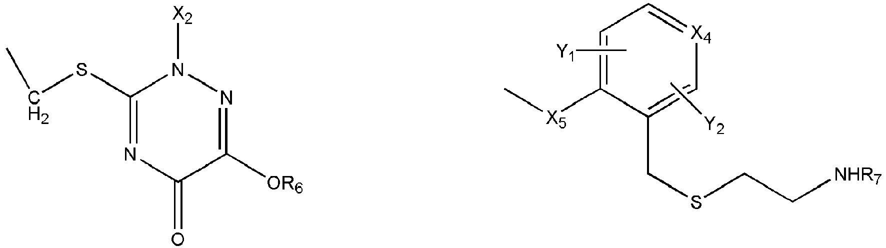 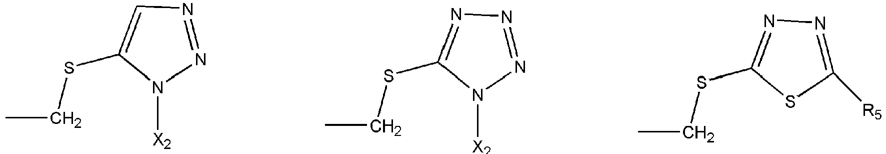 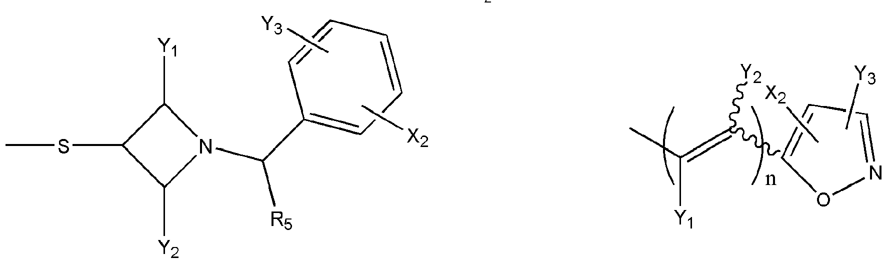 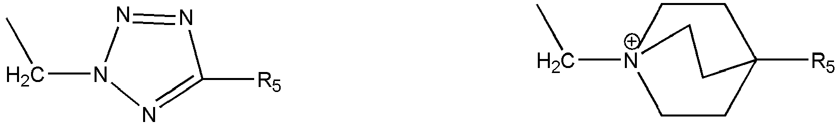 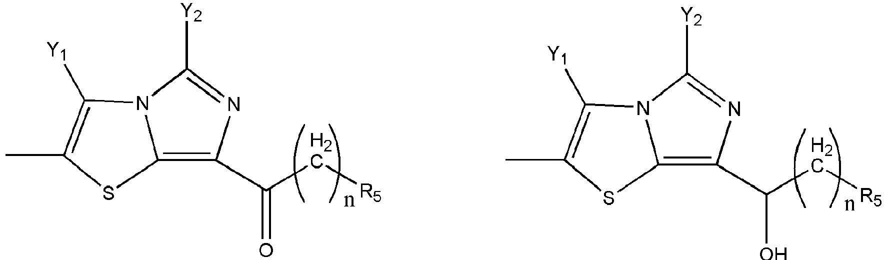 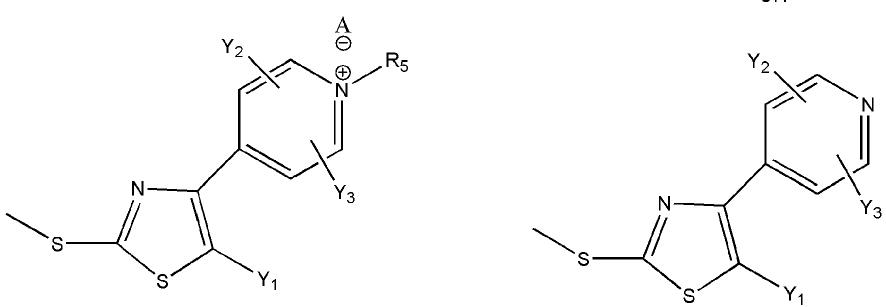 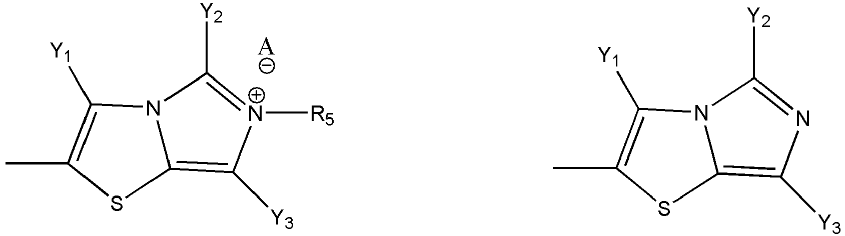 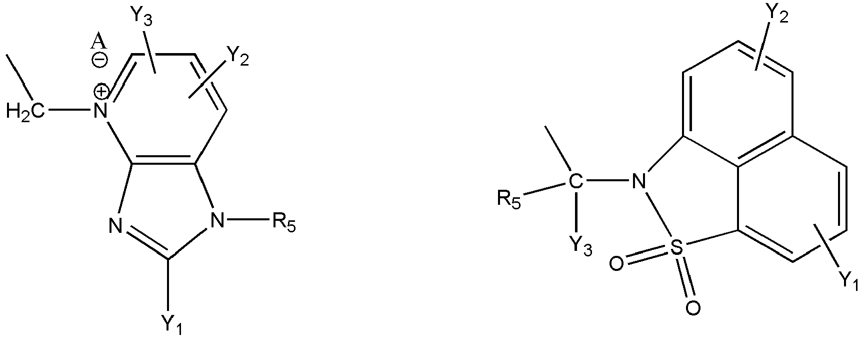 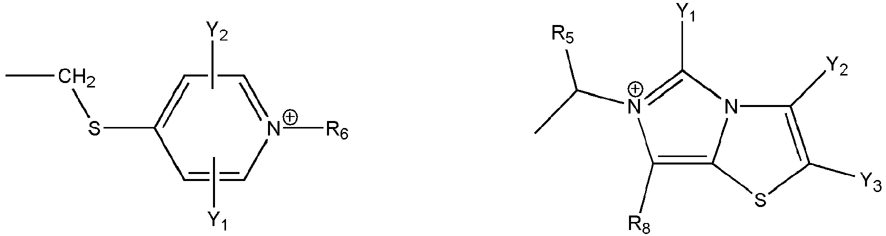 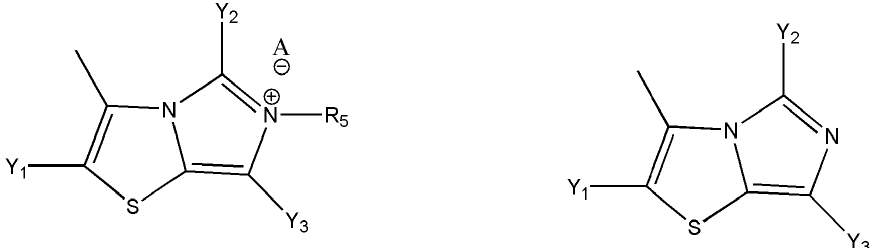 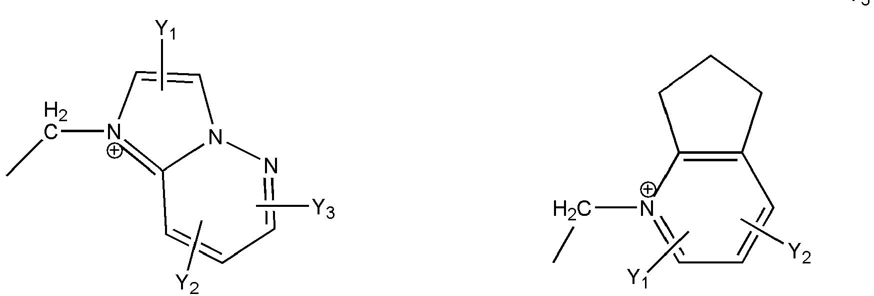 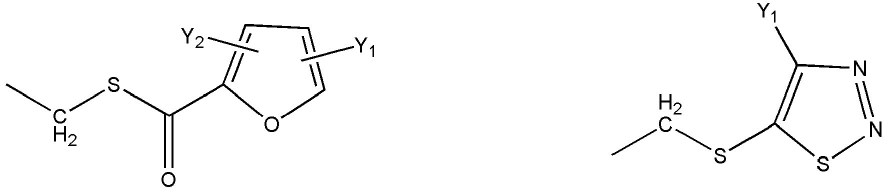 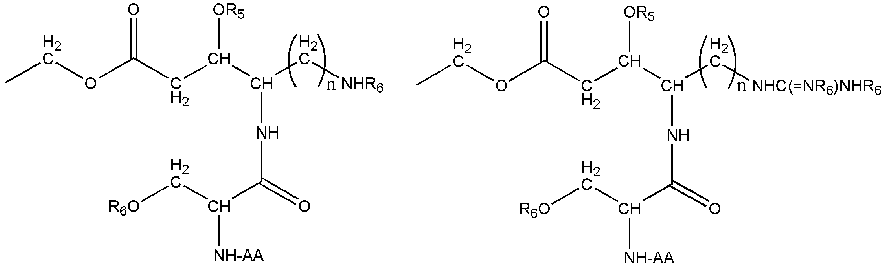 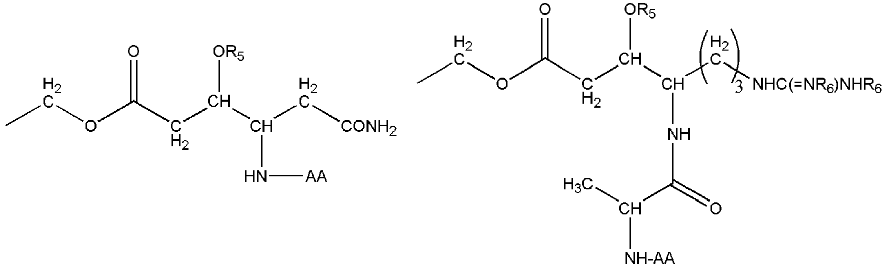 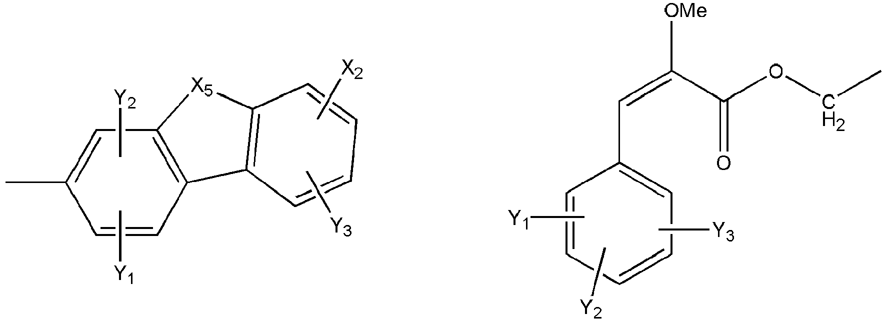 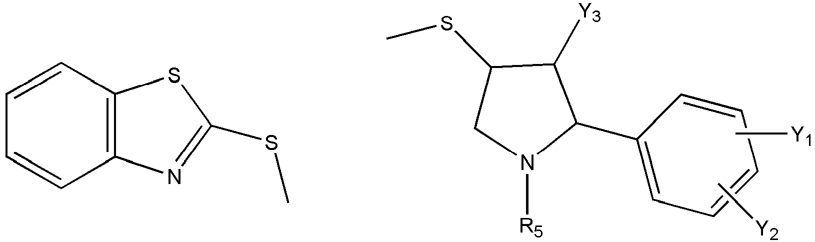 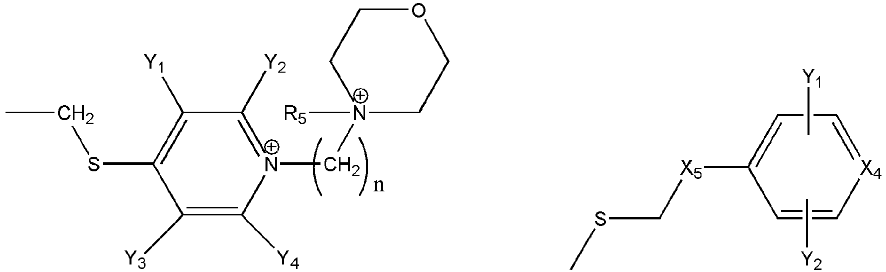 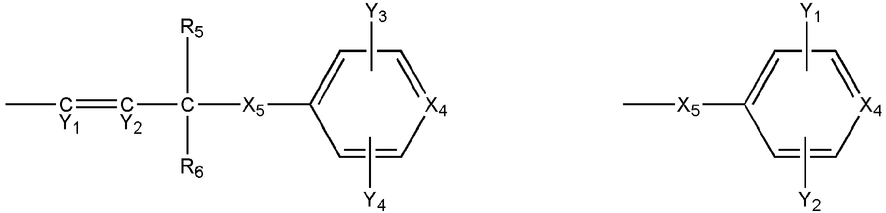 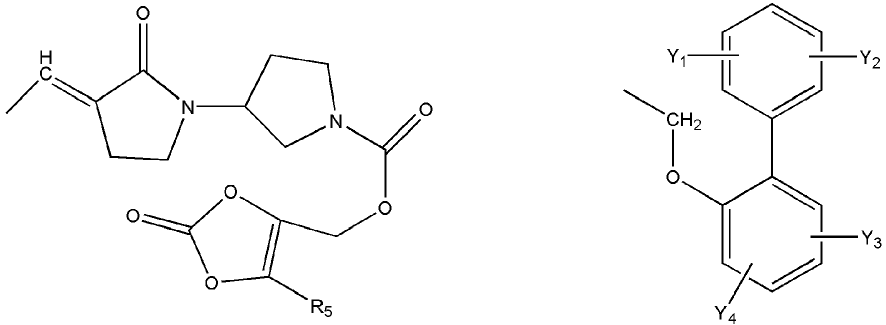 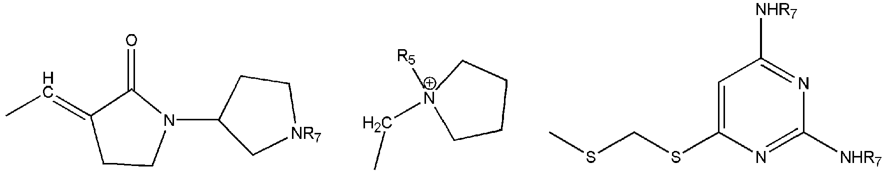 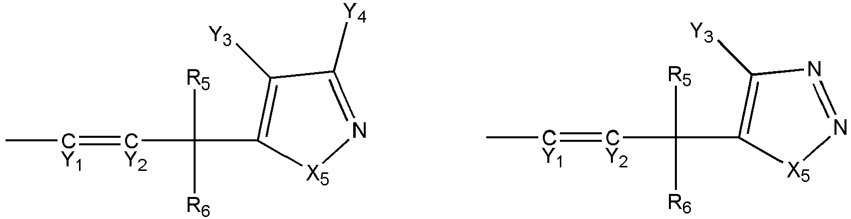 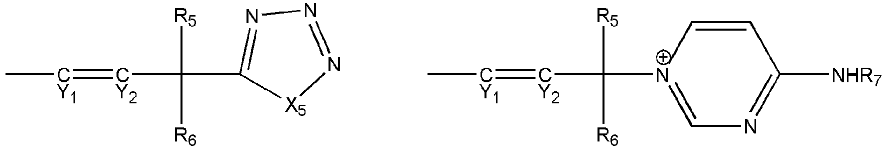 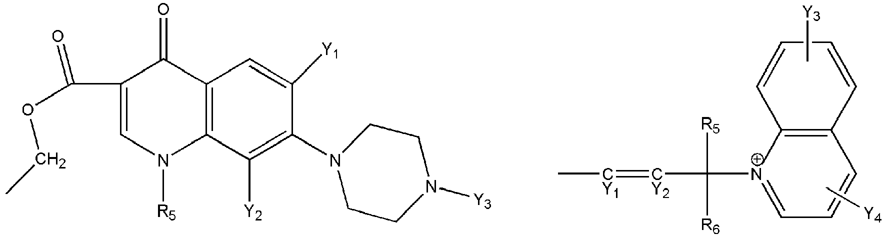 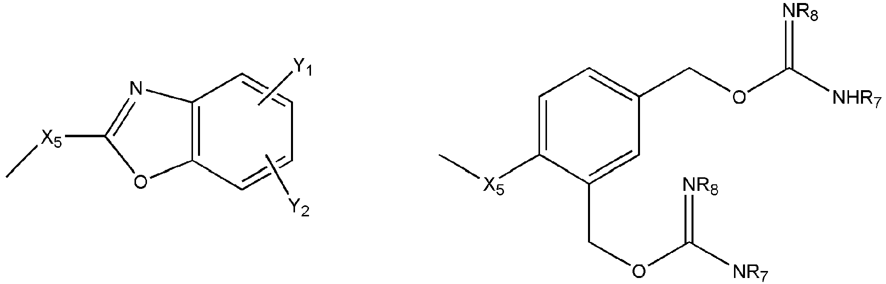 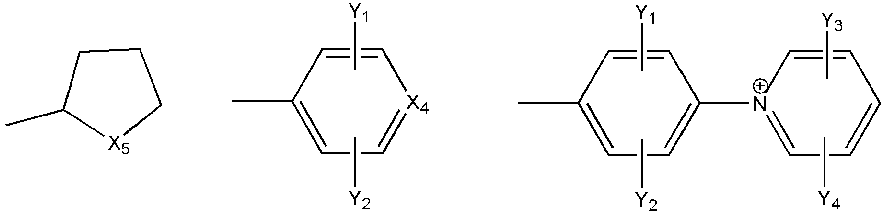 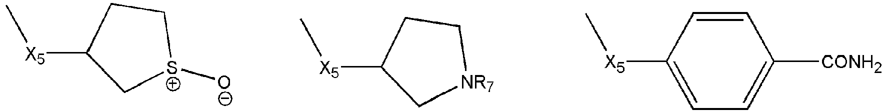 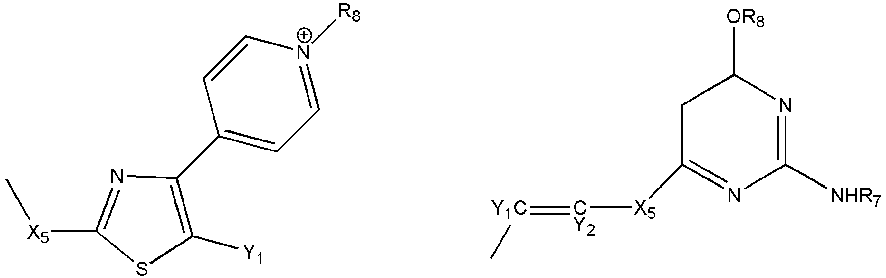 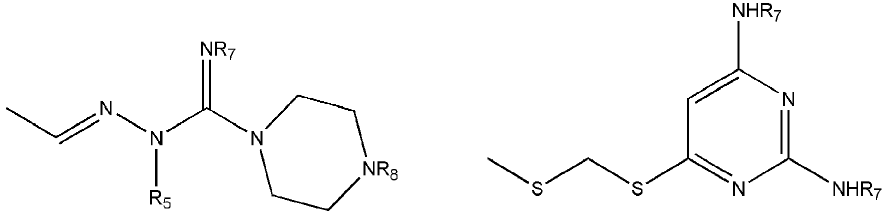 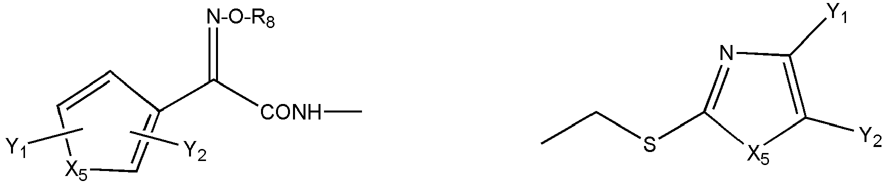 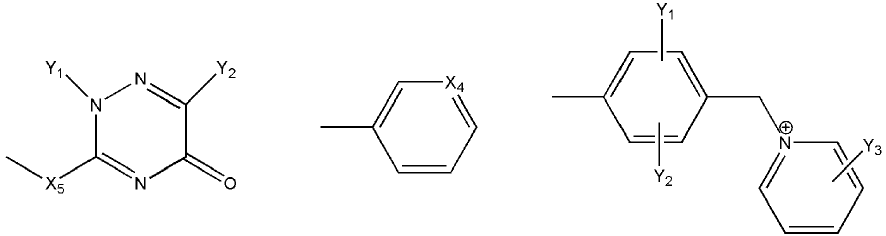 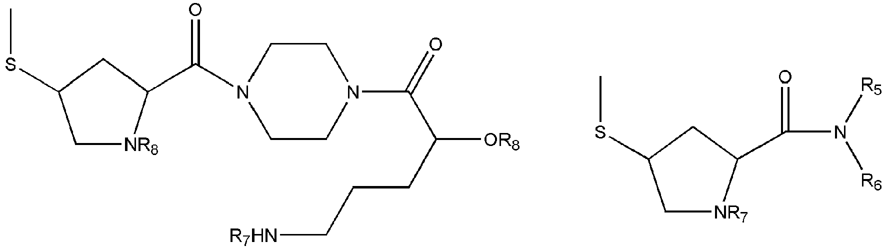 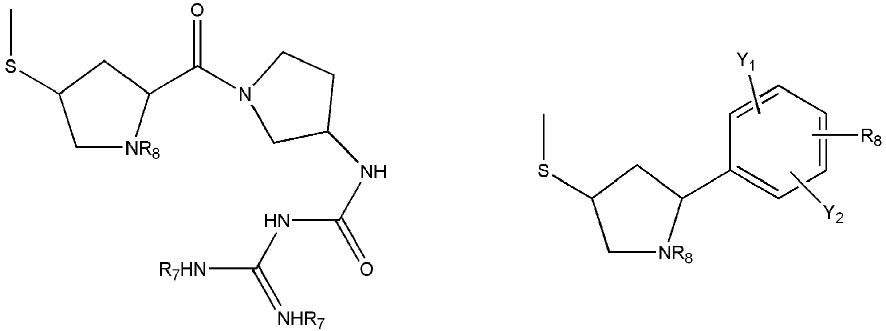 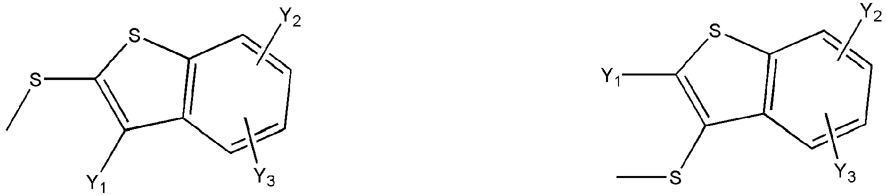 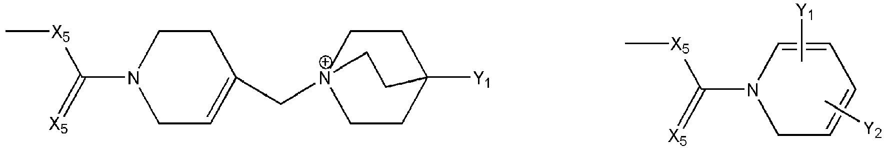 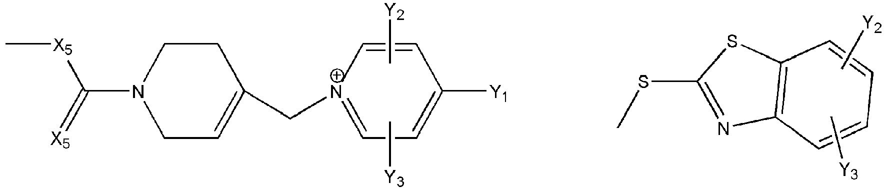 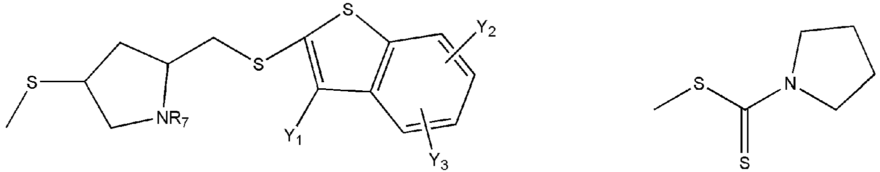 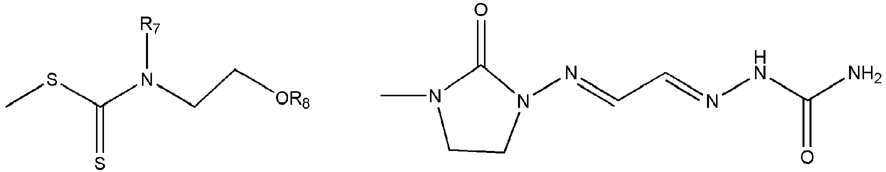 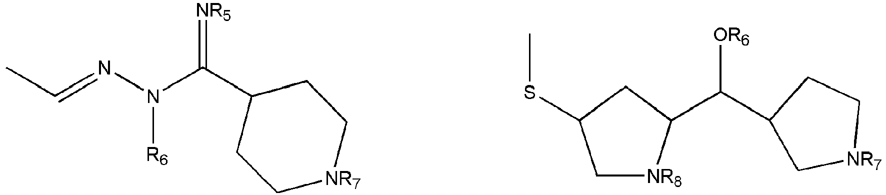 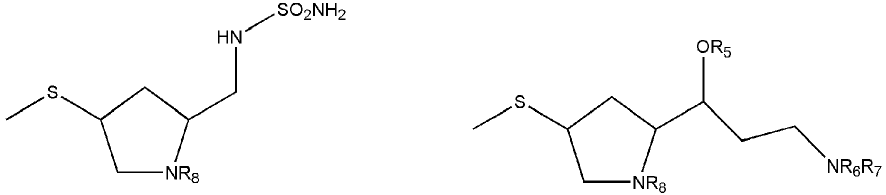 wherein, Z represents O, S, SO, SO₂, NH, CH₂, CHCH₃, or CHCH₂CH₃; R₅ represents H, CONH₂, CH₂CH₂O R₆, CH₂CH₂ N(CH₃)₂, CH₂ CH₂ N(CH₂CH₃)₂, Cl, F, Br, I, one of any alkyl, alkyloxyl, alkenyl or alkynyl residues having 1 to 12 carbon atoms, aryl or heteroaryl residues , CO-W, or W; R₆ represents H, F, Cl, Br, I, Na⁺, K⁺, COR₅, one of any alkyl, alkyloxyl, alkenyl or alkynyl residues having 1 to 12 carbon atoms, aryl or heteroaryl residues , CO-W, or W; R₇ represents H, CH₃, C₂H₅, CF₃, COCH₃, C₃H₇, C₄ H₉, C₅H₁₁, CONH₂, COR₆, COR₅ CO-W, or W; R₈ represents H, CH₃, C₂H₅, CF₃, CH₃ CH₂ CH₂F, CH₂CH₂Cl, CH₂CH₂Br, CH₂CH₂I, CH₂CHF₂, CH₂CF_{3,} CH₂F CH₂Cl, CH₂Br, CH₂I, CH₂NR₆R₇, CH(NHR₇)CH₂CONH₂, C₃H₇, C₄H₉, C₅H₁₁, CONH₂, CH₂CONH₂, CH₂OCONH₂, C(CH₃)₂COOR₆, CH(CH₃)COOR_{6,} CH₂COOR₆, COR₆, COR_{5,} CO-W, or W; X represents CH₂, S, NH, or O; X₂ represents H, CH₃, CH₂(CH₂)ₙ OR₈, Cl, F, Br, I, NO₂, CN, CF₃, OCF₃, NH₂, CH₃, C₂H₅ CONH₂, CH₂OCONH₂, CH₂COOR₅, CH₂(CH₂)ₙ N(CH₃)₂, CH₂(CH₂)ₙ SO₃R₅, C₁₋₈ alkyl, C₁₋₈ alkylthiol, C₁₋₈ alkylamine, or C₁₋₈ alkyloxy; X₃ represents H, CH₃, CH₂(CH₂)ₙOR₈, Cl, F, Br, I, NO₂, CN, CF₃, OCF₃, NH₂, CH₃, C₂ H_{5,} CONH₂, CH₂OCONH₂, CH₂COOR₅, CH₂(CH₂)ₙ N(CH₃)₂, CH₂(CH₂)ₙ SO₃R₅, C₁₋₈ alkyl, C₁₋₈ alkylthiol, C₁₋₈ alkylamine, or C₁₋₈ alkyloxy; X₄ represents N, CH , or CY₁; X₅ represents CH₂, S, O or NH; Y₁ represents H, OH, OW, OCOW, OCOCH₃, CH₃, C₂ H₅ C₃H_{7,} C₄H₉, CH₂COOR₈, OCH₃, OC₂H₅, CH₃SO₃, NO₂, CN, CF₃, OCF₃, CH₂(CH₂)ₙ NR₅R₆, CH₂(CH₂)ₙOR₆, CH(CONH₂)NHR₆, CH₂CONH₂, F ,Br,I, or Cl; Y₂ represents H, OH, OW, OCOW, CH₃, C₂H_{5,} C₃H_{7,} C₄H_{9,} CH₂COOR₈, OCOCH₃, OCH₃, OC₂H₅, CH₃SO₃, NO₂, CN, CF₃, OCF₃, CH₂(CH₂)ₙNR₅R₆, CH₂(CH₂)ₙOR₆, CH(CONH₂)NHR₆, CH₂CONH₂, F, Br, I, or Cl; Y₃ represents H, OH, OW, OCOW, OCOCH₃, CH₃, C₂H₅, C₃H_{7,} C₄H_{9,} CH₂COOR₈, OCH₃, OC₂H₅, CH₃SO₃, NO₂, CN,CF₃, OCF₃,CH₂(CH₂)ₙNR₅ R₆, CH₂(CH₂)ₙOR₆, CH(CONH₂)NHR₆, CH₂CONH₂, F, Br, I, or Cl; Y₄ represents H, OH, OW, OCOW, OCOCH₃, CH₃, C₂H₅, C₃H_{7,} C₄H_{9,} CH₂COOR₈, OCH₃, OC₂H₅, CH₃ SO₃, NO₂, CN, CF₃, OCF₃, CH₂(CH₂)ₙ NR₅R₆, CH₂(CH₂)ₙ OR_{6,} CH(CONH₂)NHR₆, CH₂ CONH₂, F, Br, I, or Cl; AA represents amino acids; n=0, 1, 2, 3, 4, 5, 6,...; Rₛ - represents: 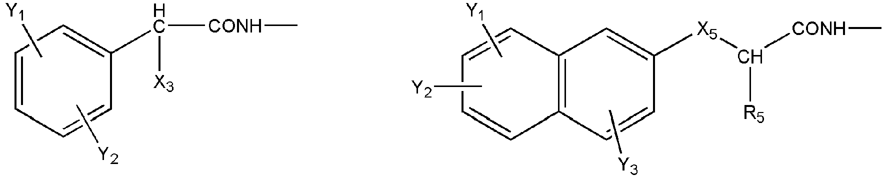 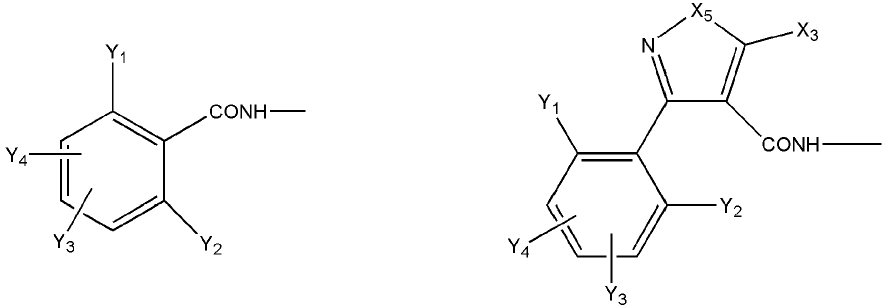 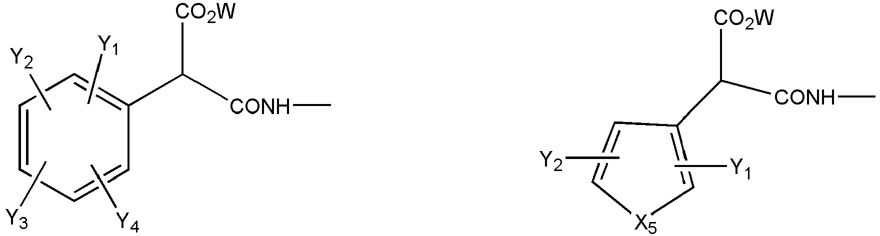 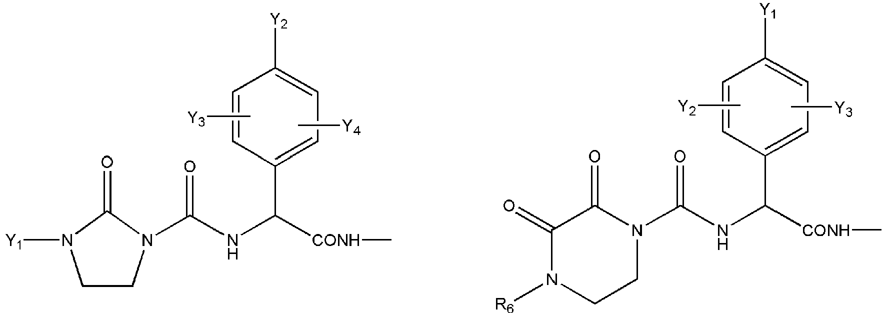 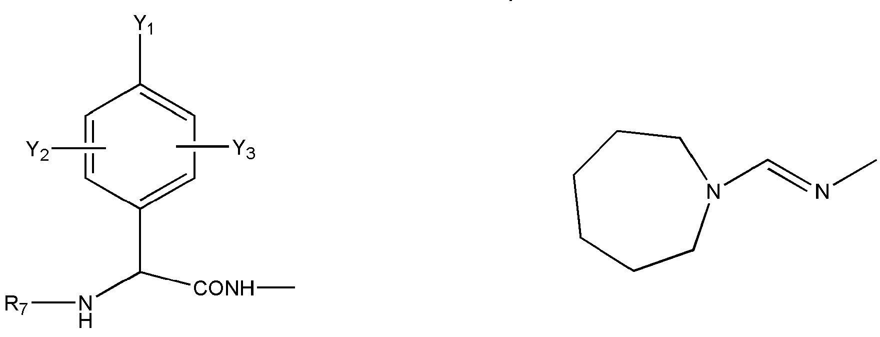 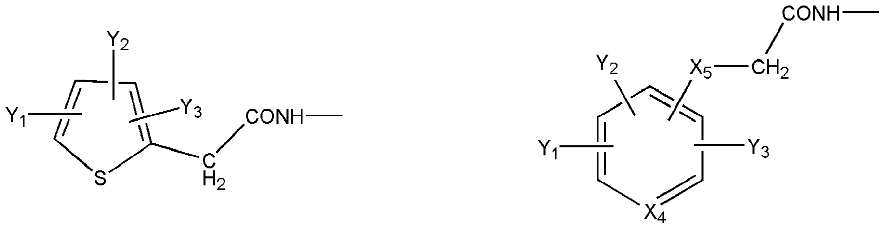 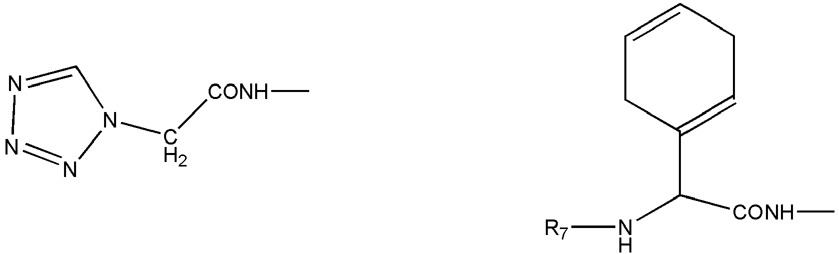 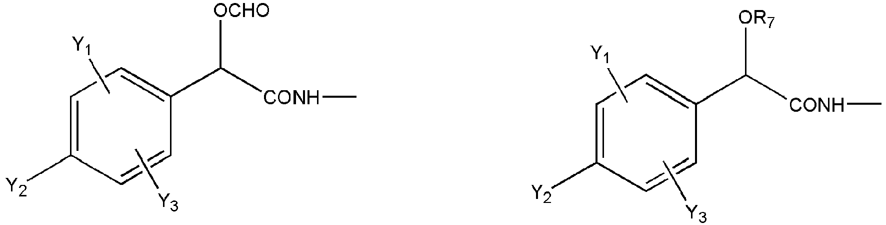 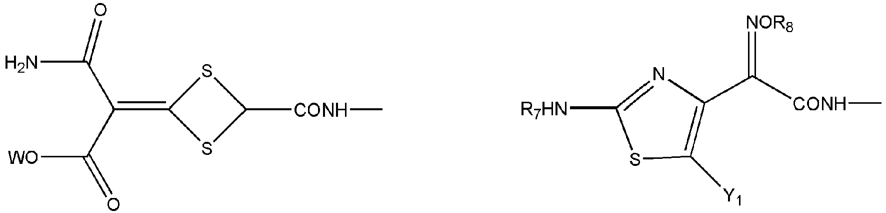 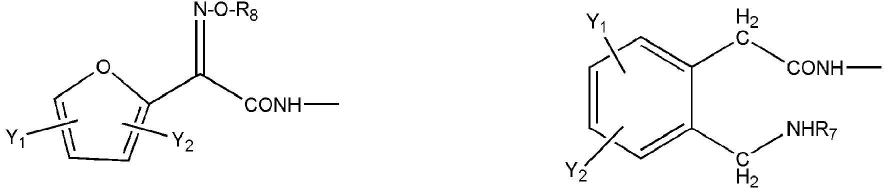 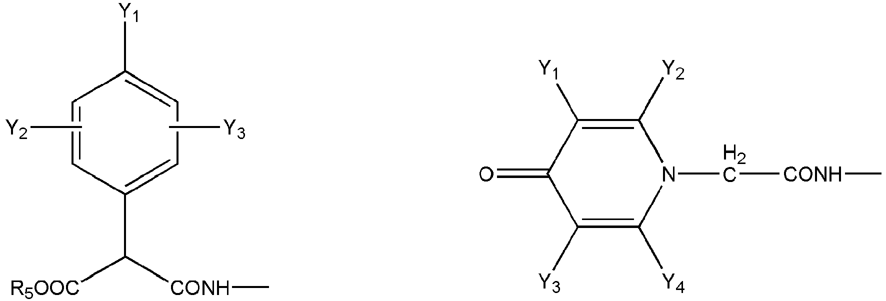 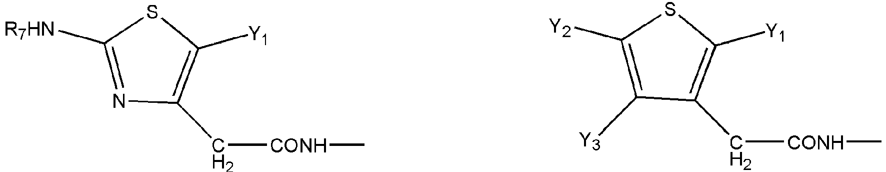 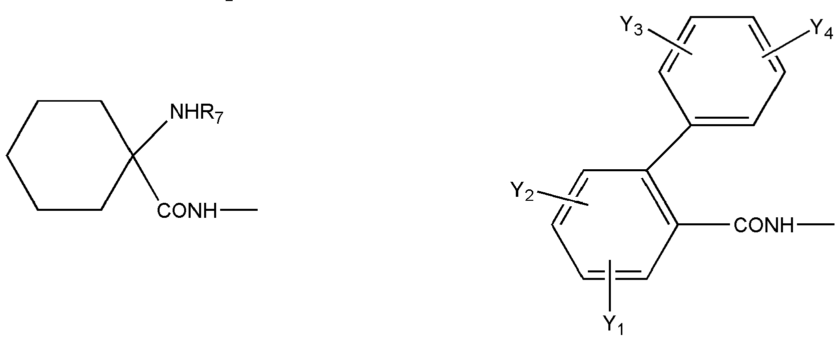 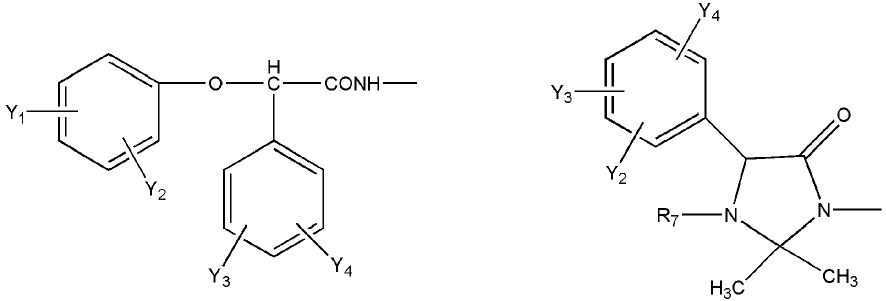 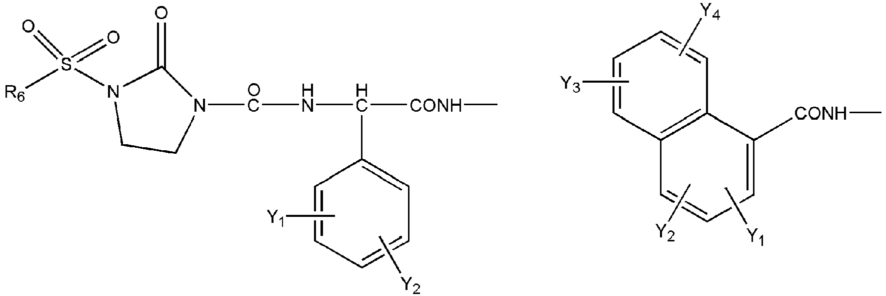 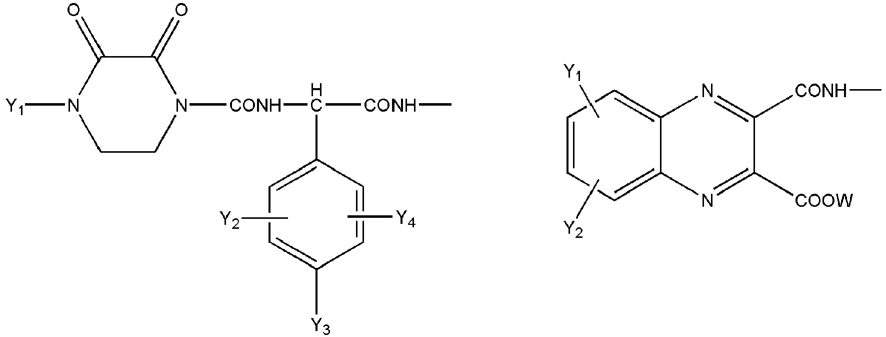 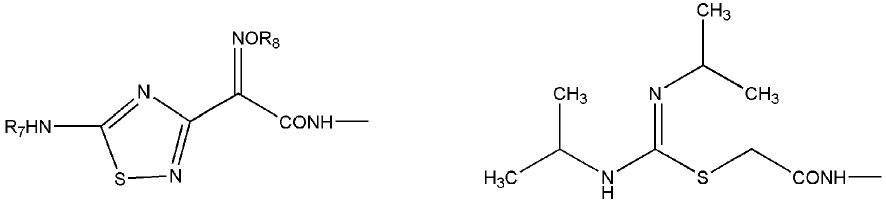 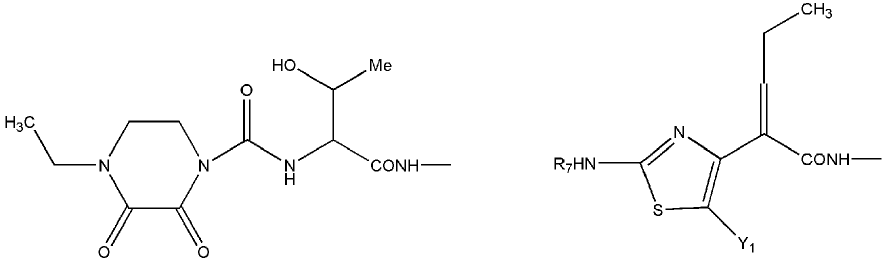 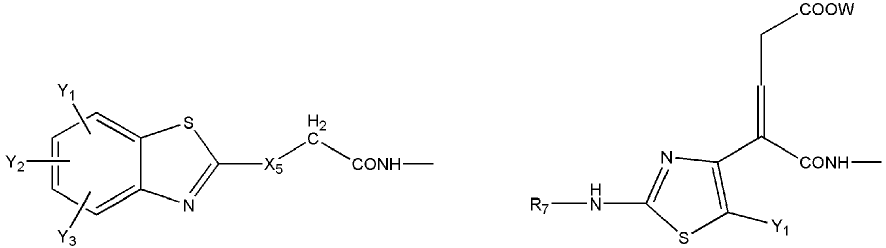 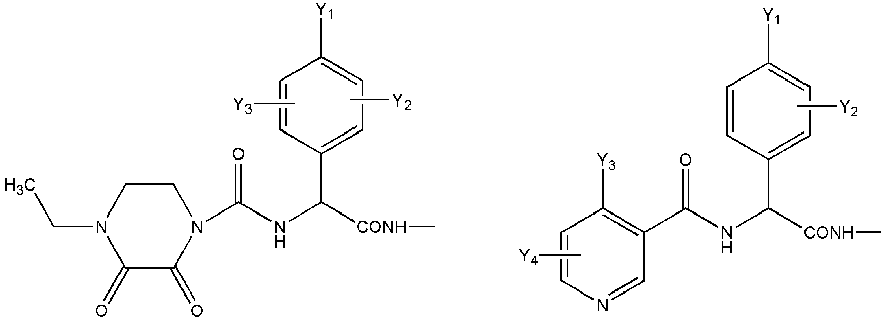 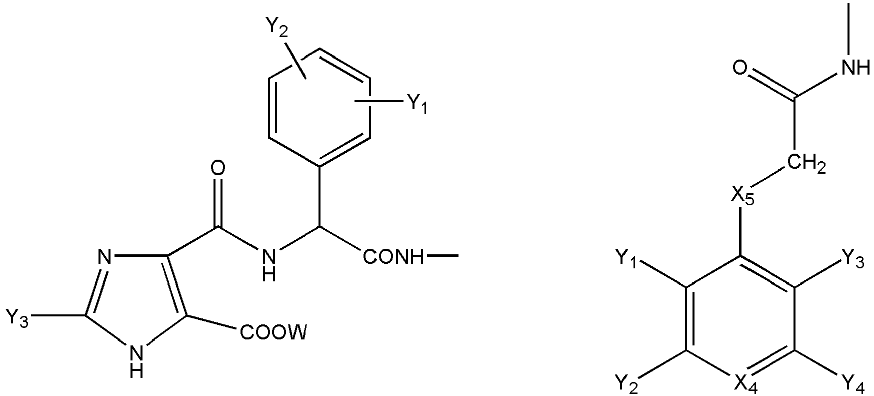 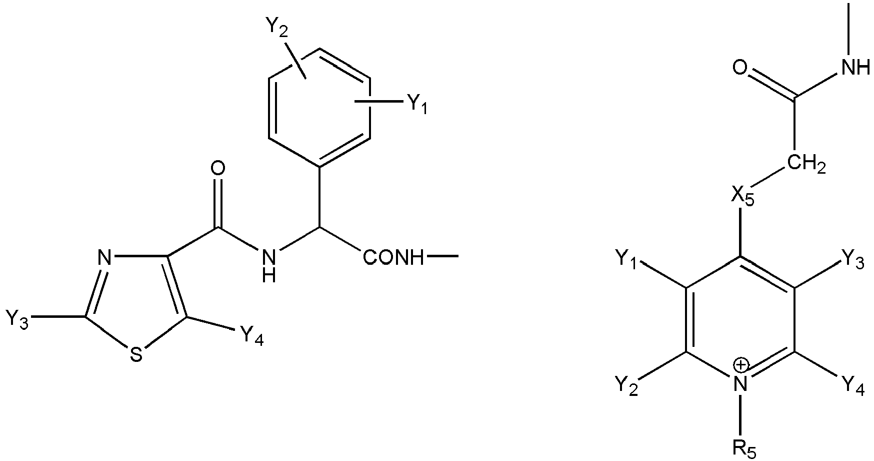 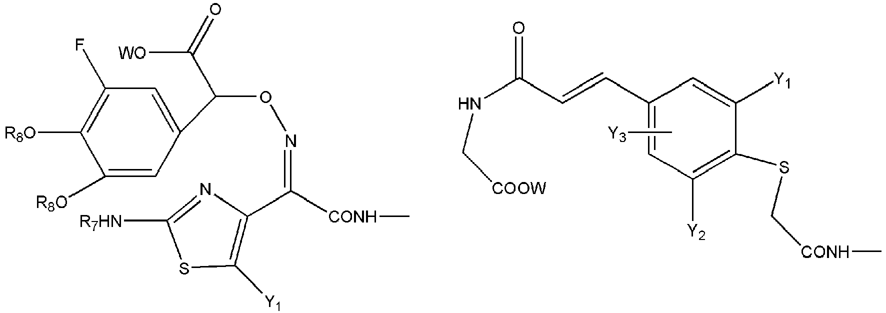 R₆ OOCCH(NHR₇)(CH₂)ₙ CONH-, R₆OOCCH(NHR₇)(CH₂)ₙ SCONH-, CF₃SCH₂ CONH-, CF₃CH₂CONH-, CHF₂SCH₂CONH-, CH₂FSCH₂CONH-, NH₂COCHFSCH₂ CONH-, R₇NHCH(COOW)CH₂SCH₂CONH-, CNCH₂SCH₂CONH-, CH₃(CH₂)ₙ CONH-, R₇N=C(NHR₇)CH₂CH₂S-, R₇N=C(NHR₇)NHCO-, CH₃C(Cl)=CHCH₂SCH₂ CONH-, (CH₃)₂C(OR₆)-, CNCH₂CONH-, CNCH₂CH₂S-, R₇N=C(NHR₇)CH₂CH₂S-, CH₂ =CHCH₂ SCH₂ CONH-, CH₃ CH(OH)-, CH₃ CH(OR₈)-, CH₃CH(Y₁)-, (CH₃)₂CH-, CH₃CH₂-, or CH₃(CH₂)ₙ CH=CH(CH₂)ₘCONH-. Wherein, n or m= 0, 1, 2, 3, 4, 5, 6, ...; X₃ represents H, N₃, SO W, F, Cl, Br, I, OH, OCH₃, OC₂H₅, CH₃, C₂H₅, COOW, OW, or W; X₄ represents N, CH , or CY₁; X₅ represents CH₂, S, O or NH; R₅ represents H, -CONH₂, CH₂CH₂N(CH₃)₂, CH₂CH₂N(CH₂CH₃)₂, CH₂CH₂OR₆, Cl, F, Br, I, one of any alkyl, alkyloxyl, alkenyl or alkynyl residues having 1 to 12 carbon atoms, aryl or heteroaryl residue, CO-W, or W; R₆ represents H, F, Cl, Br, I, 2-oxo-1-imidazolidinyl, one of any alkyl, alkyloxyl, alkenyl or alkynyl residues having 1 to 12 carbon atoms, aryl (phenyl, 5-indanyl, 2,3-dihydro-1H-inden-5-yl, et al.) or heteroaryl (4-hydroxy-1,5-naphthyridin-3-yl, et al.) residues; R₇ represents H, F, Cl, Br, I, CH₃NHCOCH₂CH(NHR₈)CO, R₅N=C(NHR₆)NHCO-, COCH₃, COR₆, PO(OR₅ )OR₆, one of any alkyl, alkyloxyl, alkenyl or alkynyl residues having 1 to 12 carbon atoms, aryl or heteroaryl residues , CO-W, or W; R₈ represents H, F, Cl, Br, I, CH₃, C ₂H₅, CF₃, CH₂CH₂F, CH₂CH₂Cl, CH₂CH₂Br, CH₂CH₂I, CH₂CHF₂, CH₂CF_{3,}CH₂F, CH₂ Cl, CH₂Br, CH₂I, C₃H₇, C₄H₉, C₅H₁₁, COR_{6,} CONH₂, CH₂OCONH₂, PO(OR₅)OR₆, C(CH₃)₂ COOR₆, CH(CH₃)COOR₆, CH₂COOR₆, CO-W, or W; Y₁ represents H, OH, OW, OCOW, OCOCH₃, CH₃, C₂H_{5,} C₃H_{7,} C₄H_{9,} SO₃R₆, CH₂COOR₈, OCH₃, OC₂H₅, CH₃SO₃, NO₂, CN, CF₃, OCF₃, CH=CHCONHCH₂COOW, CH₂(CH₂)ₙ NR₅R₆, CH₂(CH_{2 2})ₙOR₆, CH(CONH₂)NHR₆, COOR₅, F, Br, I, or Cl; Y₂ represents H, OH, OW, OCOW, CH₃, C₂H_{5,} C₃H_{7,} C₄H_{9,} CH₂COOR₈, OCOCH₃, OCH₃, OC₂H₅, CH₃SO₃, NO₂, CN, CF₃, OCF₃, CH=CHCONHCH₂COOW, CH₂ (CH₂)ₙNR₅R₆, CH₂(CH₂)ₙOR₆, CH(CONH₂ )NHR₆, SO₃R₆, COOR₅, F, Br, I, or Cl; Y₃ represents H, OH, OW, OCOW, OCOCH₃, CH₃, C₂H_{5,} C₃H_{7,} C₄H₉ CH₂COOR₈, OCH₃, OC₂H₅, CH₃SO₃, NO₂, CN, CF₃, OCF₃, CH=CHCONHCH₂ COOW, CH₂(CH)₂ₙNR₅R₆, CH₂(CH₂)ₙOR₆, CH(CONH₂)NHR₆, SO₃ R₆, COOR₅, F, Br, I, or Cl; Y₄ represents H, OH, OW, OCOW, OCOCH₃, CH₃, C₂H₅ C ₃H_{7,} C₄H_{9,} CH₂COOR₈, OCH₃, OC₂H₅, CH₃SO₃, NO₂, NR₈COR₅, SO₂NR₅R₈, COR₅, SR ₅, CN, CF₃, OCF₃, CH=CHCONHCH₂COOW, CH₂(CH₂)ₙNR₅R₆, CH₂(CH₂)ₙOR₆ CH(CONH₂)NHR₆,SO₃R₆, COOR₅, F, Br, I, or Cl; -W represents -H, one of any alkyl, alkyloxy, alkenyl or alkynyl residues having 1 to 12 carbon atoms, aryl or heteroaryl residues, or one of the following residues: 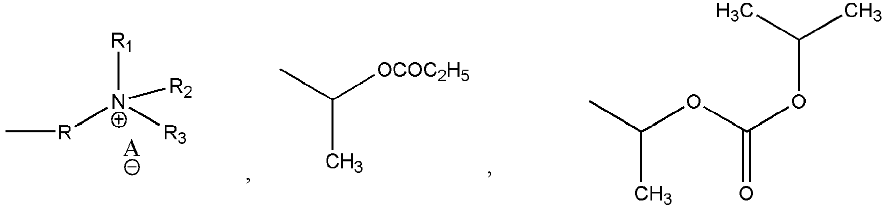 Wherein, R represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, or aryl and heteroaryl residues; R represents a branched or straight chain, -(SCH₂)ₙ-, aryl residues or heteroaryl residues; n = 0, 1, 2, 3, 4, 5, 6, 7, 8, ...; Rₛ and Y taken together are R ₆OCH₂C(R₅)=; A⁻ represents Cl⁻, Br⁻, F⁻, I ⁻, AcO⁻, citrate, or any negative ions. All double bond can be cis or trans. All R, R₁, R₂, R₃, R₄, R₅, R₆, R₇, R₈, -(CH₂)ₙ, or -(CH₂)ₘ- groups are branched or straight chains and may include C, H, O, S, or N atoms and may have single, double, and triple bonds. Any CH₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of a membrane and very hydrophobic drugs will stay in the hydrophobic layer as part of the membrane due to their similarities and cannot efficiently enter the cytosol on the inside.

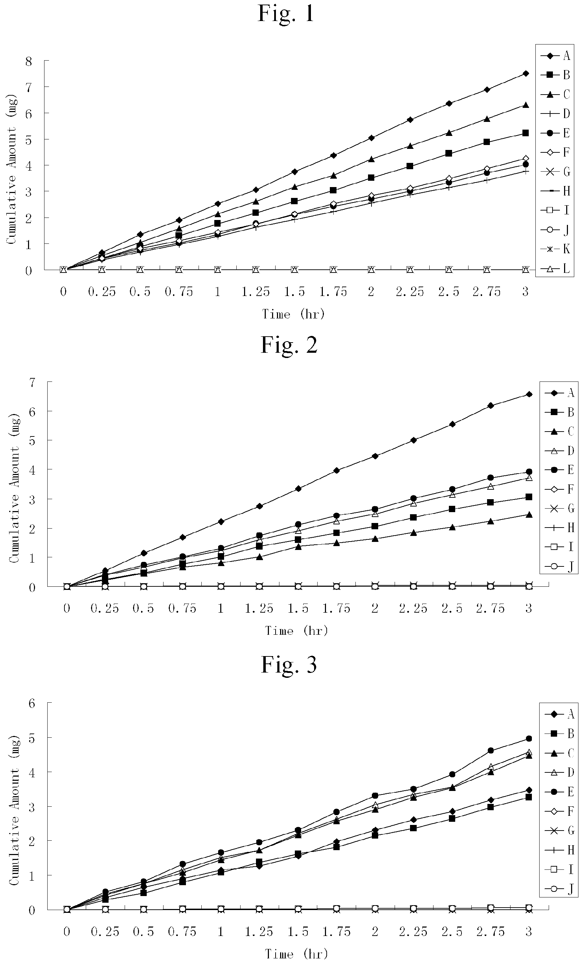

The goal of this invention is to make beta-lactam antibiotics administrable transdermally (topical application) by increasing their penetration rate through the skin barrier. These novel pro-drugs of beta-lactam antibiotics have two structural features in common: they have a lipophilic portion (which can be formed by using lipophilic alcohols to protect the carboxyl groups and lipophilic acids to protect amino, hydroxyl, or guanidino groups or other hydrophilic groups of beta-lactam antibiotics) and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water and the lipophilic portion will help the prodrugs enter the lipophilic membrane and skin barrier. When these new pro-drugs are administered transdermally in a dosage form such as solution, spray, lotion, ointment, emulsion or gel, they will dissolve in moisture of the skin surface immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. The penetration rates of some prodrugs through human skin were measured in vitro by using modified Franz cells, which were isolated from human skin tissue (360-400 µm thick) of the anterior and posterior thigh areas. The receiving fluid consisted of 2 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of a 10% solution or suspention of some of the prodrugs and beta-lactam antibiotics in 0.2mL of pH 7.4 phosphate buffer (0.2M) are shown in Figure 1, Figure 2, Figure 3, and Figure 4. Apparent flux values of 2.52 mg, 1.75 mg, 2.12 mg, 1.27 mg, 1.35 mg, 1.43 mg, 2.22 mg, 1.02 mg, 0.82 mg, 1.24 mg, 1.32 mg, 1.15 mg, 1.07 mg, 1.45 mg, 1.52 mg, 1.65 mg, 0.93 mg, 1.02 mg, 0.53 mg, 0.81 mg, 0.85 mg, 0.001 mg, 0.001 mg, 0.001 mg, 0.001 mg, 0.001 mg, 0.001 mg, 0.015 mg, 0.001 mg, mg, 0.001 mg, 0.001 mg, 0.001 mg, 0.001 mg, 0.001 mg, 0.001 mg, 0.016 mg, 0.018 mg, 0.010 mg, 0.015 mg, 0.001 mg, 0.010 mg, and 0.25 mg/cm²/h were calculated for 6-phenoxyacetacetamidopenicillanic acid 2-diethylaminoethyl ester hydrochloride, allylmercaptomethylpenicillinic acid 2-diethylaminoethyl ester hydrochloride, 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride, 6-[3-(o-chlorophenyl)-5-methyl-4-isoxazolecarboxamido]penicillinic acid 2-diethylaminoethyl ester hydrochloride, 6-[3-(2,6-dichlorophenyl)-5-methyl-4-isoxazolecarboxamido]penicillinic acid 2-diethylaminoethyl ester hydrochloride, 6-[D(-)-α-acetamidophenylacetamidopenicillinic acid 2-diethylaminoethyl ester hydrochloride, D-α-[(imidazolidin-2-on-1-yl)carbonylamino]benzylpenicillin 2-diethylaminoethyl ester hydrochloride, 6R-[2-[3-(methylsulfonyl)-2-oxo-1-imidazolidinecarboxamido]-2-phenylacetamido]pe nicillinic acid 2-diethylaminoethyl ester hydrochloride, 6-D(-)-α-(4-ethyl-2,3-dioxo-1-piperazinylcarbonylamino)-a-phenylacetamidopenicilli nic acid 2-diethylaminoethyl ester hydrochloride, 7-(2-thienylacetamido)cephalosporanic acid 2-diethylaminoethyl ester hydrochloride, 7-[(hydroxyphenylacetyl)amino]-3-[[(1-methyl-1H-tetrazol-5-yl)thio]methyl]-8-oxo-5-thia-1-azabicyclo[4.2.0]oct-2-ene-2-carboxylic acid 2-diethylaminoethyl ester hydrochloride, 3-[[(aminocarbonyl)oxy]methyl]-7-[[2-furanyl(methoxyimino)acetyl] amino]-8-oxo-5-thia-1-azabicyclo[4.2.0]oct-2-ene-2-carboxylic acid 2-diethylaminoethyl ester hydrochloride, 3-[[(aminocarbonyl)oxy]methyl]-7-methoxy-8-oxo-7-[(2-thienylacetyl)amino]-5-thia-1-azabicyclo[4.2.0]oct-2-ene-2-car boxylic acid 2-diethylaminoethyl ester hydrochloride, 7-[[[2-(acetylaminomethyl)phenyl]acetyl]amino]-3-[[[1-(ethoxylcarbonylmethyl)-1H-t etrazol-5-yl]thio]methyl]-8-oxo-5-thia-1-azabicyclo[4.2.0]oct-2-ene-2-carboxylic acid 2-diethylaminoethyl ester hydrochloride, 7-[(acetylaminophenylacetyl)amino] - 3-chloro-8-oxo-5-thia-1-azabicyclo[4.2.0]oct-2-ene-2-carboxylic acid 2-diethylaminoethyl ester hydrochloride, 3-[(acetyloxy)methyl] - 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7- [[[[(4-ethyl-2,3-dioxo-1-piperazinyl)carbonyl]amino](4-acetoxyphenyl)acetyl]amino ]-3-[[(1-methyl-1H-tetrazol-5-yl)thio]methyl]-8-oxo-5-thia-1-azabicyclo[4.2.0]oct-2-e ne-2-carboxylic acid 2-diethylaminoethyl ester hydrochloride, 7-[2-(2-acetylamino-4-thiazolyl)-2-((Z)-methoxyimino)acetamido]-3-(methoxymethyl) -8-oxo-5-thia-1-azabicyclo[4.2.0]oct-2-ene-2-carboxylic acid 2-diethylaminoethyl ester hydrochloride, 7-[2-(2-acetylamino-4-thiazolyl)-2-((Z)-ethoxycarbonylmethoxy)imino]acetamido]-3-( vinyl)-8-oxo-5-thia-1-azabicyclo[4.2.0]oct-2-ene-2-carboxylic acid 2-diethylaminoethyl ester hydrochloride, penicillin V, penicillin O, methicillin, oxacillin, cloxacillin, dicloxacillin, ampicillin, azlocillin, mezlocillin, piperacillion, cephalothin, cefamandole, cefuroxime, cefoxitin, ceforanide, cefaclor, cefotaxime, ceftizoxime, cefoperazone, cefpodoxime proxetil, or cefixime diffuse through human skin. The results suggest that most of the pro-drugs of antibiotics diffuse through human skin hundred times faster than do the parent antibiotics. Only cefpodoxime proxetil has very high skin penetration rate. The reason is that cefprodoxime has a positive charged amino group and a lipophilic portion. Watts, et al. (U.S. Pat. Nos. 6,191,143 and 5,929,086) have demonstrated that topical administration of cefpodoxime proxetil is effective for the treatment of a systemic bacterial disease, but they did not know the reason at that time. The results suggest that the positive charge on the dialkyaminoethyl group has a very important role in the passage of the drug across the membrane and skin barrier. The positive charge on the amino groups of these pro-drugs will bond to the negative charge on the phosphate head group of membrane. This bonding will disturb the membrane a little bit and may make some room for the lipophilic portion of the prodrug. When the molecules of membrane move, the membrane may 'crack' a little bit due to the bonding of the prodrug. This will let the prodrug insert into the membrane. At pH 7.4, only about 99% of amino group is protonated. When the amino group is not protonated, the bonding between the amino group of the prodrug and the phosphate head group of membrane will disassociate, and the prodrug will enter the membrane completely. When the amino group of the prodrug flips to the other side of the membrane and thus become protonated, then the prodrug is pulled into the cytosol, a semi-liquid concentrated aqueous solution or suspension.

The in vivo rates of penetration of through the skin and blood-brain barrier of intact hairless mice were studied. The donor consisted of a 20% solution of 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride and 6-[3-(o-chlorophenyl)-5-methyl-4-isoxazolecarboxamido]penicillinic acid 2-diethylaminoethyl ester hydrochloride in 1 mL of isopropanol applied to a 10 cm² on the backs of the hairless mice. After 2 hours, the mice were killed. 5 ml of methanol was added 1 g of homogenized blood, liver, kidney, muscle, or brain. The samples were centrifuged for 5 min and analyzed by HPLC. The amount of the parent drugs is 50 +/-7 µg/g in blood, 40 +/-5 µg/g in liver, 35 +/-5 µg/g in kidney, 40 +/-6 µg/g in muscle, 20 +/-5 µg/g in brain for methicillin, 55 +/-7 µg/g in blood, or 38 +/-5 µg/g in liver, 36 +/-5 µg/g in kidney, 32 +/-4 µg/g in muscle, 18 +/-5 µg/g in brain for oxacillin, and 45 +/-7 µg/g in blood, or 34 +/-5 µg/g in liver, 32 +/-5 µg/g in kidney, 30 +/-4 µg/g in muscle, 16 +/-5 µg/g in brain for cloxacillin. The results show these prodrugs have very high pentration rate of blood-brain barrier.

In vitro plasma hydrolysis studies, 10 mg of the prodrug was dissolved in 0.1 ml of 0.2M pH 7.4 phosphate buffer and 1 ml of human plasma, preheated to 37C, was added into the mixture. The mixture was kept in a water bath at 37C, and at every 2 min intervals. 0.2 ml of samples were withdrawn and added to 0.4 ml of methanol to precipitate the plasma protein. The samples were centrifuged for 5 min and analyzed by HPLC. The half life of the prodrugs are 8+/- 1 min., 8+/- 1 min., 10+/- 1 min., 12+/- 1 min., 8+/- 1 min., 12+/- 1 min., 10+/- 1 min., 9+/- 1 min., 13+/- 1 min., 15+/- 1 min., 9+/- 1 min., 10+/- 1 min., 8+/- 1 min., 7+/- 1 min., 9+/- 1 min., 8+/- 1 min., 10+/- 1 min., 11+/- 1 min., 12+/- 1 min., 8+/- 1 min., and 9+/- 1 min. for 6-phenoxyacetacetamidopenicillanic acid 2-diethylaminoethyl ester hydrochloride, allylmercaptomethylpenicillinic acid 2-diethylaminoethyl ester hydrochloride, 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride, 6-[3-(o-chlorophenyl)-5-methyl-4-isoxazolecarboxamido]penicillinic acid 2-diethylaminoethyl ester hydrochloride, 6-[3-(2,6-dichlorophenyl)-5-methyl-4-isoxazolecarboxamido]penicillinic acid 2-diethylaminoethyl ester hydrochloride, 6-[D(-)-α-acetamidophenylacetamidopenicillinic acid 2-diethylaminoethyl ester hydrochloride, D-α-[(imidazolidin-2-on-1-yl)carbonylamino]benzylpenicillin 2-diethylaminoethyl ester hydrochloride, 6R-[2-[3-(methylsulfonyl)-2-oxo-1-imidazolidinecarboxamido]-2-phenylacetamido]pe nicillinic acid 2-diethylaminoethyl ester hydrochloride, 6-D(-)-a-(4-ethyl-2,3-dioxo-1-piperazinylcarbonylamino)-α-phenylacetamidopenicilli nic acid 2-diethylaminoethyl ester hydrochloride, 7-(2-thienylacetamido)cephalosporanic acid 2-diethylaminoethyl ester hydrochloride, 7-[(hydroxyphenylacetyl)amino]-3-[[(1-methyl-1H-tetrazol-5-yl)thio]methyl]-8-oxo-5-thia-1-azabicyclo[4.2.0]oct-2-ene-2-carboxylic acid 2-diethylaminoethyl ester hydrochloride, 3-[[(aminocarbonyl)oxy]methyl]-7-[[2-furanyl(methoxyimino)acetyl] amino]-8-oxo-5-thia-1-azabicyclo[4.2.0]oct-2-ene-2-carboxylic acid 2-diethylaminoethyl ester hydrochloride, 3-[[(aminocarbonyl)oxy]methyl] - 7-methoxy-8-oxo-7-[(2-thienylacetyl)amino]-5-thia-1-azabicyclo[4.2.0]oct-2-ene-2-car boxylic acid 2-diethylaminoethyl ester hydrochloride, 7-[[[2-(acetylaminomethyl)phenyl]acetyl]amino]-3-[[[1-(ethoxylcarbonylmethyl)-1H-t etrazol-5-yl]thio]methyl]-8-oxo-5-thia-1-azabicyclo[4.2.0]oct-2-ene-2-carboxylic acid 2-diethylaminoethyl ester hydrochloride, 7-[(acetylaminophenylacetyl)amino] - 3-chloro-8-oxo-5-thia-1-azabicyclo[4.2.0]oct-2-ene-2-carboxylic acid 2-diethylaminoethyl ester hydrochloride, 3-[(acetyloxy)methyl] - 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7- -[[[[[(4-ethyl-2,3-dioxo-1-piperazinyl)carbonyl]amino](4-acetoxyphenyl)acetyl]amino ]-3-[[(1-methyl-1H-tetrazol-5-yl)thio]methyl]-8-oxo-5-thia-1-azabicyclo[4.2.0]oct-2-e ne-2-carboxylic acid 2-diethylaminoethyl ester hydrochloride, 7-[2-(2-acetylamino-4-thiazolyl)-2-((Z)-methoxyimino)acetamido]-3-(methoxymethyl) -8-oxo-5-thia-1-azabicyclo[4.2.0]oct-2-ene-2-carboxylic acid 2-diethylaminoethyl ester hydrochloride, 7-[2-(2-acetylamino-4-thiazolyl)-2-((Z)-ethoxycarbonylmethoxy)imino]acetamido]-3-( vinyl)-8-oxo-5-thia-1-azabicyclo[4.2.0]oct-2-ene-2-carboxylic acid 2-diethylaminoethyl ester hydrochloride.

To study efficacy of topical antibiotic treatment in lactating cows with clinical mastitis, 90 lactating dairy cows were recruited. Bacteriological care was considered to have been achieved if the samples taken from the affected quarter on day 17 and day 22 were free of the bacterial species isolated in the pretreatment sample. Clinical cure was defined as the disappearance of clinical signs of disease which were observed on day 1 before treatment, in other words by the return to normal feed intake, rectal temperature<39.0°C, good general condition, absence of udder edema, normal milk appearance, and normal milk yield. 500 mg of 6-phenoxyacetacetamidopenicillanic acid 2-diethylaminoethyl ester hydrochloride (penicillin V-DAEE), 6-(2,6-dimethoxybenzamido)penicillinic acid 2-diethylaminoethyl ester hydrochloride (methicillin-DAEE), or 7-[[(2-acetylamino-4-thiazolyl)(methoxyimino)acetyl]amino]-8-oxo-5-thia-1-azabicyclo[4.2.0]oct-2-ene-2-carboxylic acid 2-diethylaminoethyl ester hydrochloride (ceftizoxime-DAEE) in 10 ml of pH 7.4 phosphate buffer (0.2 M) was sprayed on the skin of udder twice per day. The results are shown in table 1 and 2.

**Table 1. Clinical cure rates of the topical treatment of cow mastitis with the novel prodrugs of antibiotics.**

| Prodrugs | Number of Cows | Cure Rate (%) | | | |
|---|---|---|---|---|---|
| | | Day 3 | Day 8 | Day 17 | Day 22 |
| Penicillin V-DAEE | 30 | 50 | 90 | 93 | 97 |
| Methicillin-DA EE | 30 | 43 | 90 | 97 | 100 |
| Ceftizoxime-D AEE | 30 | 53 | 93 | 99 | 100 |

**Table 2. Bacteriological cure rates (day 22) of the topical treatment of cow mastitis with the novel prodrugs of antibiotics.**

| Pathogens | | Prodrugs | | |
|---|---|---|---|---|
| | | Penicillin V-DAEE | Methicillin-DA EE | Ceftizoxime-DA EE |
| Staphylococcus aureus | No of cows | 6 | 5 | 6 |
| | No. cured (%) | 4 (67%) | 4 (80%) | 5 (83%) |
| Streptococcus uris | No of cows | 10 | 10 | 11 |
| | No. cured (%) | 8 (80%) | 7 (70%) | 9 (82%) |
| E. coli | No of cows | 8 | 10 | 8 |
| | No. cured (%) | 7(87.5%) | 8 (80%) | 7(87.5%) |
| Coagulase-negativ e staphylococci | No of cows | 9 | 7 | 8 |
| | No. cured (%) | 7(78%) | 6(85.7%) | 7(87.5%) |
| Enterobacteriacea e | No of cows | 7 | 8 | 6 |
| | No. cured (%) | 6 (85.7) | 6 (75%) | 5(83.3%) |

The prodrugs have demonstrated very high Clinical and Bacteriological cure rates.

After 1 hours of topical application, the milk samples were taken and analysized. The amount of the parent drugs is 80 +/-7 µg/g in milk for penicillin V, 75 +/-6 µg/g methicillin, and 70 +/-7 µg/g for ceftizoxime. The results have shown that these prodrugs have very high penetration rate of blood-milk barrier. Their very high penetration rates of blood-milk or blood-brain barrier make them very valuable for treatment of brain, breast, prostate gland and other infections.

The cell wall of gram-negative bacteria contains an outer membrane (lipophilic) and periplasm (hydrophilic) that make many antibiotics unpassible. These novel pro-drugs of beta-lactam antibiotics have a lipophilic portion and a hydrophilic portion that make them highly passible to both of outer membrane and periplasm, so they can easily reach the penicillin binding proteins. On the very outside of the cell wall of gram-positive bacteria is a set of characteristic carbohydrates and proteins that are hydrophilic. The hydrophilic portion of the prodrugs will facilitate the passage of these pro-drugs through the carbohydrates and proteins barrier to reach the penicillin binding proteins. These prodrugs should have very broad antibiotics spectrum to both of gram-negative bacteria and gram-positive bacteria and have much less antibiotic-resistance problem.

The compounds of the general formula (1) 'Structure 1' indicated above can be prepared from beta-lactam antibiotics (free acids), by reaction with compounds of the general formula (2) 'Structure 2' by using coupling reagents, such as N,N'-Dicyclohex ylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al. 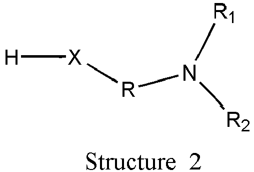 wherein, R represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R represents a branched or straight chain, -(CH₂)ₙ -, aryl residues or heteroaryl residues; n = 0, 1, 2, 3, 4, 5, 6, 7, 8, ....; X represents O, S, or NH.

When X represents O, the compounds of the general formula (1) 'Structure 1' indicated above can be prepared from metal salts, organic base salts, or immobilized base salts of beta-lactam antibiotics, by reaction with compounds of the general formula (3) 'Structure 3': 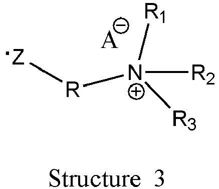 wherein, R represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R ₃ represents H, one of any alkyl, alkyloxy, alkenyl, or alkynyl residues having 1 to 12 carbon atoms, or aryl residues; Z represents F, Cl, Br, I, or p-toluenesulphonyl; A⁻represents Cl⁻, Br⁻, F, I⁻, AcO⁻, citrate, or any negative ions; and n=0,1,2,3,4,5...; R represents a branched or straight chain, -(CH₂)ₙ -, aryl residues or heteroaryl residues; n = 0, 1, 2, 3, 4, 5, 6, 7, 8, ....; X represents O, S, or NH.

### Advantageous Effects

These pro-drugs of beta-lactam antibiotics have a lipophilic portion and a hydrophilic portion (the amine groups that exist in the protonated form at physiological pH). The positively charged amino groups of these pro-drugs have two major advantages. First, it makes prodrugs soluble in water; when these new pro-drugs are administered transdermally in a dosage form such as solution, spray, lotion, ointment, emulsion or gel, they will mix with moisture on the skin, eye, genital area, mouth, nose, or other part of the body immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. This bonding will also disturb the membrane a little bit and may make some room for the lipophilic portion (by modification of the polar groups with lipophilic alkyl groups) of the prodrug. When the molecules of membrane move, the membrane may 'crack' a little bit due to the bonding of the prodrug. This will let the prodrug insert into the membrane. At pH 7.4, only about 99% of amino group is protonated. When the amino group is not protonated, the bonding between the amino group of the prodrug and the phosphate head group of membrane will disassociate, and the prodrug will enter the membrane completely. When the amino group of the prodrug flips to the other side of the membrane and thus become protonated, then the prodrug is pulled into the cytosol, a semi-liquid concentrated aqueous solution or suspension. The high penetration rates of the skin, membrance, blood-brain and blood-milk barriers make them very valuable for treatment of brain, breast, prostate gland and other infections. These prodrugs have very broad antibiotics spectrum to both of gram-negative bacteria and gram-positive bacteria and have much less antibiotic-resistance problem.

### Description of Drawings

Figure 1: Cumulative amounts of 6-phenoxyacetacetamidopenicillanic acid 2-diethylaminoethyl ester hydrochloride, allylmercaptomethylpenicillinic acid 2-diethylaminoethyl ester hydrochloride, 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride, 6-[3-(o-chlorophenyl)-5-methyl-4-isoxazolecarboxamido]penicillinic acid 2-diethylaminoethyl ester hydrochloride, 6-[3-(2,6-dichlorophenyl)-5-methyl-4-isoxazolecarboxamido]penicillinic acid 2-diethylaminoethyl ester hydrochloride, penicillin V, penicillin O, methicillin, oxacillin, cloxacillin, dicloxacillin, crossing isolated human skin tissue in Franz cells (n=5). In each case, the vehicle was pH 7.4 phosphate buffer (0.2 M).
Figure 2: Cumulative amounts of 6-[D(-)-α-acetamidophenylacetamidopenicillinic acid 2-diethylaminoethyl ester hydrochloride, D-α-[(imidazolidin-2-on-1-yl)carbonylamino]benzylpenicillin 2-diethylaminoethyl ester hydrochloride, 6R-[2-[3-(methylsulfonyl)-2-oxo-1-imidazolidinecarboxamido]-2-phenylacetamido]penicillinic acid 2-diethylaminoethyl ester hydrochloride, 6-D(-)-α-(4-ethyl-2,3-dioxo-1-piperazinylcarbonylamino)-α-phenylacetamidopenicilli nic acid 2-diethylaminoethyl ester hydrochloride, 7-(2-thienylacetamido)cephalosporanic acid 2-diethylaminoethyl ester hydrochloride, ampicillin, azlocillin, mezlocillin, piperacillion, cephalothin, crossing isolated human skin tissue in Franz cells (n=5). In each case, the vehicle was pH 7.4 phosphate buffer (0.2 M).
Figure 3: Cumulative amounts of 7-[(hydroxyphenylacetyl)amino] - 3-[[(1-methyl-1H-tetrazol-5-yl)thiolmethyl]-8-oxo-5-thia-1-azabicyclo[4.2.0]oct-2-ene -2-carboxylic acid 2-diethylaminoethyl ester hydrochloride, 3-[[(aminocarbonyl)oxy] methyl]-7-[[2-furanyl(methoxyimino)acetyl]amino]-8-oxo-5-thia-1-azabicyclo[4.2.0] oct-2-ene-2-carboxylic acid 2-diethylaminoethyl ester hydrochloride, 3-[[(aminocarbonyl)oxy]methyl]-7-methoxy-8-oxo-7-[(2-thienylacetyl)amino]-5-thia-1 -azabicyclo[4.2.0]oct-2-ene-2-carboxylic acid 2-diethylaminoethyl ester hydrochloride, 7-[[[2-(acetylaminomethyl)phenyl]acetyl]amino]-3-[[[1-(ethoxylcarbonylmethyl)-1H-t etrazol-5-yl]thio]methyl]-8-oxo-5-thia-1-azabicyclo[4.2.0]oct-2-ene-2-carboxylic acid 2-diethylaminoethyl ester hydrochloride, 7-[(acetylaminophenylacetyl)amino] - 3-chloro-8-oxo-5-thia-1-azabicyclo[4.2.0]oct-2-ene-2-carboxylic acid 2-diethylaminoethyl ester hydrochloride, cefamandole, cefuroxime, cefoxitin, ceforanide, cefaclor, crossing isolated human skin tissue in Franz cells (n=5). In each case, the vehicle was pH 7.4 phosphate buffer (0.2 M).

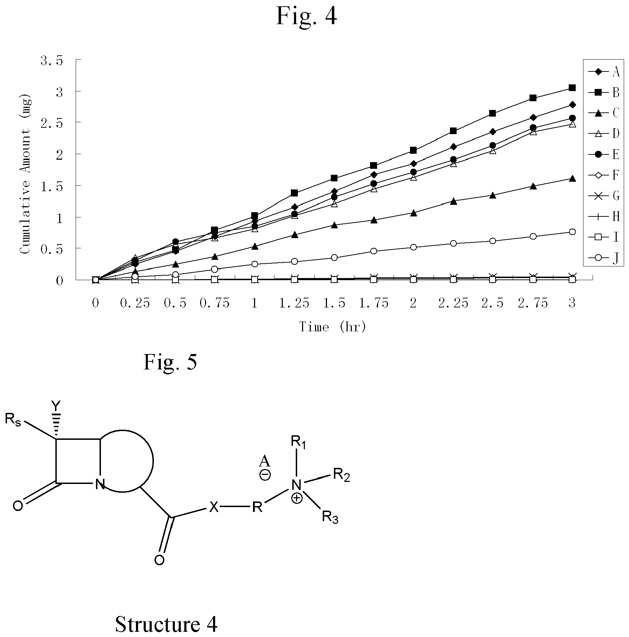

Figure 4: Cumulative amounts of 3-[(acetyloxy)methyl]-7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7- -[[[[(4-ethyl-2,3-dioxo-1-piperazinyl)carbonyl]amino](4-acetoxyphenyl)acetyl]amino ]-3-[[(1-methyl-1H-tetrazol-5-yl)thio]methyl]-8-oxo-5-thia-1-azabicyclo[4.2.0]oct-2-e ne-2-carboxylic acid 2-diethylaminoethyl ester hydrochloride, 7-[2-(2-acetylamino-4-thiazolyl)-2-((Z)-methoxyimino)acetamido]-3-(methoxymethyl) -8-oxo-5-thia-1-azabicyclo[4.2.0]oct-2-ene-2-carboxylic acid 2-diethylaminoethyl ester hydrochloride, 7-[2-(2-acetylamino-4-thiazolyl)-2-((Z)-ethoxycarbonylmethoxy)imino]acetamido]-3-( vinyl)-8-oxo-5-thia-1-azabicyclo[4.2.0]oct-2-ene-2-carboxylic acid 2-diethylaminoethyl ester hydrochloride, cefotaxime, ceftizoxime, cefoperazone, cefpodoxime proxetil, or cefixime, crossing isolated human skin tissue in Franz cells (n=5). In each case, the vehicle was pH 7.4 phosphate buffer (0.2 M).
Figure 5. Wherein, X represents O, S, or NH; Y represents H, OH, NHCHO, CH₂ CH₂NHC(=NR₇)NHR₇, OCOCH₃, OCH₃, OC₂H₅, CH₃SO₃, NO₂, CN, CF₃, OCF₃, F, Br, I, or Cl; R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; R represents a branched or straight chain, -(CH₂)ₙ -, aryl residues or heteroaryl residues; n = 0, 1, 2, 3, 4, 5, 6, 7, 8, ...; Rₛ represents the side chains of beta-lactam antibiotics; A⁻ represents Cl⁻, Br⁻, F⁻, I⁻, AcO⁻, citrate, or any negative ions. All R, -(CH₂)ₙ- or -(CH₂)ₘ- groups are branched or straight chains and may include C, H, O, S, or N atoms and may have single, double, and triple bonds. Any CH₂ groups may be replaced with O, S, or NH.

### Best Mode

### Preparation of 6-phenoxyacetacetamidopenicillanic acid 2-diethylaminoethyl ester hydrochloride

39 g (0.1 mol) of Penicillin V potassium was dissolved in 100 ml of acetonitrile. 39 g (0.15 mol) of 2-Bromo-N,N-diethylethylamine.HBr in ethyl acetate was added into the reaction mixture. The mixture was stirred for 3 h at RT. Then 8 g of sodium bicarbonate was added into the reaction mixture. The mixture is stirred for another 2 h at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 3.5 g of HCl in 50 ml of ether was added into the reaction mixture with stirring. The solid product was collected by filtration. After drying, it yielded 38 g of the desired hygroscopic product (78.2%). Solubility in water: 50 mg/ml; Elementary analysis: C ₂₂H₃₂ ClN₃O₅S; MW: 486.0. Calculated % C: 54.37; H: 6.64; N: 8.65; Cl: 7.29; O: 16.46; S: 6.60; Found % C: 54.32; H: 6.68; N: 8.61; Cl: 7.32; O: 16.51; S: 6.56. ¹H-NMR (400 MHz, D₂O): δ: 1.28 (s, 6H), 1.35 (t, 6H), 3.21 (m, 4H), 3.43 (m, 2H), 4.51 (m, 2H), 4.68 (s, 1 H), 4.79 (s, 2 H), 4.86 (m, 1 H), 5.19 (m, 1H), 6.68 (m, 2H), 6.80 (m, 1H), 7.11 (m, 2H), 7.21 (b, 1H).

The other compounds of the general formula (1) 'Structure 1' can be synthesized by the same processure.

### Mode for Invention

### Preparation of 6-(2,6-dimethoxybenzamido)penicillinic acid 2-diethylaminoethyl ester hydrochloride

38 g (0.1 mol) of 6-(2,6-dimethoxybenzamido)penicillinic acid was dissolved in 300 ml of chloroform. 20.6 g of N, N'-Dicyclohexylcarbodiimide was added into the reaction mixture. 11.7 g of N,N-dimethylaminoethanol and 2g of 4-dimethylaminopyridine were added into the reaction mixture. The mixture was stirred for 10 hours at RT. The solid was removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 3.5 g of HCl in 50 ml of ether was added into the reaction mixture with stirring. The solid product was collected by filtration. After drying, it yielded 40 g of the desired hygroscopic product (77.5%). Solubility in water: 50 mg/ ml; Elementary analysis: C ₂₃H₃₄ClN₃O₆S; MW: 516.05. Calculated % C: 53.53; H: 6.64; N: 8.14; Cl: 6.87; O: 18.60; S: 6.21; Found % C: 53.49; H: 6.68; N: 8.11; Cl: 6.90; O: 18.64; S:6.18. ¹H-NMR (400 MHz, D₂O): δ: 1.28 (s, 6H), 1.35 (t, 6H), 3.21 (m, 4H), 3.75 (s, 6H), 3.43 (m, 2H), 4.51 (m, 2H), 4.68 (s, 1 H), 4.84 (m, 1 H), 5.17 (m, 1H), 6.50 (m, 2H), 7.21 (m, 1H), 7.28 (b, 1H).

The other compounds of the general formula (1) 'Structure 1' can be synthesized by the same processure.

### Industrial Applicability

The compounds of the general formula (1) 'Structure 1' can penetrate the skin, blood-brain, and blood-milk barriers easily. They are very valuable for treatment of skin, brain, breast, prostate gland and other infections due to their high penetration rates of the skin, blood-brain, and blood-milk barriers. These prodrugs have very broad antibiotics spectrum to both of gram-negative bacteria and gram-positive bacteria and have much less antibiotic-resistance problem.

Further preferred embodiments are given in the following paragraphs:
[1] The compounds of the general formula (1) 'Structure l'except penethamate hydriodide (3,3-dimethyl-7-oxo-6-[(phenylacetyl)amino] - 4-thia-1-azabicyclo[3.2.0]heptane-2-carboxylic acid 2-(diethylamino)ethyl ester monohydriodide) which is a known compound (the high penetration rate through skin, blood-brain, and blood-milk barriers of this compound have never been mentioned). 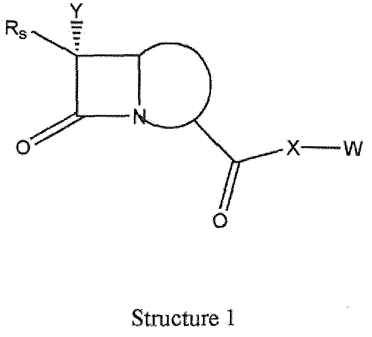 Wherein, X represents O, S, or NH; Y represents H, OH, OR₅, NHCHO, CH₂CH₂ NHC(=NR₇)NHR₇, OCOCH₃, OCH₃, OC₂H₅, CH₃SO₃, NO₂, CN, CF₃, OCF₃, F, Br, I, or Cl; 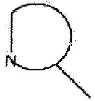 represents 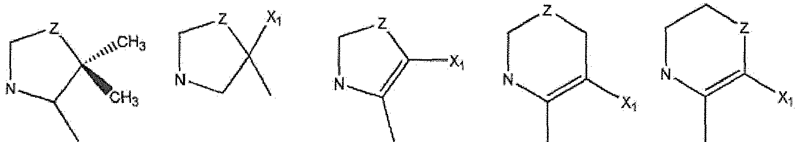 wherein, Z represents S, O, SO, SO₂, NH, CHCH₃, CHCH₂CH₃, or CH₂; -X₁ represents: -H, -OH, -OCH₃, -OC₂H₅, OC₃H₇, -CONH₂, -CH₂OCONH₂, -CH₂ OCOCH₃, -OCOCH₃, -CH₂OCOCH₃, -CH₂OCH₃, -CH₃, -C₂H_{5,} -C₃H_{7,} -C₄H₉, C₅H ₁₁, C₆H₁₃, -Cl, -F, -Br, -I, HC=CHCH₃, -HC=CH₂, -CH₂OCH₃, -S(CH₂)ₙ -NHR₇, 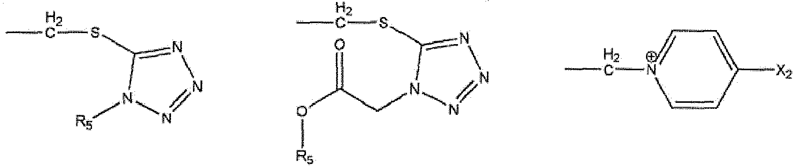 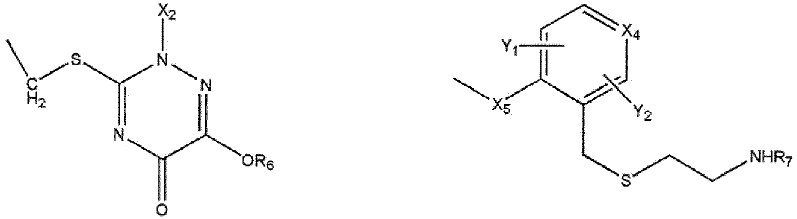 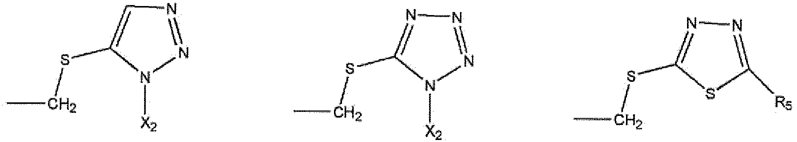 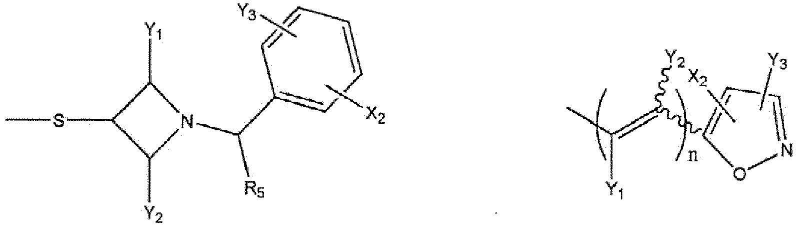 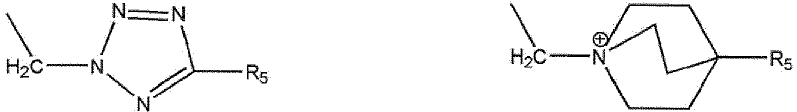 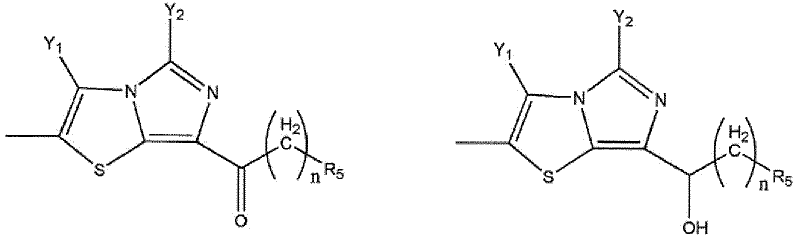 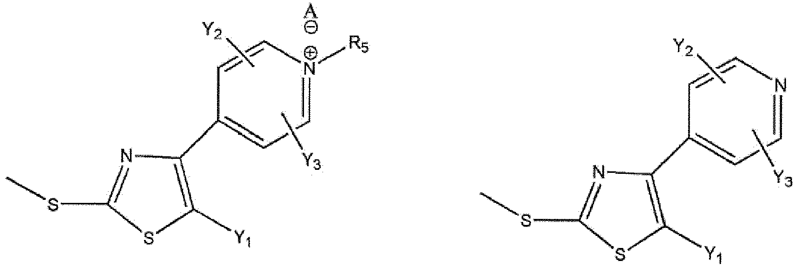 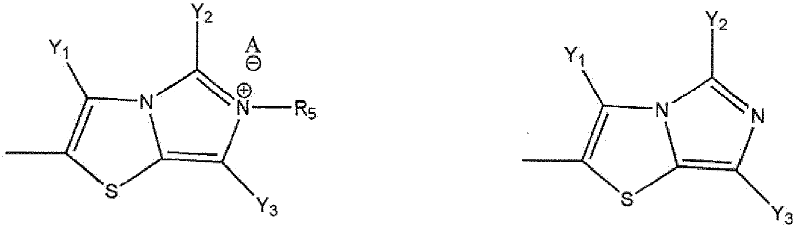 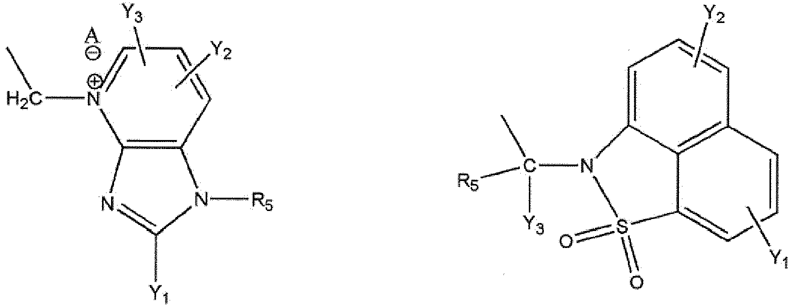 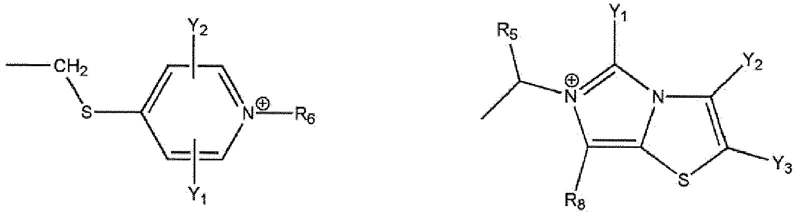 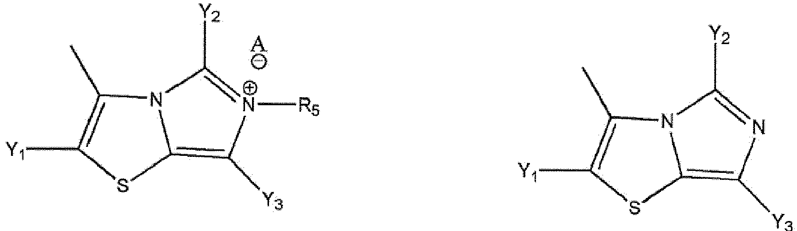 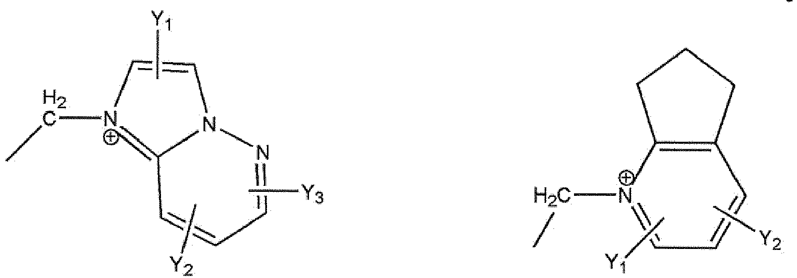 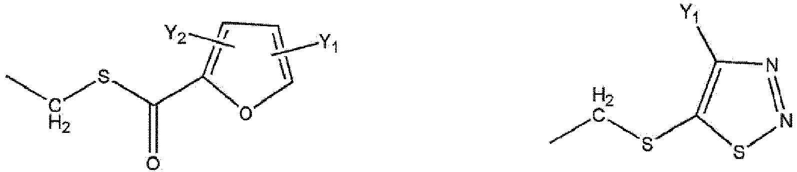 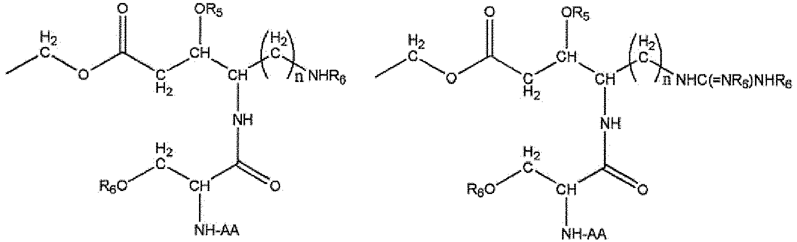 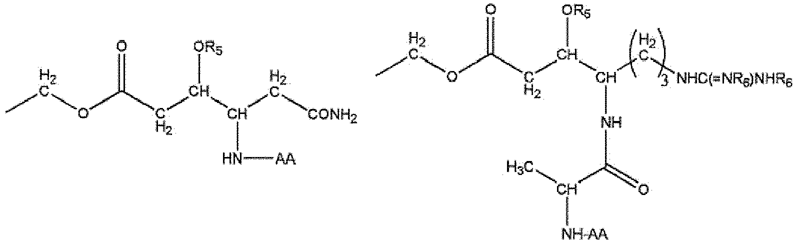 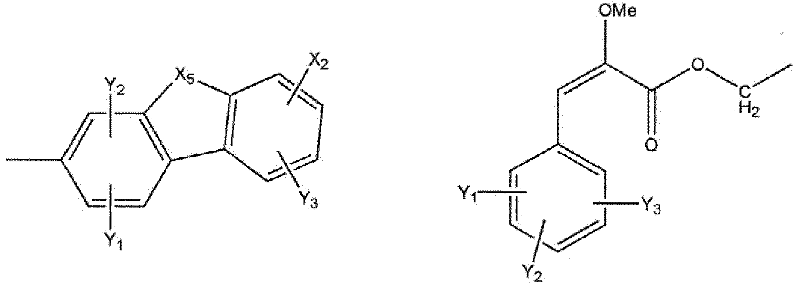 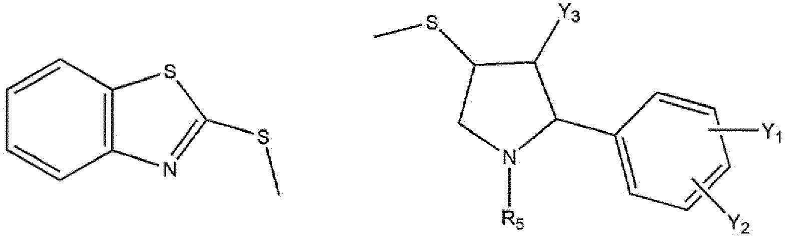 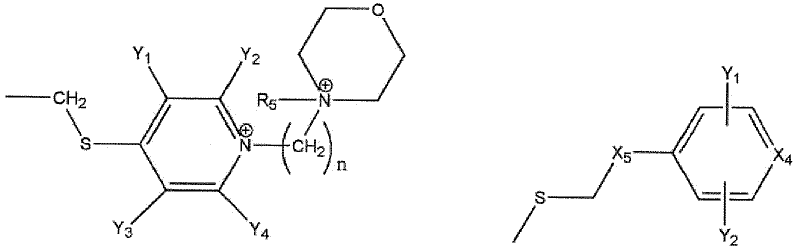 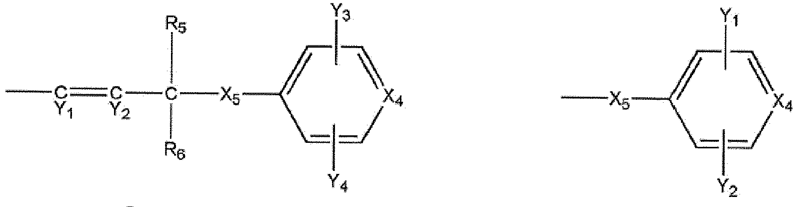 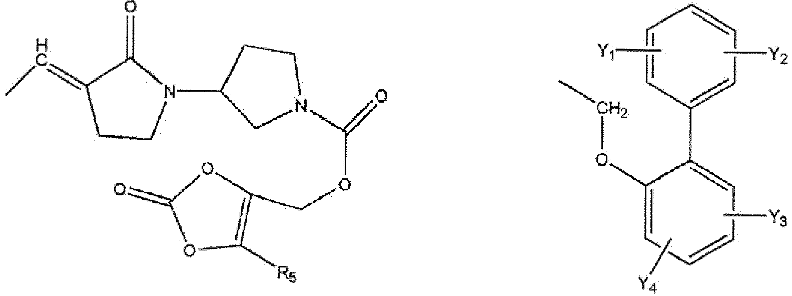 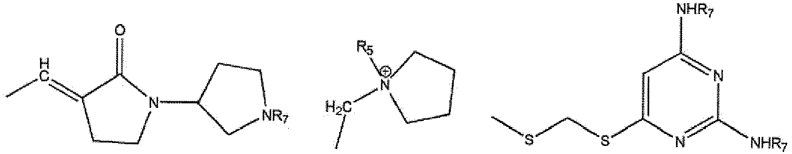 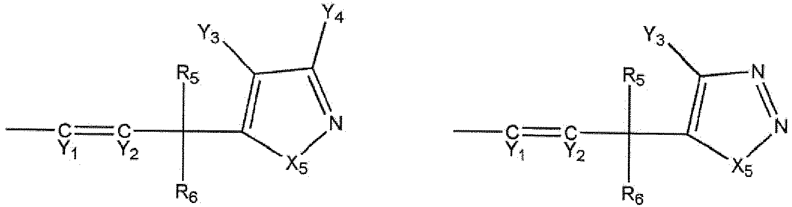 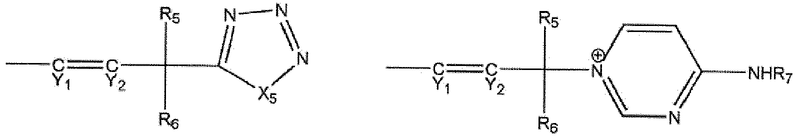 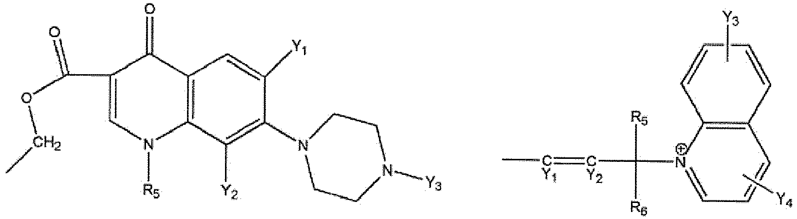 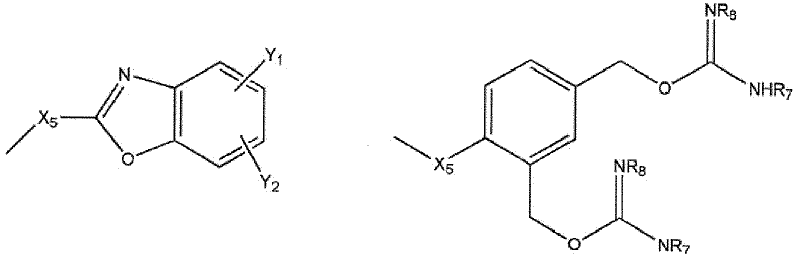 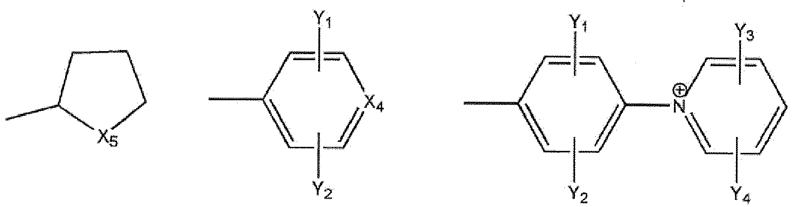 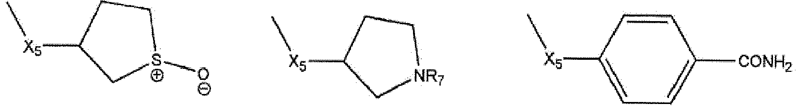 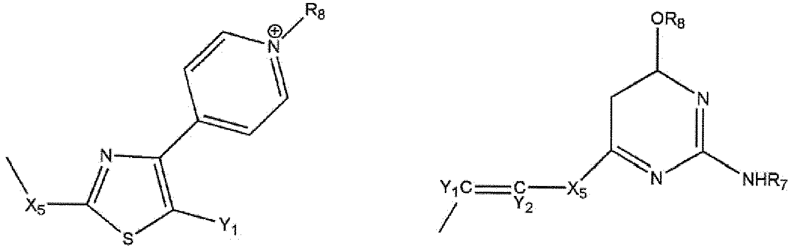 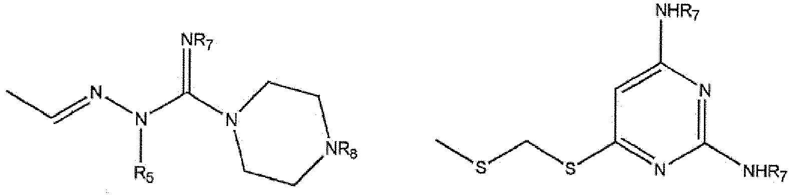 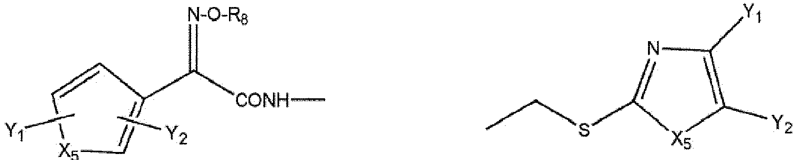 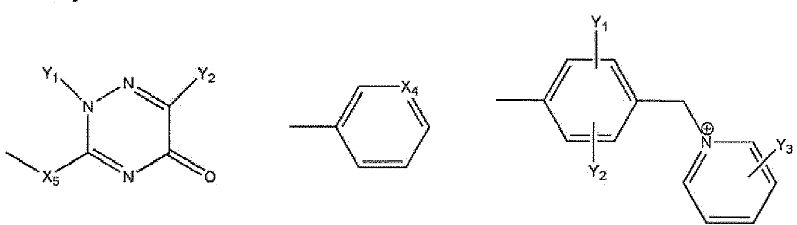 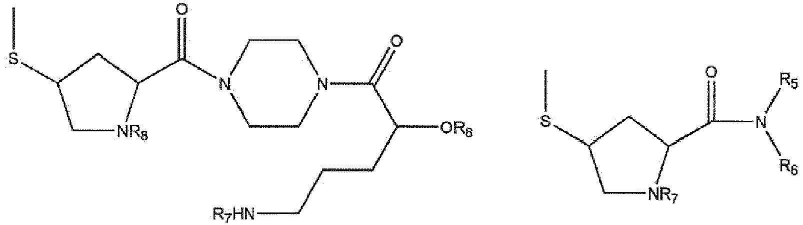 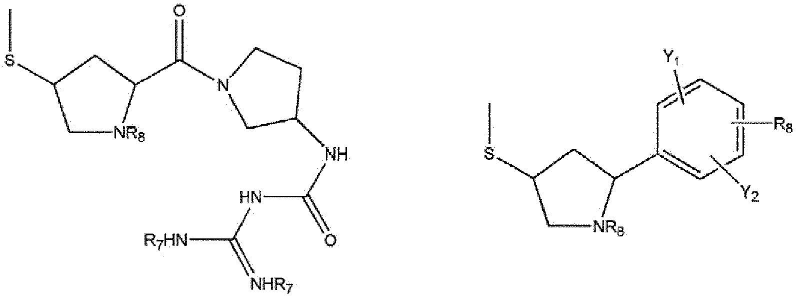 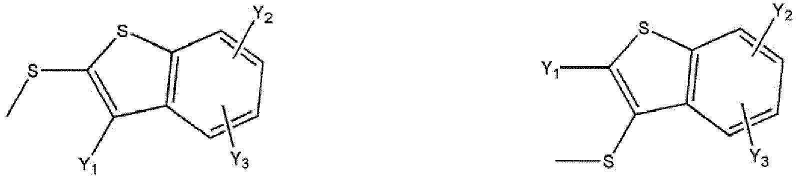 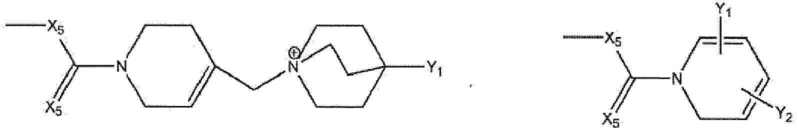 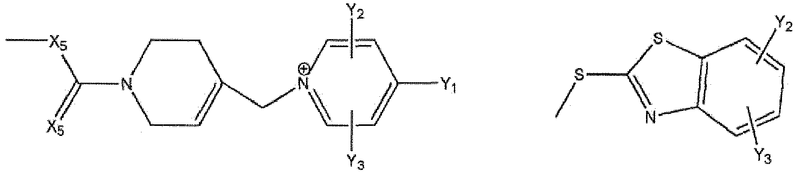 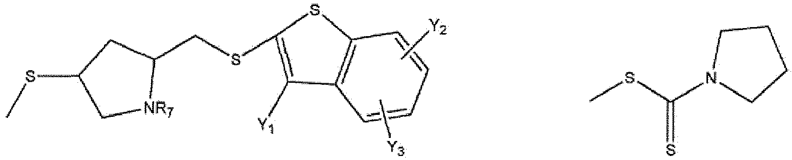 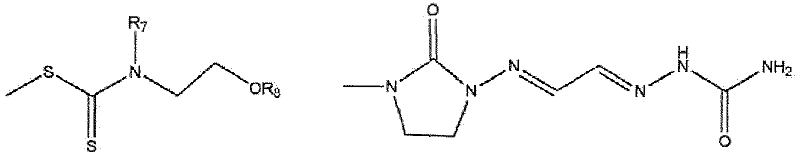 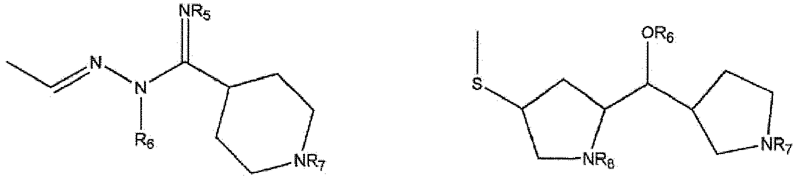 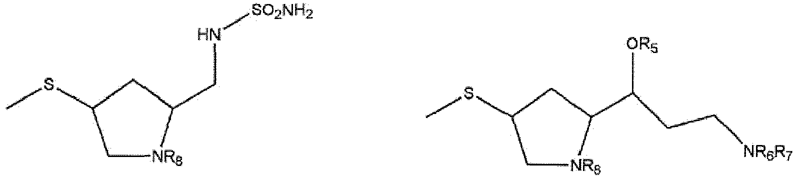 wherein, Z represents S, O, SO, SO₂, NH, CH₂, CHCH₃, or CHCH₂CH₃; R₅ represents H, CONH₂, CH₂CH₂OR_{6,} CH₂CH₂N(CH₃)₂, CH₂CH₂N(CH₂CH₃)_{2,} Cl, F, Br, I, one of any alkyl, alkyloxy, alkenyl or alkynyl residues having 1 to 12 carbon atoms, aryl or heteroaryl residues , CO-W, or W; R₆ represents H, F, Cl, Br, I, Na⁺, K⁺, COR_{5,} one of any alkyl, alkyloxy, alkenyl or alkynyl residues having 1 to 12 carbon atoms, aryl or heteroaryl residues, CO-W, or W; R₇ represents H, CH₃, C₂H₅, CF₃, COCH₃, C₃H₇, C₄H₉, C₅H₁₁, CONH₂, COR₆, COR_{5,} CO-W, or W; R₈ represents H, CH₃, C₂H₅, CF₃, CH₂CH₂F, CH₂CH₂Cl, CH₂CH₂ Br, CH₂CH₂I, CH₂CHF₂,CH₂CF_{3,} CH₂F_{,} CH₂Cl, CH₂Br, CH₂I, CH₂NR₆R₇, CH(NHR₇)CH₂CONH₂, C₃H₇, C₄H₉, C₅H₁₁, CONH₂, CH₂CONH_{2,} CH₂OCONH₂, C(CH₃)₂COOR₆, CH(CH₃)COOR_{6,} CH₂COOR₆, COR₆, COR_{5,} CO-W, or W; X represents O, S, NH, or CH₂; X₂ represents H, CH₃, CH₂(CH₂)ₙOR₈, Cl, F, Br, I, NO₂, CN, CF₃, OCF₃, NH₂, CH₃, C₂H_{5,} CONH₂, CH₂OCONH₂, CH₂COOR₅, CH₂ (CH₂)ₙN(CH₃)₂, CH₂(CH₂)ₙSO₃R₅, C₁₋₈alkyl, C₁₋₈alkylthiol, C₁₋₈alkylamine, or C ₁₋₈alkyloxy; X₃ represents H, CH₃, CH₂(CH₂)ₙOR₈, Cl, F, Br, I, NO₂, CN, CF₃, OCF₃, NH₂, CH₃, C₂H_{5,} CONH₂, CH₂OCONH₂, CH₂COOR₅, CH₂(CH₂)ₙN(CH₃)₂, CH₂(CH₂)ₙSO₃R₅, C₁₋₈alkyl, C₁₋₈alkylthiol, C₁₋₈alkylamine, or C₁₋₈alkyloxy; X₄ represents N, CH , or CY₁ ; X₅ represents CH₂, CHY₁, S, O or NH; Y₁ represents H, OH, OW, OCOW, OCOCH₃, CH₃, C₂H_{5,} C₃H_{7,} CH CH₂COOR₈, OCH₃, OC₂ H₅, CH₃SO₃, NO₂, CN, CF₃, OCF₃, CH₂(CH₂)ₙNR₅R₆, CH₂(CH₂)ₙOR_{6,} CH(CONH₂)NHR₆, CH₂CONH_{2,} F, Br, I, or Cl; Y₂ represents H, OH, OW, OCOW, CH₃, C₂H_{5,} C₃H_{7,} C₄H_{9,} CH₂COOR₈, OCOCH₃, OCH₃, OC₂H₅, CH₃SO₃, NO₂, CN, CF₃, OCF₃, CH₂(CH₂)ₙNR₅R₆, CH₂(CH₂)ₙOR_{6,} CH(CONH₂)NHR₆, CH ₂CONH_{2,} F, Br, I, or Cl; Y₃ represents H, OH, OW, OCOW, OCOCH₃. CH₃, C₂H ₅, C₃H₇, C₄H₉, CH₂COOR₈, OCH₃, OC₂H₅, CH₃SO₃, NO₂, CN, CF₃, OCF₃, CH₂ (CH₂)ₙNR₅R₆, CH₂(CH₂)ₙOR₆, CH(CONH₂)NHR₆, CH₂CONH_{2,} F, Br, I, or Cl; Y ₄ represents H, OH, OW, OCOW, OCOCH₃, CH₃, C₂H₅, C₃H₇, C₄H₉, CH₂COOR₈, OCH₃, OC₂H₅, CH₃SO₃, NO₂, CN, CF₃, OCF₃, CH₂(CH₂)ₙNR₅R₆, CH₂(CH₂)ₙOR_{6,} CH(CONH₂)NHR₆, CH₂CONH_{2,} F, Br, I, or Cl; AA represents any amino acids; n=0, 1, 2, 3, 4, 5, 6,...; R₈- represents: 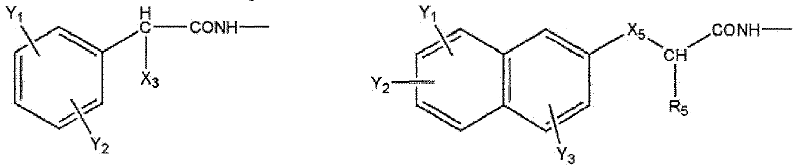 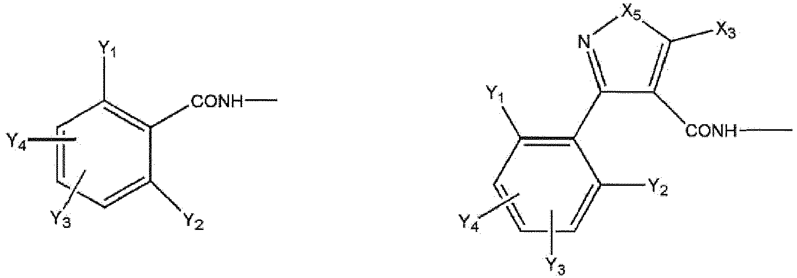 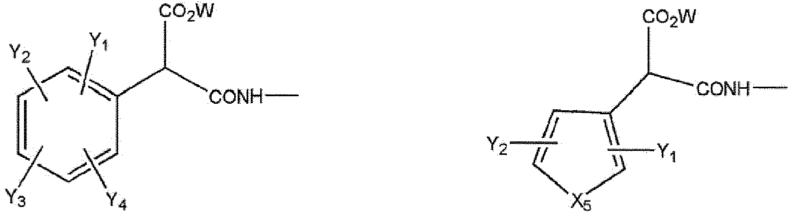 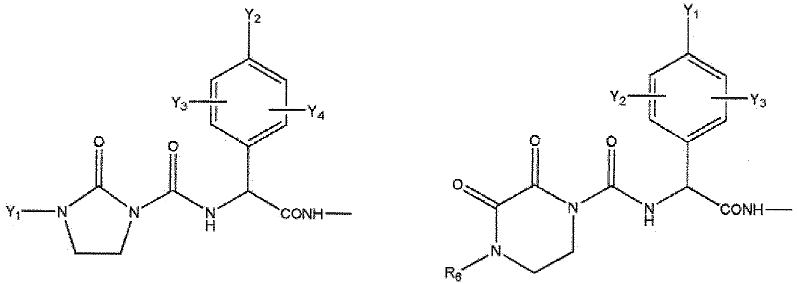 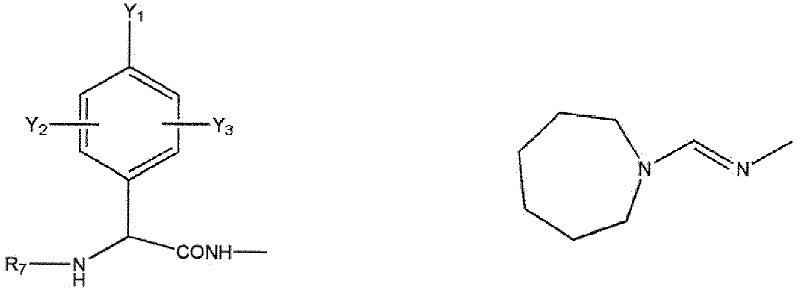 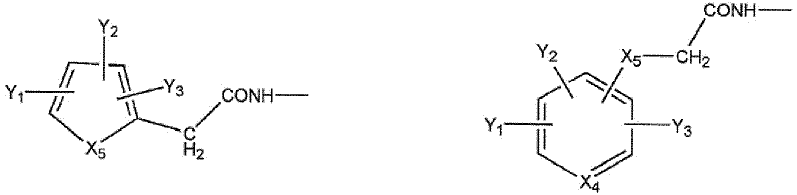 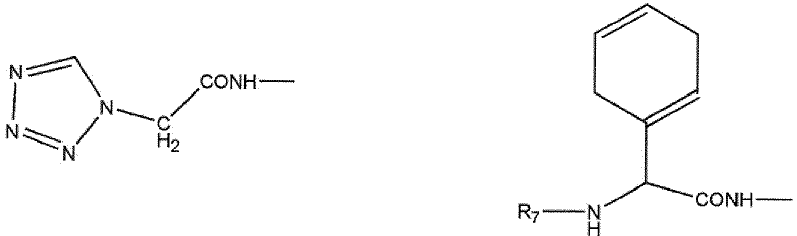 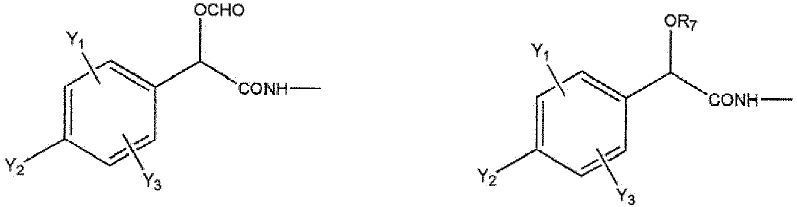 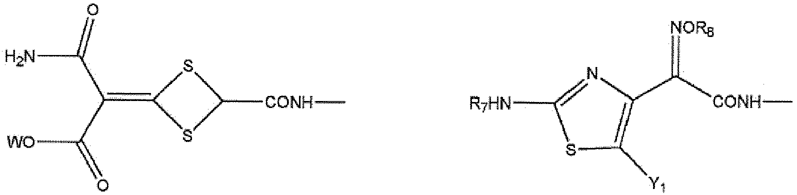 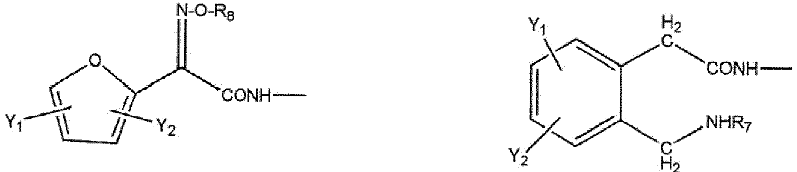 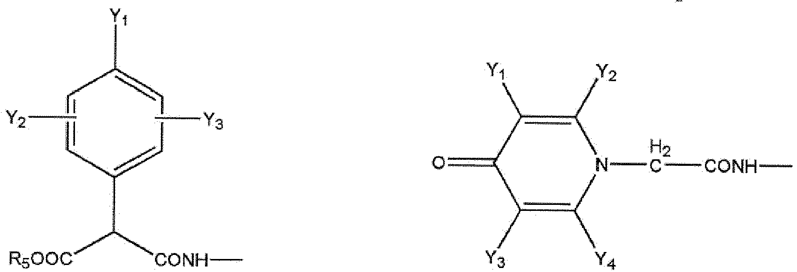 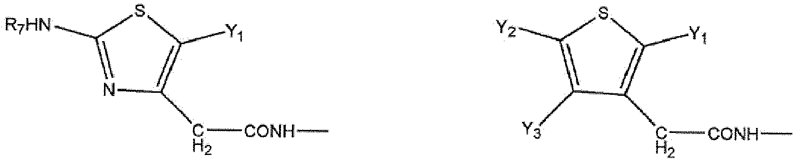 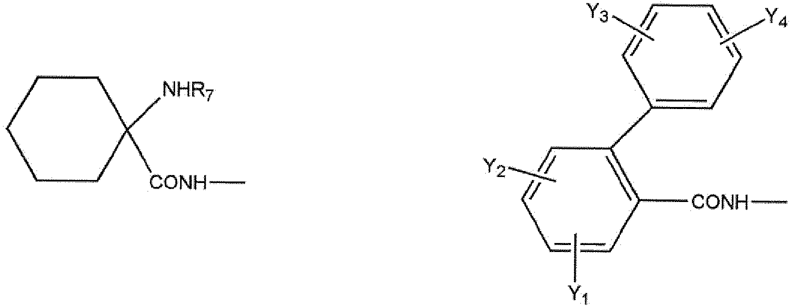 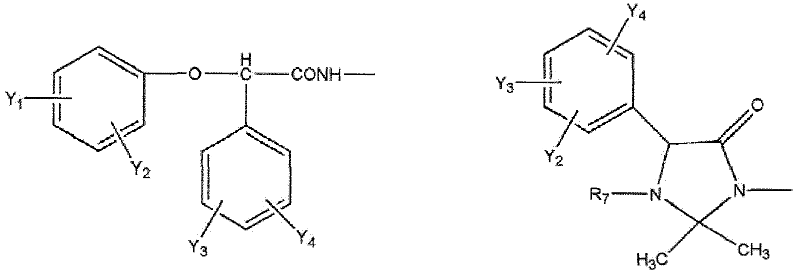 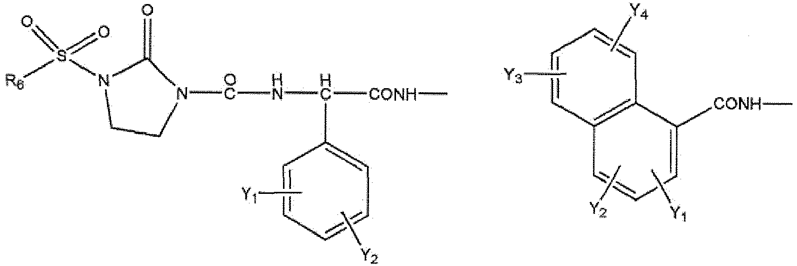 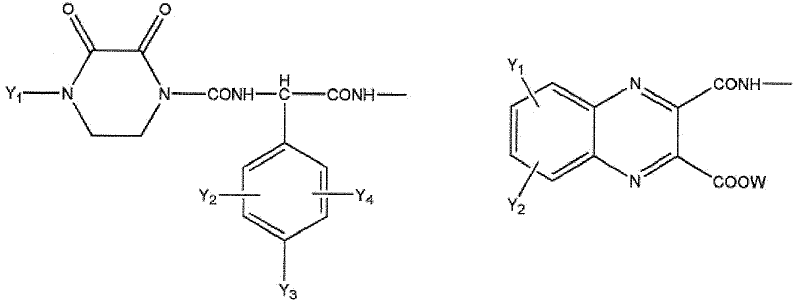 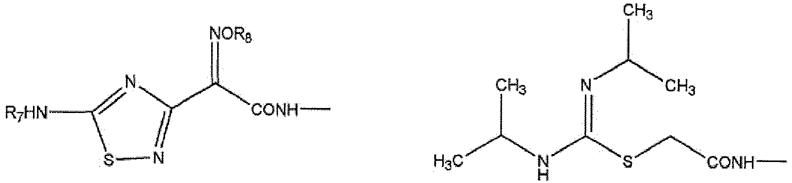 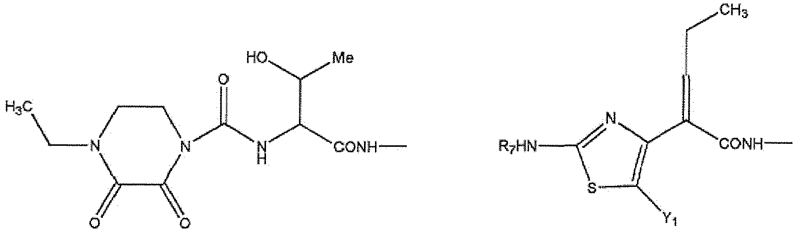 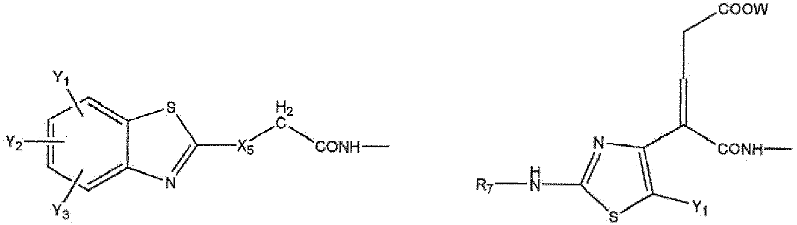 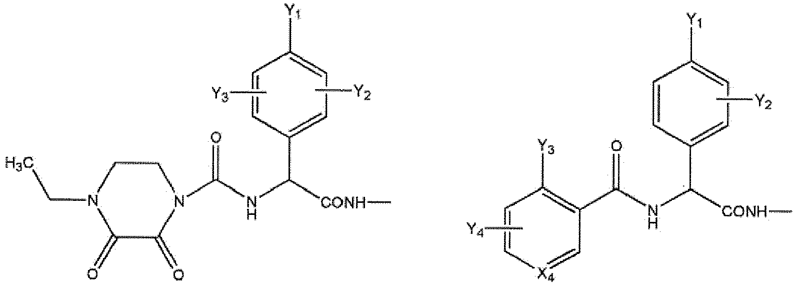 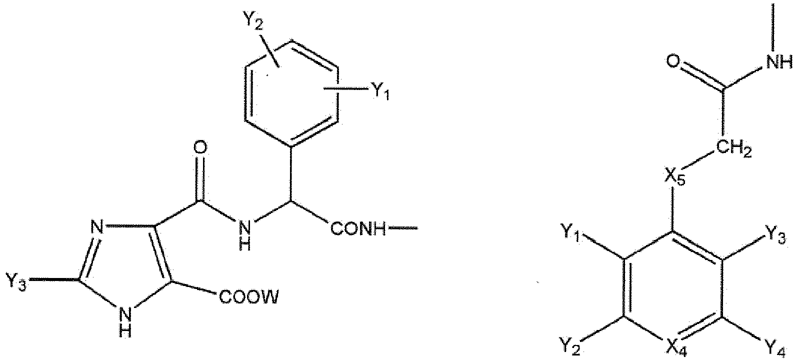 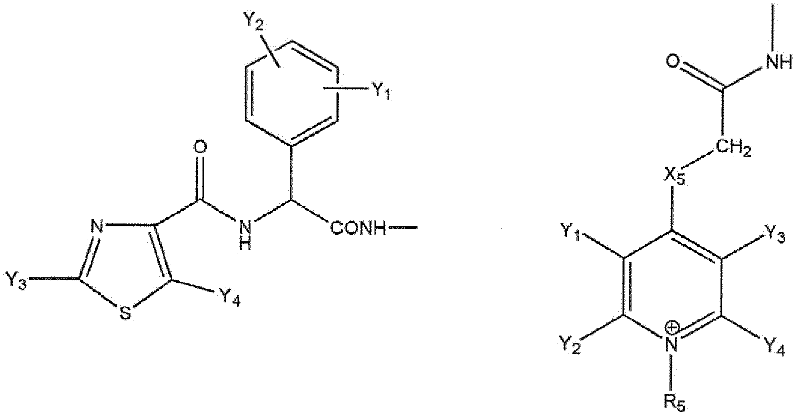 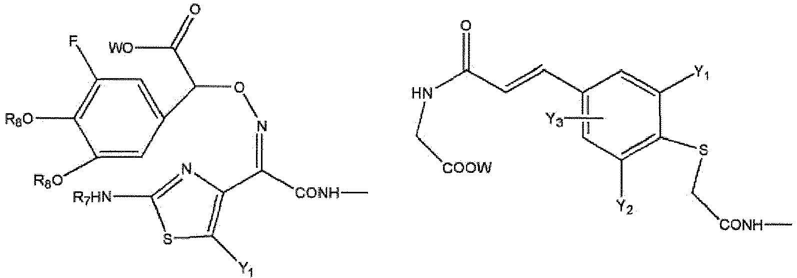 R₆OOCCH(NHR₇)(CH₂)ₙCONH-, R₆OOCCH(NHR₇)(CH₂)ₙSCONH- , CF₃SCH₂ CONH-, CF₃CH₂CONH-, CHF₂SCH₂CONH-, CH₂FSCH₂CONN-, NH₂ COCHFSCH₂CONH-, R₇NHCH(COOW)CH₂SCH₂CONH-, CNCH₂SCH₂ CONH-, CH₃(CH₂)ₙCONH-, R₇N=C(NR₇)CH₂CH₂S-, R₇N=C(NHR₇)NHCO-, CH₃C(Cl)=CHCH₂SCH₂CONH-, (CH₃)₂C(OR₆)-, CNCH₂CONH-, CNCH₂CH₂S-, R₇N=C(NR₇)CH₂CH₂S-, CH₂=CHCH₂SCH₂CONH-, CH₃CH(OH)-, CH₃CH(OR₈ )-, CH₃CH(Y₁)-, (CH₃)₂CH-, CH₃CH₂-, or CH₃(CH₂)ₙCH=CH(CH₂)ₘCONH-. Wherein, n or m= 0, 1, 2, 3, 4, 5, 6, ...; X₃ represents H, N₃, SO₃W, F, Cl, Br, I, OH, OCH₃, OC₂H₅, CH₃, C₂H₅, COOW, OW, or W; X₄ represents N, CH, or CY ₁; X₅ represents CH₂, S, O or NH; R₅ represents H, -CONH₂, CH₂CH₂N(CH₃)₂, CH₂CH₂N(CH₂CH₃)_{2,} CH₂CH₂O R_{6,} Cl, F, Br, I, one of any alkyl, alkyloxyl, alkenyl or alkynyl residues having 1 to 12 carbon atoms, aryl or heteroaryl residue, CO-W, or W; R₆ represents H, F, Cl, Br, I, 2-oxo-1-imidazolidinyl, one of any alkyl, alkyloxyl, alkenyl or alkynyl residues having 1 to 12 carbon atoms, aryl (phenyl, 5-indanyl, 2,3-dihydro-1H-inden-5-yl, et al.) or heteroaryl (4-hydroxy-1,5-naphthyridin-3-yl, et al.) residues; R₇ represents H, F, Cl, Br, I, CH₃NHCOCH₂CH(NHR₈)CO), R₅N=C(NHR₆)NHCO-, COCH₃, COR₆, PO(OR₅ )OR₆, one of any alkyl, alkyloxyl, alkenyl or alkynyl residues having 1 to 12 carbon atoms, aryl or heteroaryl residues, CO-W, or W; R₈ represents H, F, Cl, Br, I, CH₃, C₂H₅, CF₃, CH₂CH₂F, CH₂CH₂Cl, CH₂CH₂Br, CH₂CH₂I, CH₂CHF₂, CH₂CF_{3,} CH₂F_{,} CH₂Cl, CH₂Br, CH₂I, C₃H₇, C₄H₉, C₅H₁₁, COR_{6,} CONH₂, CH₂ OCONH₂, PO(OR₅)OR₆, C(CH₃)₂COOR₆, CH(CH₃)COOR_{6,} CH₂COOR₆, CO-W, or W; Y₁ represents H, OH, OW, OCOW, OCOCH₃, CH₃, C₂H_{5,} C₃H_{7,} C₄H_{9,} SO₃ R₆, CH₂COOR₈, OCH₃, OC₂H₅, CH₃SO₃, NO₂, CN, CF₃, OCF₃, CH=CHCONHCH₂COOW, CH₂(CH₂)ₙNR₅R₆, CH₂(CH₂)ₙOR_{6,} CH(CONH₂)NHR ₆, COOR₅, F, Br, I, or Cl; Y₂ represents H, OH, OW, OCOW, CH₃, C₂H_{5,} C₃H_{7,} C ₄H_{9,} CH₂COOR₈, OCOCH₃, OCH₃, OC₂H₅, CH₃SO₃, NO₂, CN, CF₃, OCF₃, CH=CHCONHCH₂COOW, CH₂(CH₂)ₙNR₅R₆, CH₂(CH₂)ₙOR_{6,} CH(CONH₂)NHR ₆, SO₃R₆, COOR₅, F, Br, I, or Cl; Y₃ represents H, OH, OW, OCOW, OCOCH₃, CH₃,C₂H_{5,} C₃H_{7,} C₄H_{9,} CH₂COOR₈, OCH₃, OC₂H₅, CH₃SO₃,NO₂,CN,CF₃,OCF ₃, CH=CHCONHCH₂COOW, CH₂(CH₂)ₙNR₅R₆, CH₂(CH₂)ₙOR_{6,} CH(CONH₂ )NHR_{6,} SO₃R₆, COOR₅, F, Br, I, or Cl; Y₄ represents H, OH, OW, OCOW, OCOCH₃, CH₃, C₂H₅ C₃H_{7,} C₄H_{9,} CH₂COOR₈, OCH₃, OC₂H₅,CH₃SO₃, NO₂, NR₈ COR₅, SO₂NR₅R₈, COR₅, SR₅, CN, CF₃, OCF₃, CH=CHCONHCH₂COOW, CH₂ (CH₂)ₙNR₅R₆, CH₂(CH₂)ₙ OR_{6,} CH(CONH₂)NHR₆, SO₃R₆, COOR₅, F, Br, I, or Cl; -W represents -H, one of any alkyl, alkyloxy, alkenyl or alkynyl residues having 1 to 12 carbon atoms, aryl or heteroaryl residues, or one of the following residues: 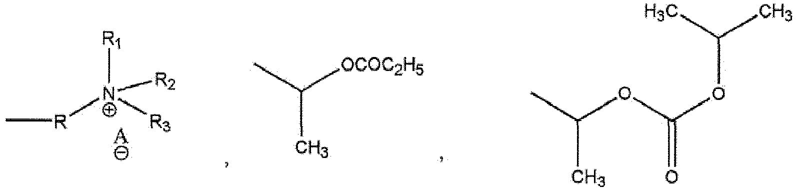 wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, or aryl and heteroaryl residues; R represents a branched or straight chain, -(CH₂)ₙ-, aryl residues or heteroaryl residues; n = 0, 1, 2, 3, 4, 5, 6, 7, 8, ...; R₈ and Y taken together are R₆OCH₂C(R₅ )=; A⁻ represents Cl⁻, Br⁻, F⁻, I⁻, AcO⁻, citrate, or any negative ions. All double bond can be cis or trans. All R, R₁, R₂, R₃, R₄, R₅, R₆, R₇, R₈, -(CH₂)ₙ -, or -(CH₂)ₘ - groups are branched or straight chains and can include C, H, O, S, or N atoms and may have single, double, and triple bonds. Any CH₂ groups can be replaced with O, S, or NH.
[2] The design of transdermal delivery systems of beta-lactam antibiotics. The principles for designing the prodrugs of beta-lactam antibiotics are: (1) The prodrug must have a lipophilic portion and a primary, secondary, or tertiary amine group, a quanidino, or monoprotected quanidino group that exists in the protonated form (hydrophilic portion) at physiological pH. (2) Every prodrug of beta-lactam antibiotics should have only one or two (preferably one) primary, secondary, or tertiary amine group, a quanidino, or monoprotected quanidino group that exists in the protonated form (hydrophilic portion) at physiological pH. (3) The primary, secondary, or tertiary amine group, quanidino, or monoprotected quanidino groups can be on any part of the compound. The design in which one end of the compound is the positive charge and other end is the lipophilic portion is preferable. (4) Carboxyl groups, amino groups, guanidine groups or other hydrophilic groups can be protected with an alkyl, aryl, or heteroaryl ester or amide group to make beta-lactam antibiotics more lipophilic.
[3] Processes for the preparation of compounds of the general formula (1) 'Structure 1' according to embodiment 1, wherein the compounds can be prepared from beta-lactam antibiotics (free acids), by reaction with compounds of the general formula (2) 'Structure 2' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)- N,N,N',N'-tetramethyluronium hexafluorophosphate, Benzotriazol-1-yl-oxy-tris (dimethylamino)phosphonium hexafluorophosphate, et al. 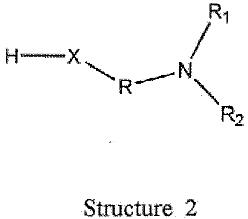 Wherein, R₁ represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R represents a branched or straight chain, -(CH₂)ₙ-, aryl residues or heteroaryl residues; n = 0, 1, 2, 3, 4, 5, 6, 7, 8, ....; X represents O, S, or NH.
[4] Processes for the preparation of compounds of the general formula (1) 'Structure 1' according to embodiment 1, wherein the compounds can be prepared from metal salts, organic base salts, or immobilized base salts of beta-lactam antibiotics, by reaction with compounds of the general formula (3) 'Structure 3'. 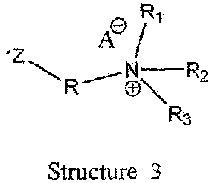 Wherein, R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; Z represents F, Cl, Br, I, methylsulphonyl or p-toluenesulphonyl; A represents Cl⁻, Br⁻, F⁻, I⁻, AcO⁻, citrate, or any negative ions; and n=0,1,2,3,4,5...; R represents a branched or straight chain, -(CH₂)ₙ-, aryl or heteroaryl residues; n = 0, 1, 2, 3, 4, 5, 6, 7, 8, ....
[5] A compound or compounds (including penethamate hydriodide) of the general formula (1)'Structure 1' or a composition comprising of at least one compound (including penethamate hydriodide) of the general formula (1) 'Structure 1', as an active ingredient, according to embodiment 1, be administered orally, transdermally, or by injection for treating any beta-lactam antibiotics-treatable conditions in humans or animals. The beta-lactam antibiotics-treatable conditions include, but are not limited to infections of the upper and lower respiratory tract, acute respiratory distress, pneumonia, meningitis, septic shock, septicemia, otitis media and sinusitis, meningicoccal meningitis and brain abscess, meningococcal and pneumococcal septicemia, streptococcal endocarditis, pelvic inflammatory disease, gonorrhea, syphilis, lyme disease, gas gangrene, tetanus, skin and related soft tissue, gastrointestinal tract, urinary tract, bones and joints, as well as septicemias and endocarditis and intra-abdominal and bile tract infections.
[6] Methods for treating any beta-lactam antibiotics-treatable conditions in humans or animals by administering transdermally to any part of body (in the form of a solution, spray, lotion, ointment, emulsion or gel) to deliver therapeutically effective plasma levels of a compound or compounds (including penethamate hydriodide) of the general formula (1) 'Structure 1' or a composition comprising of at least one compound (including penethamate hydriodide) of the general formula (1) 'Structure 1', as an active ingredient, according to embodiment 1.
[7] Methods for topically treating skin, brain, breast, prostate gland, the upper and lower respiratory tract, urinary tract, bones and joints, genital area, and other infections in humans or animals by administering transdermally to the inflamed area or the nearby area a therapeutically effective amount of a compound or compounds (including penethamate hydriodide) of the general formula (1) 'Structure 1' or a composition comprising at least one compound (including penethamate hydriodide) of the general formula (1) 'Structure 1', as an active ingredient, according to embodiment 1.
[8] Transdermal therapeutic application systems of a compound or compounds (including penethamate hydriodide) of the general formula (1) 'Structure 1' or a composition comprising of at least one compound (including penethamate hydriodide) of the general formula (2) 'Structure 2', as an active ingredient, according to embodiment 1, for treating any beta-lactam antibiotics-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables any beta-lactam antibiotics to reach constantly optimal therapeutic blood levels to increase effectiveness and reduce the side effects of any beta-lactam antibiotics.

## Claims

1. A compound of Structure 1 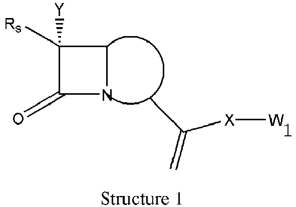 wherein
X is selected from O, S, CH₂, and NH;
Y is selected from H, OH, NHCHO, OCOCH₃, OCH₃, OC₂H₅, CH₃SO₃, NO₂, CN, CF₃, OCF₃, F, Br, I, and Cl; 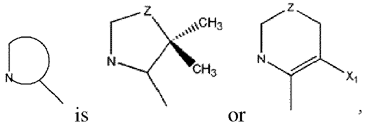
wherein
Z is S;
-X₁ is selected from -H, -OH, -OCH₃, -OC₂H₅, OC₃H₇, -CONH₂, -CH₂OCONH₂, - CH₂OCOCH₃, -OCOCH₃, -CH₂OCOCH₃, -CH₂OCH₃, -CH₃, -C₂H₅, -C₃H₇, -C₄H₉, C₅H₁₁, C₆H₁₃, -Cl, -F, -Br, -I, -HC=CHCH₃, -HC=CH₂, -CH₂OCH₃, 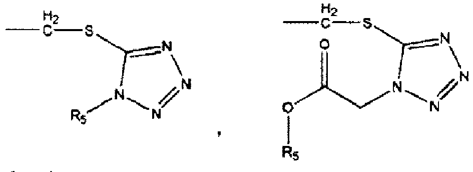
wherein
R₅ is selected from H, CONH₂, CH₂CH₂N(CH₃)₂, CH₂CH₂N(CH₂CH₃)₂, Cl, F, Br, I, alkyl residues having 1 to 12 carbon atoms, alkyloxyl residues having 1 to 12 carbon atoms, alkenyl residues having up to 12 carbon atoms, and alkynyl residues having up to 12 carbon atoms;
Rₛ- is selected from: 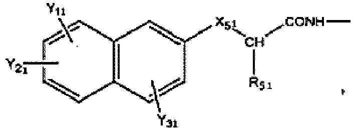 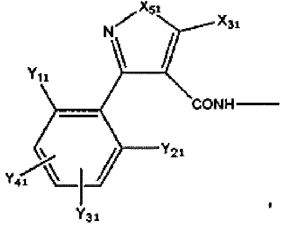 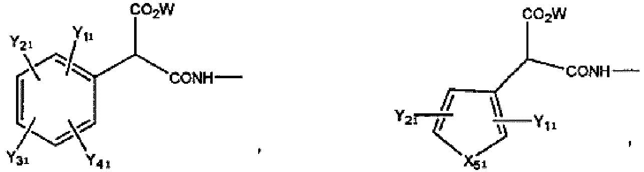 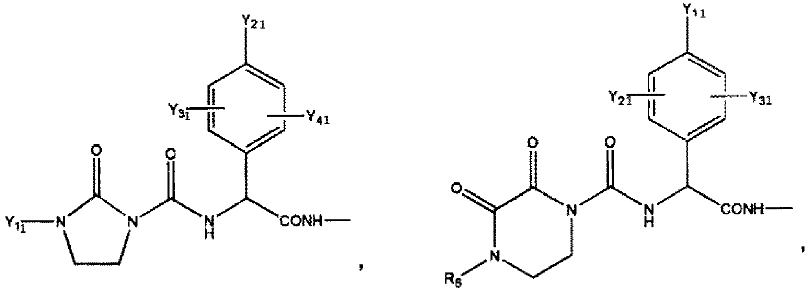 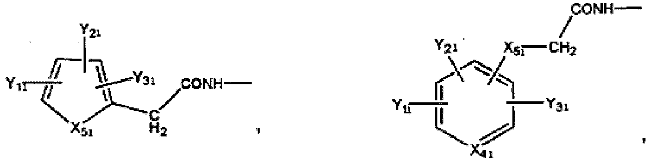 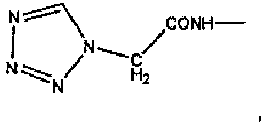 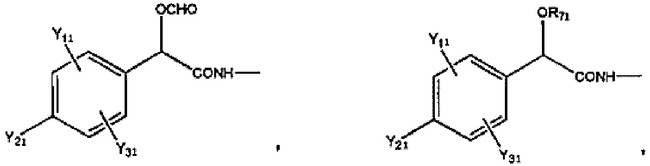 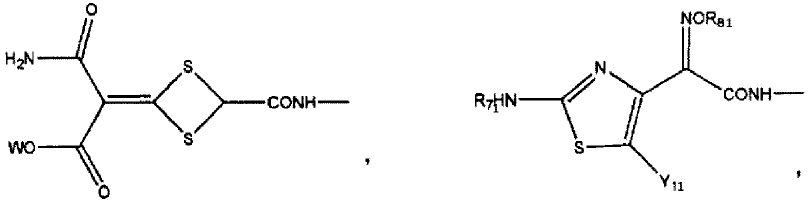 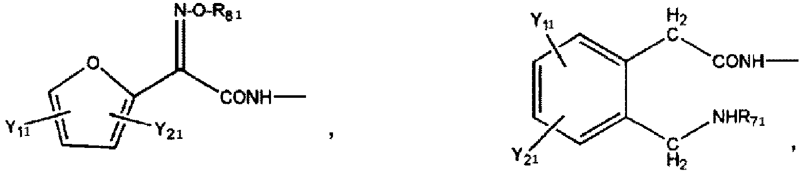 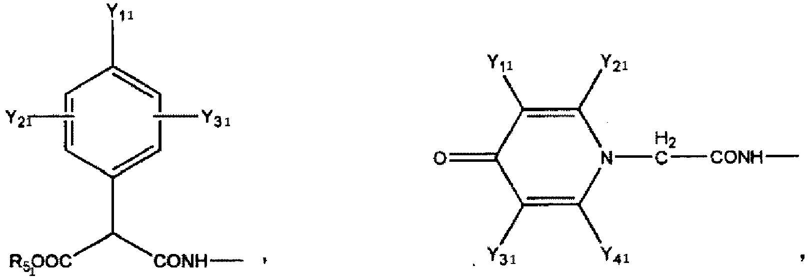 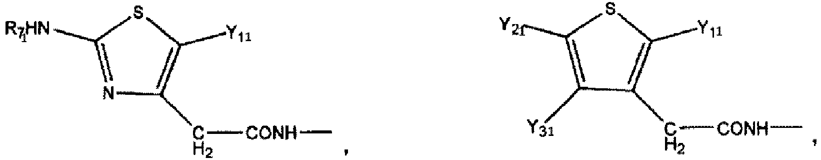 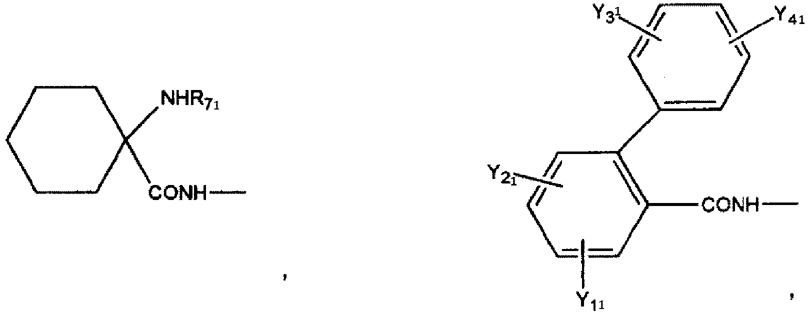 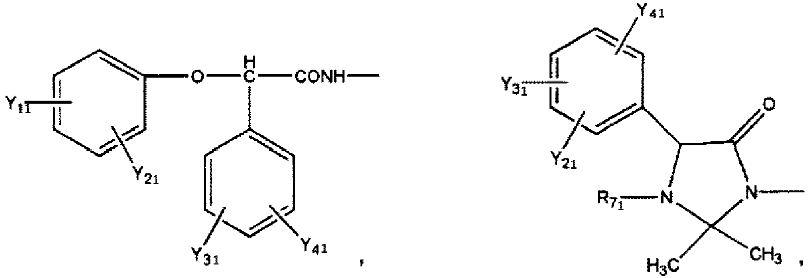 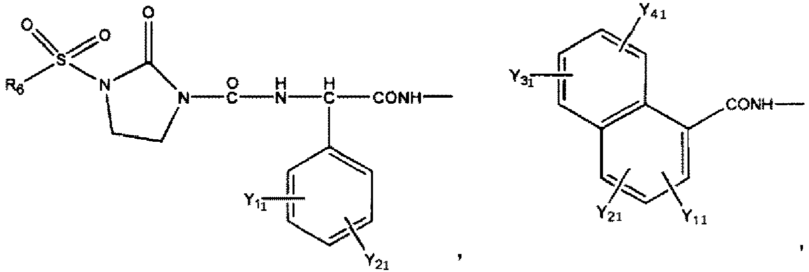 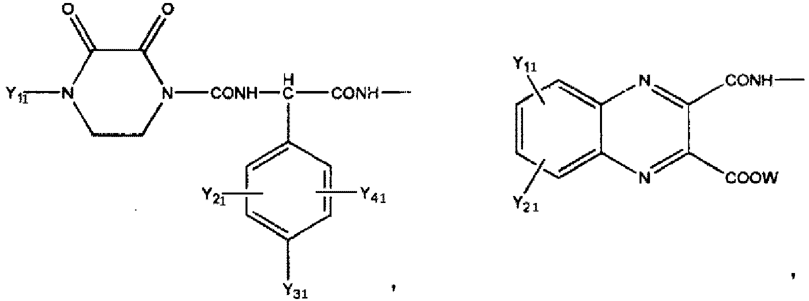 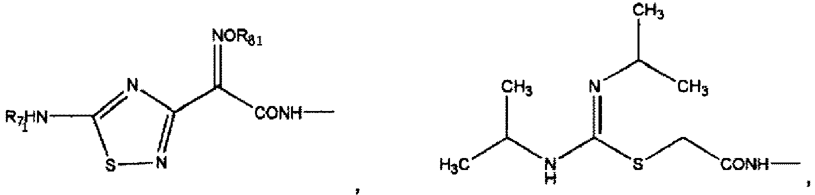 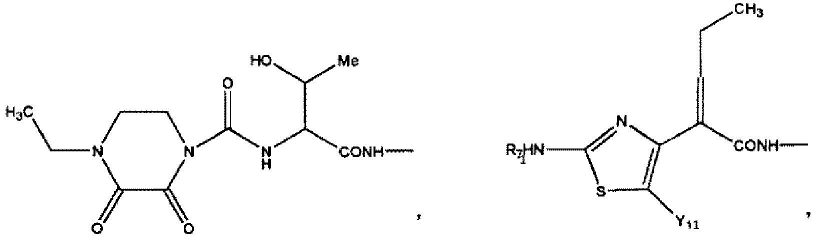 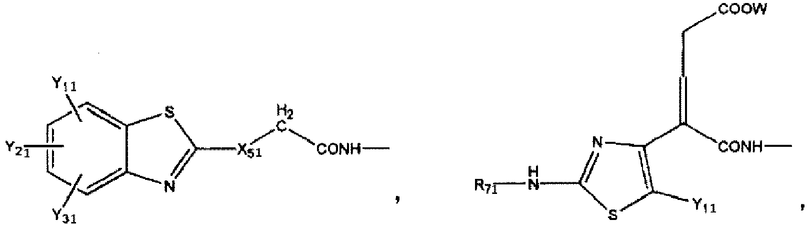 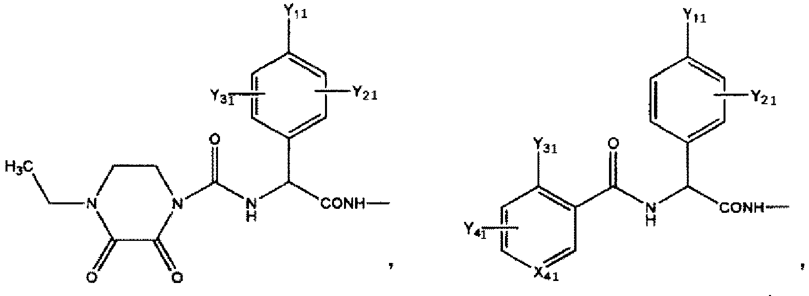 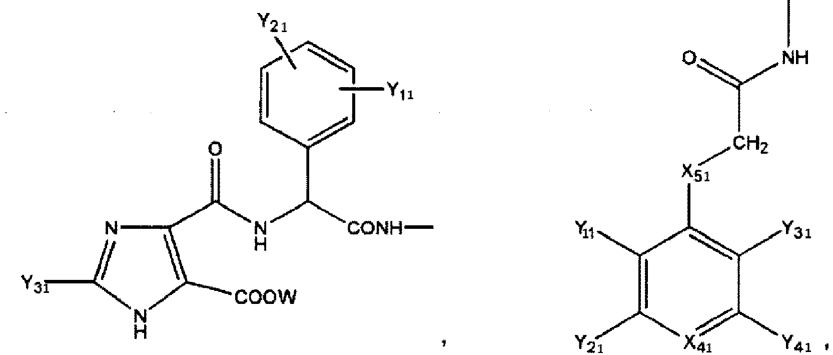 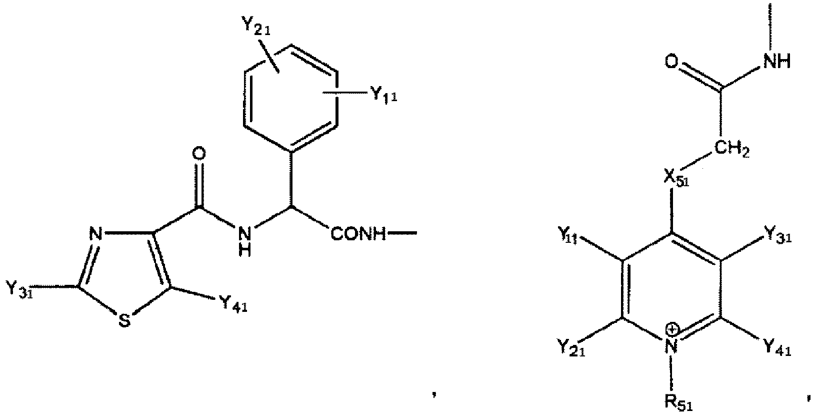 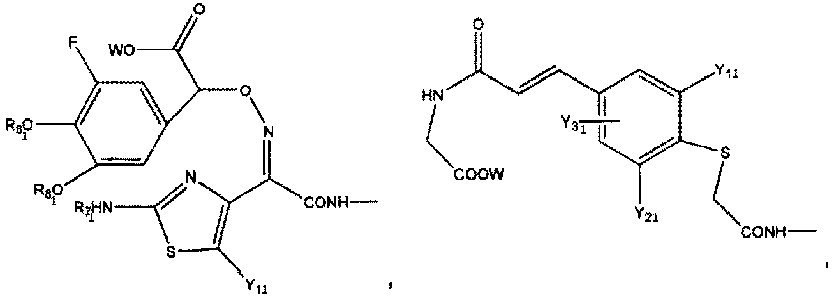 R₆₁OOCCH(NHR₇₁)(CH₂)ₙCONH-, R₆₁OOCCH(NHR₇)(CH₂)ₙSCONH-, CF₃SCH₂CONH-, CF₃CH₂CONH-, CHF₂SCH₂CONH-, CH₂FSCH₂CONH-, NH₂COCHFSCH₂CONH-, R₇₁NHCH(COOW), CH₂SCH₂CONH-, CNCH₂SCH₂CONH-, CH₃(CH₂)ₙCONH-, R₇₁N=C(NR₇₁)CH₂CH₂S-, R₇₁N=C(NHR₇₁)NHCO-, CH₃C(Cl)=CHCH₂SCH₂CONH-, (CH₃)₂C(OR₆₁)-, CNCH₂CONH-, CNCH₂CH₂S-, R₇₁N=C(NR₇₁)CH₂CH₂S-, CH₂=CHCH₂SCH₂CONH-, CH₃CH(OH)-, CH₃CH(OR₈₁)-, CH₃CH(Y₁₁)-, (CH₃)₂CH-, CH₃CH₂-, and CH₃(CH₂)ₙCH=CH(CH₂)ₘCONH-, or Rₛ and Y taken together are
R₆₁OCH₂C(R₅₁)=, wherein
n and m are each independently selected from 0, 1, 2, 3, 4, 5, and 6;
X₃₁ is selected from H, N₃, SO₃W, F, Cl, Br, I, OH, OCH₃, OC₂H₅, CH₃, and C₂H₅;
X₄₁ is N or CH;
X₅₁ is selected from CH₂, S, O, and NH;
R₅₁ is selected from H, -CONH₂, CH₂CH₂N(CH₃)₂, CH₂CH₂N(CH₂CH₃)₂, CH₂CH₂OR₆₁, Cl,
F, Br, I, alkyl residues having 1 to 12 carbon atoms, alkyloxyl residues having 1 to 12 carbon atoms, alkenyl residues having up to 12 carbon atoms, alkynyl residues having up to 12 carbon atoms, aryl residues, and heteroaryl residues;
R₆₁ is selected from H, F, Cl, Br, I, 2-oxo-1-imidazolidinyl, alkyl residues having 1 to 12 carbon atoms, alkyloxyl residues having 1 to 12 carbon atoms, alkenyl residues having up to 12 carbon atoms, alkynyl residues having up to 12 carbon atoms, aryl (phenyl, 5-indanyl, 2,3-dihydro-1H-inden-5-yl) residues and heteroaryl (4-hydroxy-1,5-naphthyridin-3-yl) residues;
R₇₁ is selected from H, F, Cl, Br, I, CH₃NHCOCH₂CH(NHR₈₁)CO, R₅₁N=C(NHR₆₁)NHCO-, COCH₃, COR₆₁, PO(OR₅₁)OR₆₁, alkyl residues having 1 to 12 carbon atoms, alkyloxyl residues having 1 to 12 carbon atoms, alkenyl residues having 1 to 12 carbon atoms,alkynyl residues having 1 to 12 carbon atoms, aryl residues, and heteroaryl residues;
R₈₁ is selected from the group consisting of H, F, Cl, Br, I, CH₃, C₂H₅, CF₃, CH₂CH₂ F, CH₂CH₂Cl, CH₂CH₂Br, CH₂CH₂I, CH₂CHF₂, CH₂CF₃, CH₂F, CH₂Cl, CH₂Br, CH₂I, C₃H₇, C₄H₉, C₅H₁₁, CONH₂, and CH₂OCONH₂;
Y₁₁, Y₂₁, Y₃₁ and Y₄₁ are each independently selected from H, OH, OW, OCOW, OCOCH₃, CH₃, C₂H₅, C₃H₇, C₄H₉, SO₃R₆₁, CH₂COOR₈₁, OCH₃, OC₂H₅, CH₃SO₃, NO₂, CN, CF₃, OCF₃, CH=CHCONHCH₂COOW, CH₂(CH₂)ₙNR₅₁R₆₁, CH₂(CH₂)ₙOR₆₁, CH(CONH₂)NHR₆₁, COOR₅₁, F, Br, I, and Cl;
-W is selected from alkyl residues having 1 to 12 carbon atoms, alkyloxy residues having 1 to 12 carbon atoms, alkenyl residues having up to 12 carbon atoms, alkynyl residues having up to 12 carbon atoms, aryl residues, heteroaryl residues 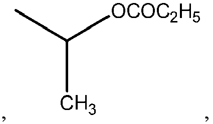 and 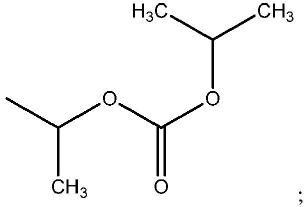
-W₁ is -RN⁺R₁R₂HA;
R₁, and R₂ are independently selected from alkyl residues having 1 to 12 carbon atoms, alkyloxyl residue having 1 to 12 carbon atoms, alkenyl residues having up to 12 carbon atoms, and alkynyl residues having up to 12 carbon atoms;
R is a branched or straight chain, -(CH₂)ₙ₁-;
n1 is selected from 0, 1, 2, 3, 4, 5, 6, 7, and 8; and
A⁻ is a negative ion selected from the group consisting of chloride, bromide, fluoride, acetate, and citrate.

2. A compound according to claim 1, which is:
6-phenoxyacetacetamidopenicillanic acid 2-diethylaminoethyl ester.HCl;
allylmercaptomethylpenicillinic acid 2-diethylaminoethyl ester.HCl;
6-[D(-)-α-acetamidophenylacetamidopenicillinic acid 2-diethylaminoethyl ester.HCl;
D-α-[(imidazolidin-2-on-1-yl)carbonylamino]benzylpenicillin 2-diethylaminoethyl ester.HCl;
6-[3-(2,6-dichlorophenyl)-5-methyl-4-isoxazolecarboxamido]penicillinic acid 2-diethylaminoethyl ester.HCl;
6R-[2-[3-(methylsulfonyl)-2-oxo-1-imidazolidinecarboxamido]-2-phenylacetamido]penicillinic acid 2-diethylaminoethyl ester.HCl;
6-(5-methyl-3-phenyl-2-isoxazoline-4-carboxamido)penicillinic acid 2-diethylaminoethyl ester.HCl;
6-[3-(o-chlorophenyl)-5-methyl-4-isoxazolecarboxamido]penicillinic acid 2-diethylaminoethyl ester.HCl;
6-D(-)-α-(4-ethyl-2,3-dioxo-1-piperazinylcarbonylamino)-α-phenylacetamidopenicillinic acid 2-diethylaminoethyl ester.HCl;
7-(2-thienylacetamido)cephalosporanic acid 2-diethylaminoethyl ester.HCl;
7-[(hydroxyphenylacetyl)amino]-3-[(1-methyl-1H-tetrazol-5-yl)thio]methyl]-8-oxo-5-thia-1-azabicyclo[4.2.0]oct-2-ene-2-carboxylic acid 2-diethylaminoethyl ester.HCl;
3-[[(aminocarbonyl)oxy]methyl]-7-[[2-furanyl(methoxyimino)acetyl] amino]-8-oxo-5-thia-1-azabicyclo[4.2.0]oct-2-ene-2-carboxylic acid 2-diethylaminoethyl ester.HCl;
3-[[(aminocarbonyl)oxy]methyl]-7-methoxy-8-oxo-7-[(2-thienylacetyl)amino]-5-thia-1-azabicyclo[4.2.0]oct-2-ene-2-carboxylic acid 2-diethylaminoethyl ester.HCl;
7-[[[2-(acetylaminomethyl)phenyl]acetyl]amino]-3-[[[1-(ethoxylcarbonylmethyl)-1*H*-tetrazol-5-yl]thio]methyl]-8-oxo-5-thia-1-azabicyclo[4.2.0]oct-2-ene-2-carboxylic acid 2-diethylaminoethyl ester.HCl;
7-[(acetylaminophenylacetyl)amino]-3-chloro-8-oxo-5-thia-1-azabicyclo[4.2.0]oct-2-ene-2-carboxylic acid 2-diethylaminoethyl ester.HCl;
3-[(acetyloxy)methyl]-7-[[(2-acetylamino-4-thiazolyl)(methoxyimino)acetyl]amino]-8-oxo-5-thia-1-azabicyclo[4.2.0]oct-2-ene-2-carboxylic acid 2-diethylaminoethyl ester.HCl;
7-[[(2-acetylamino-4-thiazolyl)(methoxyimino)acetyl]amino]-8-oxo-5-thia-1-azabicyclo[4.2.0]oct-2-ene-2-carboxylic acid 2-diethylaminoethyl ester.HCl;
7-[[[[[(4-ethyl-2,3-dioxo-1-piperazinyl)carbonyl]amino]((4-acetoxyphenyl)acetyl]amino]-3-[[(1-methyl-1H-tetrazol-5-yl)thio]methyl]-8-oxo-5-thia-1-azabicyclo[[4.2.0]oct-2-ene-2-carboxylic acid 2-diethylaminoethyl ester.HCl;
7-[2-(2-acetylamino-4-thiazolyl)-2-((Z)-methoxyimino)acetamido]-3-(methoxymethyl)-8-oxo-5-thia-1-azabicyclo[4.2.0]oct-2-ene-2-carboxylic acid 2-diethylaminoethyl ester.HCl;
7-[2-(2-acetylamino-4-thiazolyl)-2-((Z)-ethoxycarbonylmethoxy)imino]acetamido]-3-(vinyl)-8-oxo-5-thia-1-azabicyclo[4.2.0]oct-2-ene-2-carboxylic acid 2-diethylaminoethyl ester.HCl;

3. A composition comprising one or more compounds according to claim 1 or 2.

4. A compound according to claim 1 or 2 or the composition according to claim 3 for use in the treatment of any beta-lactam antibiotics-treatable condition in a human or animal, wherein the compound or composition is administered via transdermal administration.

5. The compound or composition for use according to claim 4, wherein the beta-lactam antibiotics-treatable condition is selected from infections of the upper and lower respiratory tract, acute respiratory distress, pneumonia, meningitis, septic shock, septicemia, otitis media and sinusitis, meningicoccal meningitis and pneumococcal septicemia, streptococcal endocarditis, pelvix inflammatory disease, gonorrhea, syphilis, lyme disease, gas gangrene, tetanus, skin and related soft tissue, gastrointestinal tract, urinary tract, bones and joints, as well as septimicemias and endocarditis and intra-abdominal and bile tract infections.

6. The compound or composition for use according to claim 4, wherein the compound or composition is in the form of a solution, spray, lotion, ointment, emulsion, or gel.

7. The compound or composition for use according to claim 4, wherein the compound or composition is transdermally administered to the skin, brain, breast, prostate gland, the upper and lower respiratory tract, urinary tract, bones and joints, genital area, or at or nearby an inflammed area.

8. A transdermal therapeutic application system comprising a compound according to claim 1 or 2 or a composition according to claim 3 for use in the treatment of any beta-lactam antibiotics-treatable condition in a human or animal.

9. The transdermal therapeutic application system for use according to claim 8, **characterized in that** the
system is a bandage or a patch comprising one active substance-containing matrix layer and an impermeable backing layer.

10. The transdermal therapeutic application system for use according to claims 8 or 9, **characterized by**
having an active substance reservoir, which has a permeable bottom facing the skin.

11. The transdermal therapeutic application system for use according to any one of claims 8 to 10,
**characterized by** a controlling means controlling the rate of release.

## Patentansprüche

1. Verbindung der Struktur 1 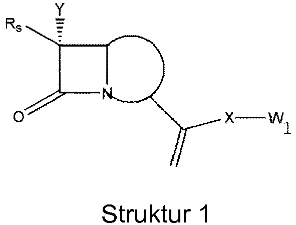 wobei
X ausgewählt ist aus O, S, CH₂ und NH;
Y ausgewählt ist aus H, OH, NHCHO, OCOCH₃, OCH₃, OC₂H₅, CH₃SO₃, NO₂, CN, CF₃, OCF₃, F, Br, I und Cl; 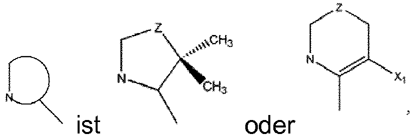
wobei
Z S ist;
-X₁ ausgewählt ist aus -H, -OH, -OCH₃, -OC₂H₅, OC₃H₇, -CONH₂, -CH₂OCONH₂, -CH₂OCOCH₃, -OCOCH₃, -CH₂OCOCH₃, -CH₂OCH₃, -CH₃, -C₂H₅, -C₃H₇, -C₄H₉, C₅H₁₁, C₆H₁₃, -Cl, -F, -Br, -I, -HC=CHCH₃, -HC=CH₂, -CH₂OCH₃, 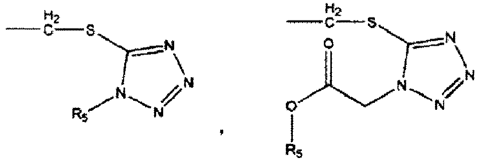
wobei
R₅ ausgewählt ist aus H, CONH₂, CH₂CH₂N(CH₃)₂, CH₂CH₂N(CH₂CH₃)₂, Cl, F, Br, I, Alkylresten mit 1 bis 12 Kohlenstoffatomen, Alkyloxylresten mit 1 bis 12 Kohlenstoffatomen, Alkenylresten mit bis zu 12 Kohlenstoffatomen und Alkynylresten mit bis zu 12 Kohlenstoffatomen;
Rₛ- ausgewählt ist aus: 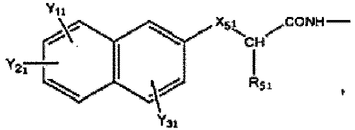 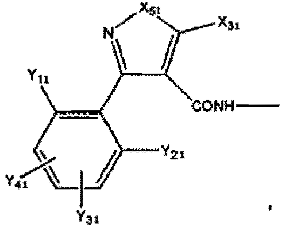 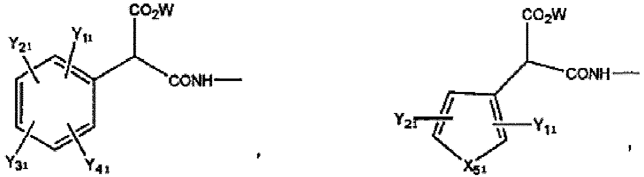 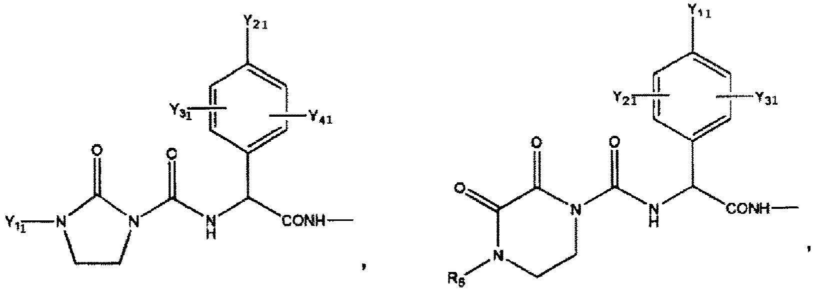 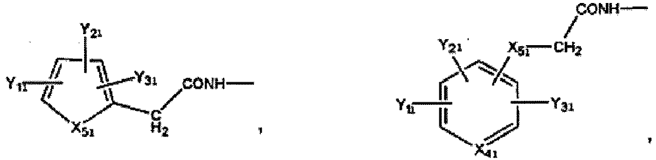 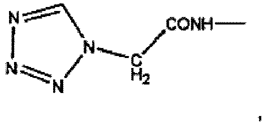 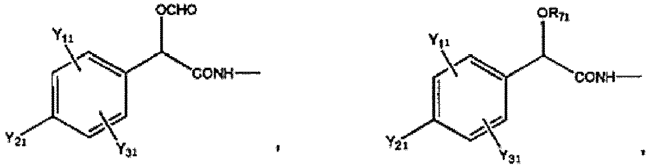 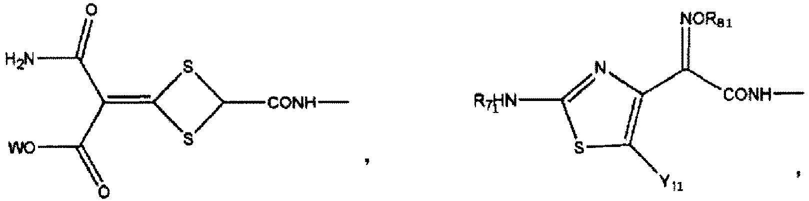 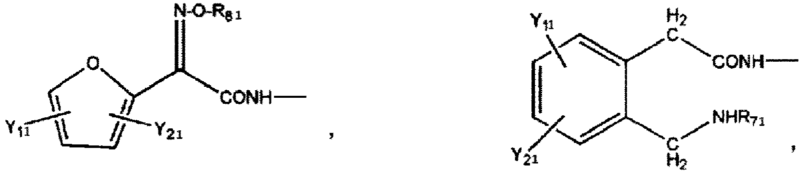 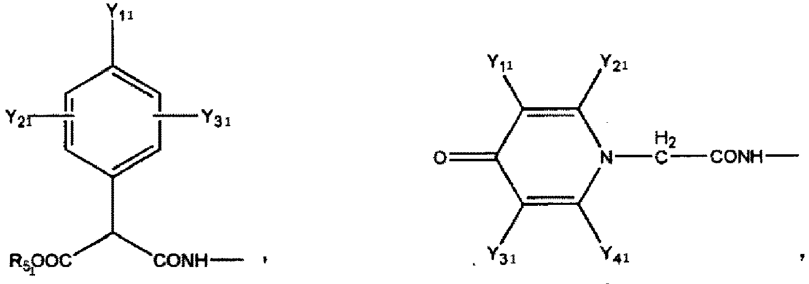 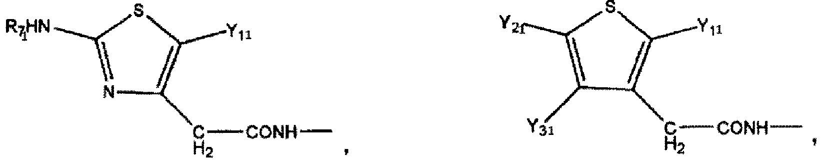 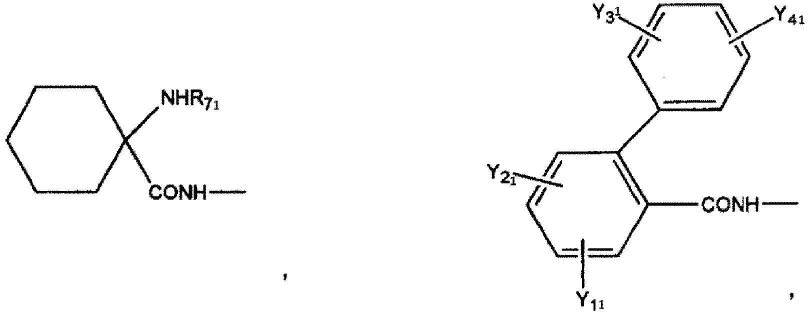 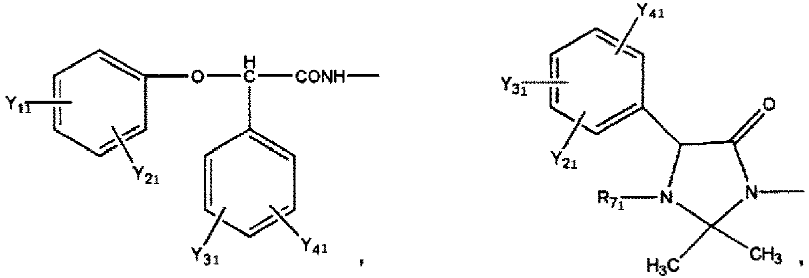 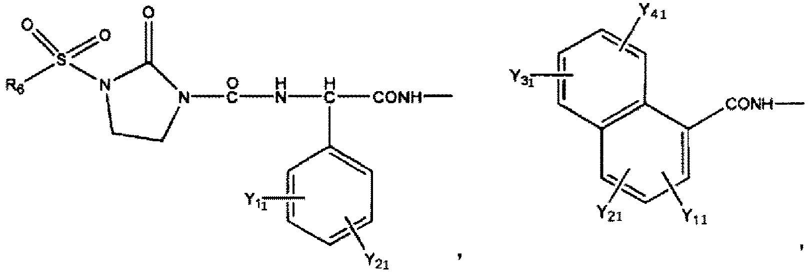 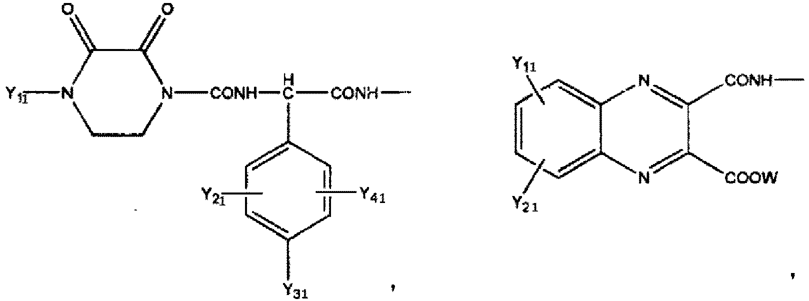 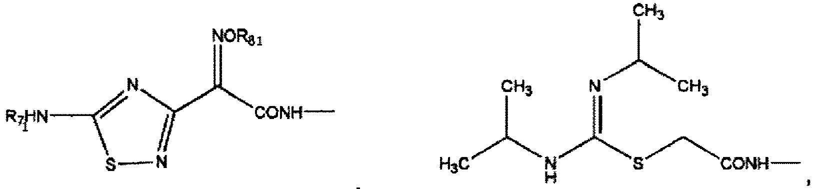 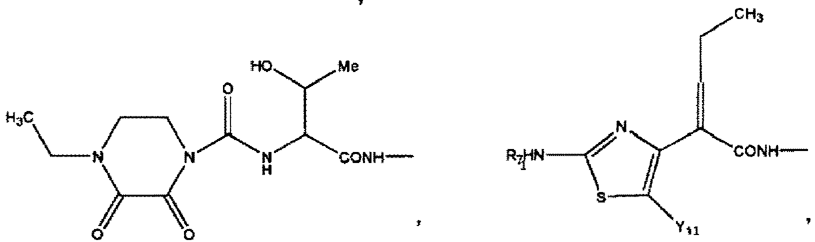 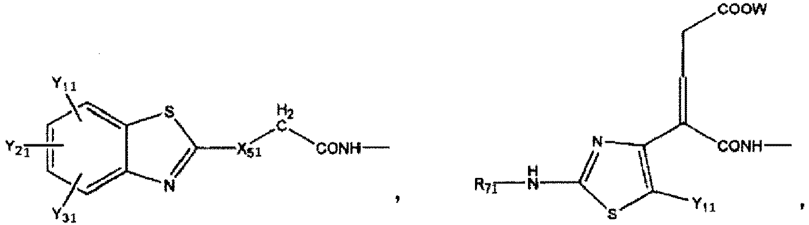 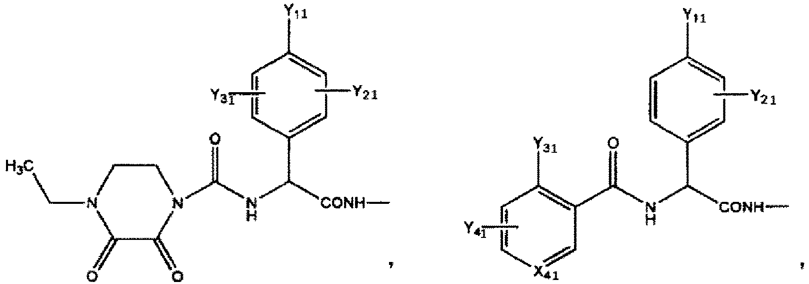 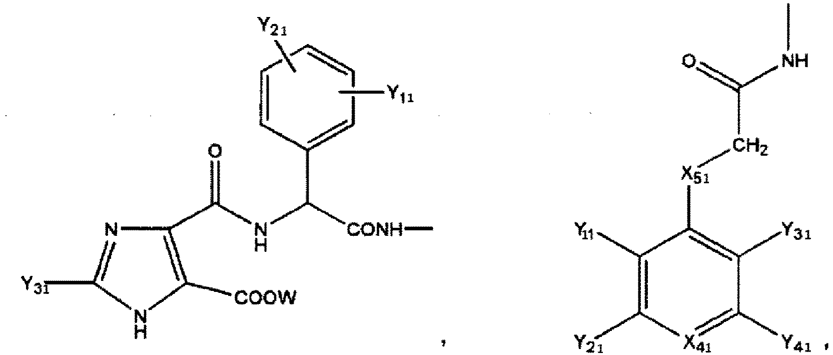 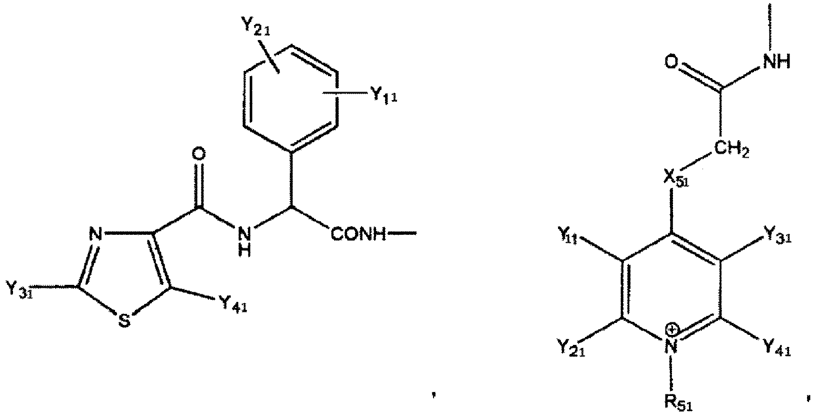 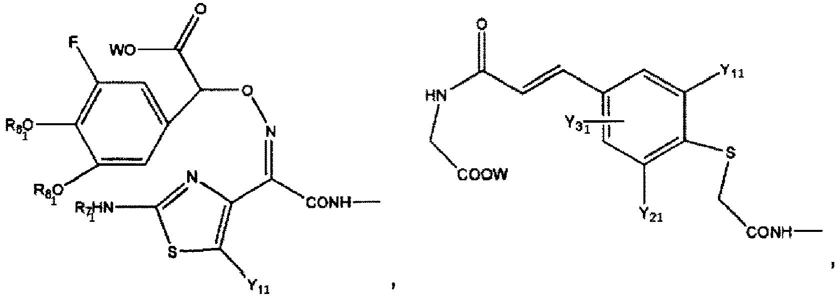 R₆₁OOCCH(NHR₇₁)(CH₂)ₙCONH-, R₆₁OOCCH(NHR₇)(CH₂)ₙSCONH-, CF₃SCH₂CONH-, CF₃CH₂CONH-, CHF₂SCH₂CONH-, CHF₂FSCH₂CONH-, NH₂COCHFSCH₂CONH-, R₇₁NHCH(COOW), CH₂SCH₂CONH-, CNCH₂SCH₂CONH-, CH₃(CH₂)ₙCONH-, R₇₁N=C(NR₇₁)CH₂CH₂S-, R₇₁N=C(NHR₇₁)NHCO-, CH₃C(Cl)=CHCHSCH₂CONH-, (CH₃)₂C(OR₆₁)-, CNCH₂CONH-, CNCH₂CH₂S-, R₇₁N=C(NR₇₁)CH₂CH₂S-, CH₂=CHCH₂SCH₂CONH-, CH₃CH(OH)-, CH₃CH(OR₈₁)-, CH₃CH(Y₁₁)-, (CH₃)₂CH-, CH₃CH₂-, und CH₃(CH₂)ₙCH=CH(CH₂)ₘCONH-, oder Rₛ und Y zusammen genommen R₆₁OCH₂C(R₅₁)= sind, wobei
n und m jeweils unabhängig ausgewählt sind aus 0, 1, 2, 3, 4, 5 und 6;
X₃₁ ausgewählt ist aus H, N₃, SO₃W, F, Cl, Br, I, OH, OCH₃, OC₂H₅, CH₃ und C₂H₅;
X₄₁ N oder CH ist;
X₅₁ ausgewählt ist aus CH₂, S, O und NH;
R₅₁ ausgewählt ist aus H, -CONH₂, CH₂CH₂N(CH₃)₂, CH₂CH₂N(CH₂CH₃)₂, CH₂CH₂OR₆₁, Cl,
F, Br, I Alkylreste mit 1 bis 12 Kohlenstoffatomen, Alkyloxylreste mit 1 bis 12 Kohlenstoffatomen, Alkenylreste mit bis zu 12 Kohlenstoffatomen, Alkynylreste mit bis zu 12 Kohlenstoffatomen, Aryreste und Heteroarylreste sind;
R₆₁ ausgewählt ist aus H, F, Cl, Br, I, 2-Oxo-1-imidazolidinyl, Alkylresten mit 1 bis 12 Kohlenstoffatomen, Alkyloxylresten mit 1 bis 12 Kohlenstoffatomen, Alkenylresten mit bis zu 12 Kohlenstoffatomen, Alkynylresten mit bis zu 12 Kohlenstoffatomen, Aryl(phenyl,5-indanyl,2,3- dihydro-1H-inden-5-yl)-Resten und Heteroaryl(4-hydroxy-1,5-naphthyridin-3-yl)-Resten;
R₇₁ ausgewählt ist aus H, F, Cl, Br, I, CH₃NHCOCH₂CH(NHR₈₁)CO, R₅₁N=C(NHR₆₁)NHCO-, COCH₃, COR₆₁, PO(OR₅₁)OR₆₁, Alkylresten mit 1 bis 12 Kohlenstoffatomen, Alkyloxylresten mit 1 bis 12 Kohlenstoffatomen, Alkenylresten mit 1 bis 12 Kohlenstoffatomen, Alkynylresten mit 1 bis 12 Kohlenstoffatomen, Arylresten und Heteroarylresten;
R₈₁ ausgewählt ist aus der Gruppe bestehend aus H, F, Cl, Br, I, CH₃, C₂H₅, CF₃, CH₂CH₂F, CH₂CH₂Cl, CH₂CH₂Br, CH₂CH₂I, CH₂CHF₂, CH₂CF₃, CH₂F, CH₂Cl, CH₂Br, CH₂I, C₃H₇, C₄H₉, C₅H₁₁, CONH₂ und CH₂OCONH₂;
Y₁₁, Y₂₁, Y₃₁ und Y₄₁ jeweils unabhängig ausgewählt sind aus H, OH, OW, OCOW, OCOCH₃, CH₃, C₂H₅, C₃H₇, C₄H₉, SO₃R₆₁, CH₂COOR₈₁, OCH₃, OC₂H₅, CH₃SO₃, NO₂, CN, CF₃, OCF₃, CH=CHCONHCH₂COOW, CH₂(CH₂)ₙNR₅₁R₆₁, CH₂(CH₂)ₙOR₆₁, CH(CONH₂)NHR₆₁, COOR₅₁, F, Br, I und Cl;
-W ausgewählt ist aus Alkylresten mit 1 bis 12 Kohlenstoffatomen, Alkyloxyresten mit 1 bis 12 Kohlenstoffatomen, Alkenylresten mit bis zu 12 Kohlenstoffatomen, Alkynylresten mit bis zu 12 Kohlenstoffatomen, Arylresten, Heteroarylresten, 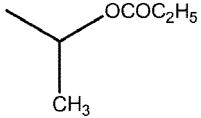 und 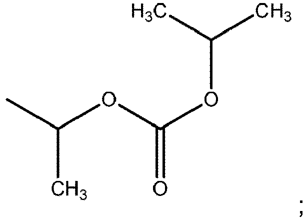
-W₁ -RN⁺R₁R₂HA ist;
R₁ und R₂ unabhängig ausgewählt sind aus Alkylresten mit 1 bis 12 Kohlenstoffatomen, Alkyloxylresten mit 1 bis 12 Kohlenstoffatomen, Alkenylresten mit bis zu 12 Kohlenstoffatomen und Alkynylresten mit bis zu 12 Kohlenstoffatomen;
R verzweigt- oder geradkettiges -(CH₂)ₙ₁- ist;
n1 ausgewählt ist aus 0, 1, 2, 3, 4, 5, 6, 7 und 8; und
A⁻ ein negatives Ion ist, das ausgewählt ist aus der Gruppe bestehend aus Chlorid, Bromid, Fluorid, Acetat und Citrat.

2. Verbindung nach Anspruch 1, die folgendes ist:
6-Phenoxyacetacetamidopenicillansäure 2-diethylaminoethylester.HCl;
Allylmercaptomethylpenicillansäure 2-diethylaminoethylester.HCl
6-[D(-)-α-Acetamidophenylacetamidopenicillansäure 2-diethylaminoethylester. HCl;
D-α-[(Imidazolidin-2-on-1-yl)carbonylamino]benzylpenicillan 2-diethylaminoethylester.HCl;
6-[3-(2,6-Dichlorphenyl)-5-methyl-4-isoxazolecarboxamido]penicillansäure 2-diethylaminoethylester.HCl;
6R-[2-[3-(Methylsulfony1)-2-oxo-1-imidazolidincarboxamido]-2-phenylacetamido]penicillansäure 2-diethylaminoethylester.HCl;
6-(5-Methyl-3-phenyl-2-isoxazolin-4-carboxamido)penicillansäure 2-diethylaminoethylester.HCl;
6-[3-(o-Chlorphenyl)-5-methyl-4-isoxazolecarboxamido]penicillansäure 2-diethylaminoethylester.HCl;
6-D(-)-α-(4-ethyl-2,3-dioxo-1-piperazinylcarbonylamino)-α-phenylacetamidopenicillansäure 2-diethylaminoethylester.HCl;
7-(2-Thienylacetamido)cephalosporansäure 2-diethylaminoethylester.HCl;
7-[(Hydroxyphenylacetyl)amino]-3-[[(1-methyl-1 H-tetrazol-5-yl)thio]methyl]-8-oxo-5-thia-1-azabicyclo[4.2.0]oct-2-en-2-carboxylsäure 2-diethylaminoethylester.HCl;
3-[[(Aminocarbonyl)oxy]methyl]-7-[[2-furanyl(methoxyimino)acetyl]amino]-8-oxo-5-thia-1-azabicyclo[4.2.0]oct-2-en-2-carboxylsäure 2-diethylaminoethylester.HCl;
3-[[(Aminocarbonyl)oxy]methyl]-7-methoxy-8-oxo-7-[(2-thienylacetyl)amino]-5-thia-1-azabicyclo[4.2.0]oct-2-en-2-carboxylsäure 2-diethylaminoethylester.HCl;
7-[[[2-(Acetylaminomethyl)phenyl]acetyl]amino]-3-[[[1-(ethoxylcarbonylmethyl)-1H-tetrazol-5-yl]thio]methyl]-8-oxo-5-thia-1-azabicyclo[4.2.0]oct-2-en-2-carboxylsäure 2-diethylaminoethylester.HCl;
7-[(Acetylaminophenylacetyl)amino]-3-chlor-8-oxo-5-thia-1-azabicyclo[4.2.0]oct-2-en-2-carboxylsäure 2-diethylaminoethylester.HCl;
3-[(Acetyloxy)methyl]-7-[[(2-acetylamino-4-thiazolyl)(methoxyimino)acetyl]amino]-8-oxo-5-thia-1-azabicyclo[4.2.0]oct-2-en-2-carboxylsäure 2-diethylaminoethylester.HCl;
7-[[(2-Acetylamino-4-thiazolyl)(methoxyimino)acetyl]amino]-8-oxo-5-thia-1-azabicyclo[4.2.0]oct-2-en-2-carboxylsäure 2-diethylaminoethylester.HCl;
7-[[[[(4-Ethyl-2,3-dioxo-1-piperazinyl)carbonyl]amino](4-acetoxyphenyl)acetyl]amino]-3-[[(1-methyl-1H-tetrazol-5-yl)thio]methyl]-8-oxo-5-thia-1-azabicyclo[4.2.0]oct-2-en-2-carboxylsäure 2-diethylaminoethylester.HCl;
7-[2-(2-Acetylamino-4-thiazolyl)-2-((Z)-methoxyimino)acetamido]-3-(methoxymethyl)-8-oxo-5-thia-1-azabicyclo[4.2.0]oct-2-en-2-carboxylsäure 2-diethylaminoethylester.HCl;
7-[2-(2-Acetylamino-4-thiazolyl)-2-((Z)-ethoxycarbonylmethoxy)imino]acetamido]-3-(vinyl)-8-oxo-5-thia-1-azabicyclo[4.2.0]oct-2-en-2-carboxylsäure 2-diethylaminoethylester.HCl.

3. Zusammensetzung, die eine oder mehrere Verbindungen nach Anspruch 1 oder 2 umfasst.

4. Verbindung nach Anspruch 1 oder 2 oder Zusammensetzung nach Anspruch 3 zur Verwendung in der Behandlung eines beliebigen mit Beta-Laktam-Antibiotika behandelbaren Zustands in einem Menschen oder Tier, wobei die Verbindung oder Zusammensetzung mittels transdermale Verabreichung verabreicht wird.

5. Verbindung oder Zusammensetzung zur Verwendung nach Anspruch 4, wobei der mit Beta-Laktam-Antibiotika behandelbare Zustand ausgewählt ist aus Infektionen des oberen und unteren Atemtrakts, akutem Atemnotsyndrom, Pneumonie, Meningitis, septischem Schock, Sepsis, Mittelohrentzündung und Nasennebenhöhlenentzündung, Meningokokkenmeningitis und Pneumokokkensepsis, Endokarditis durch Streptokokken, Unterleibsentzündung, Gonorrhö, Syphilis, Lyme-Krankheit, Gasbrand, Tetanus, der Haut und betroffenem Weichgewebe, des Magen-Darm-Trakts, der Harnwege, Knochen und Gelenke sowie Sepsis und Endokarditis und intraabdominale Infektionen und Infektionen des Gallengangs.

6. Verbindung oder Zusammensetzung zur Verwendung nach Anspruch 4, wobei die Verbindung oder Zusammensetzung in Form einer Lösung, eines Sprays, einer Lotion, einer Salbe, einer Emulsion oder eines Gels gegeben ist.

7. Verbindung oder Zusammensetzung zur Verwendung nach Anspruch 4, wobei die Verbindung oder Zusammensetzung in oder nahe eines entzündeten Bereichs transdermal verabreicht wird an die Haut, das Gehirn, die Brist, die Prostata, den oberen und unteren Atemtrakt, die Harnwege, Knochen und Gelenke oder den Genitalbereich.

8. Transdermales therapeutisches Applikationssystem, umfassend eine Verbindung nach Anspruch 1 oder 2 oder eine Zusammensetzung nach Anspruch 3 zur Verwendung in der Behandlung beliebigen mit Beta-Laktam-Antibiotika behandelbaren Zustands in einem Menschen oder Tier.

9. Transdermales therapeutisches Applikationssystem zur Verwendung nach Anspruch 8, **dadurch gekennzeichnet, dass** das System eine Bandage oder ein Pflaster ist, das eine Matrixschicht umfasst, die einen Wirkstoff enthält, und eine undurchlässige Trägerschicht.

10. Transdermales therapeutisches Applikationssystem zur Verwendung nach Anspruch 8 oder 9, **dadurch gekennzeichnet, dass** es einen Wirkstoffspeicher aufweist, der eine durchlässige Unterseite aufweist, die zu der Haut ausgerichtet ist.

11. Transdermales therapeutisches Applikationssystem zur Verwendung nach einem der Ansprüche 8 bis 10, **gekennzeichnet durch** ein Regelungsmittel, welches die Freisetzungsrate regelt.

## Revendications

1. Composé de Structure 1 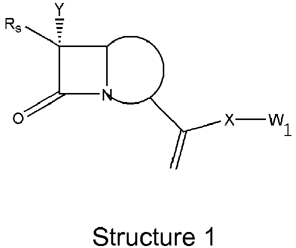 dans laquelle
X est choisi parmi O, S, CH₂ et NH;
Y est choisi parmi H, OH, NHCHO, OCOCH₃, OCH₃, OC₂H₅, CH₃SO₃, NO₂, CN, CF₃, OCF₃, F, Br, I, et Cl ; 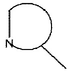
représente 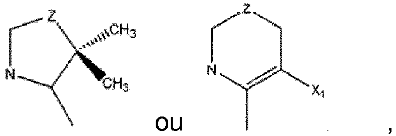 dans laquelle
Z représente S ;
-X₁ est choisi parmi -H, -OH, -OCH₃, -OC₂H₅, OC₃H₇, -CONH₂,-CH₂OCONH₂, -CH₂ OCOCH₃, -OCOCH₃, -CH₂OCOCH₃, -CH₂OCH₃, -CH₃,-C₂H₅, -C₃H₇, -C₄H₉, C₅H 11, C₆H₁₃, -Cl, -F, -Br, -I, -HC=CHCH₃, -HC=CH₂,-CH₂OCH₃, 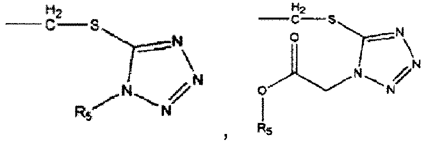
dans laquelle
R₅ est choisi parmi H, CONH₂, CH₂CH₂N(CH₃)₂, CH₂CH₂N(CH₃CH₃)₂, CI, F, Br, I, des résidus alkyle possédant entre 1 et 12 atomes de carbone, des résidus alkyloxy possédant entre 1 et 12 atomes de carbone, des résidus alcényle possédant jusqu'à 12 atomes de carbone, et des résidus alcynyle possédant jusqu'à 12 atomes de carbone ;
Rₛ₋ est choisi parmi : 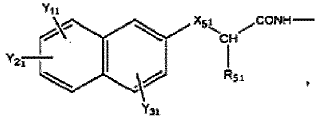 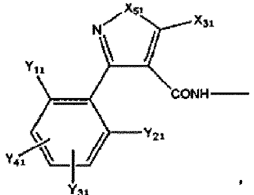 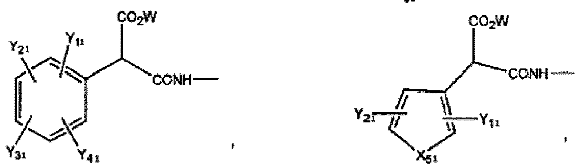 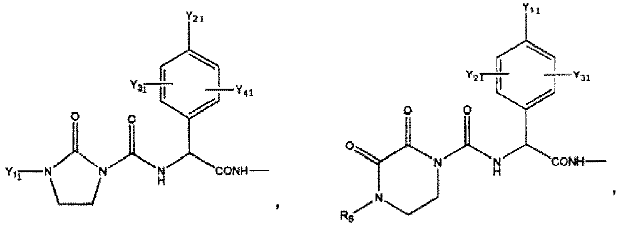 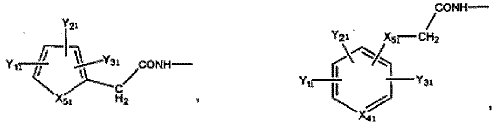 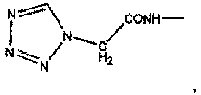 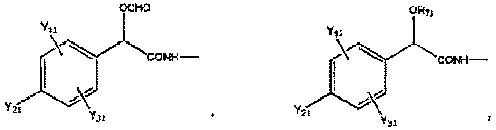 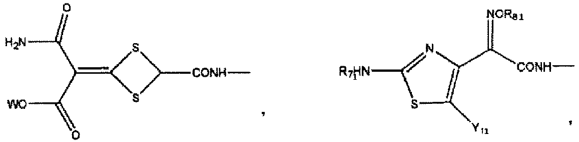 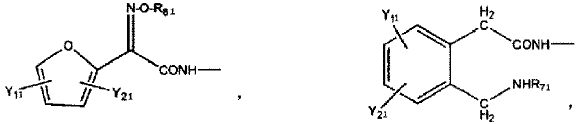 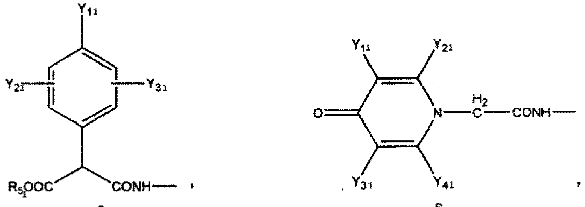 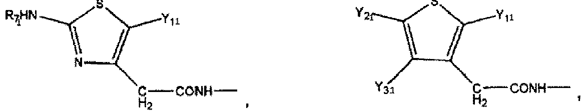 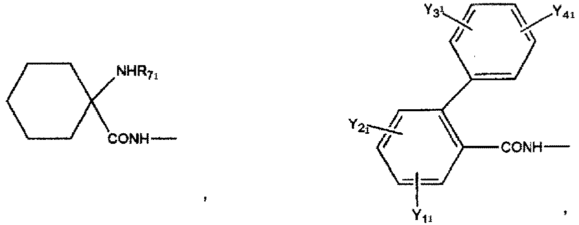 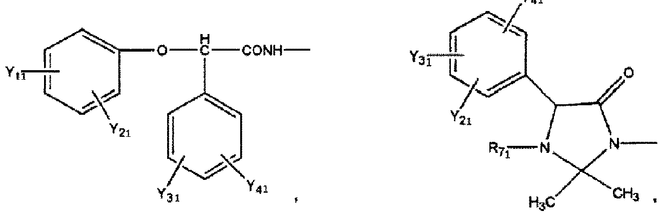 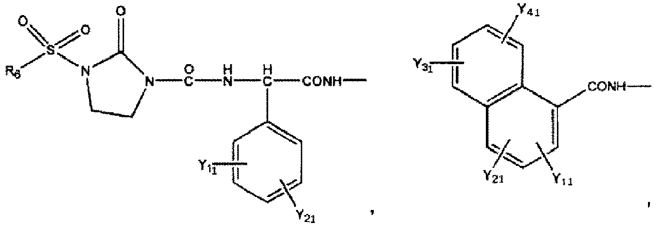 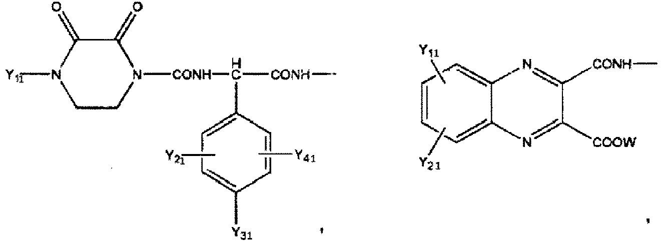 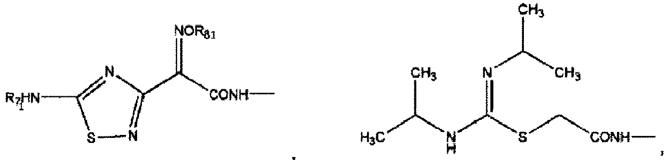 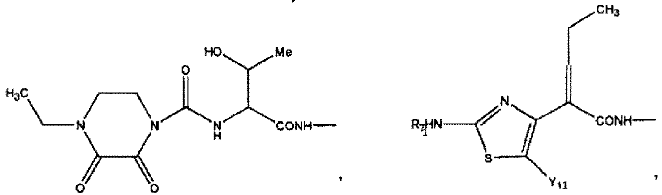 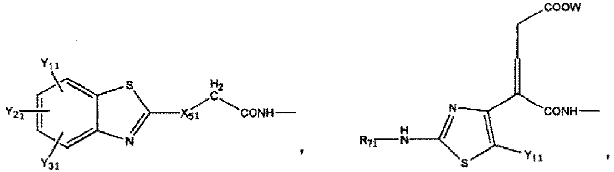 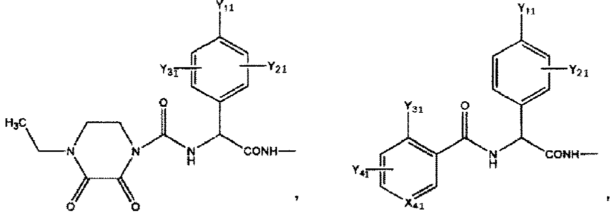 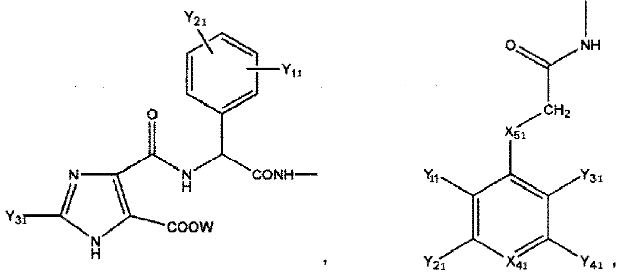 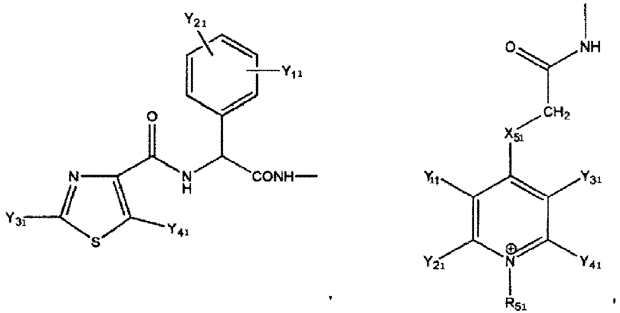 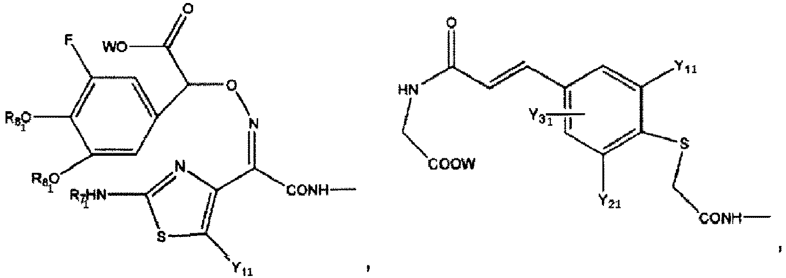 R₆₁OOCCH(NHR₇₁)(CH₂)ₙCONH-, R₆₁OOCCH(NHR₇)(CH₂)ₙSCONH-, CF₃SCH₂CONH-, CF₃CH₂CONH-, CHF₂SCH₂CONH-, CH₂FSCH₂CONH-, NH₂COCHFSCH₂CONH-, R₇₁NHCH(COOW), CH₂SCH₂CONH-, CNCH₂SCH₂CONH-, CH₃(CH₂)ₙCONH-, R₇₁N=C(NR₇₁)CH₂CH₂S-, R₇₁N=C(NHR₇₁)NHCO-, CH₃C(Cl)=CHCH₂SCH₂CONH-, (CH₃)₂C(OR₆₁)-, CNCH₂CONH-, CNCH₂CH₂S-, R₇₁N=C(NR₇₁)CH₂CH₂S-, CH₂=CHCH₂SCH₂CONH-, CH₃CH(OH)-, CH₃CH(OR₈₁)-, CH₃CH(Y₁₁)-, (CH₃)₂CH-, CH₃CH₂-, et CH₃(CH₂)ₙCH=CH(CH₂)ₘCONH-, ou Rₛ et Y pris ensemble représentent R₆₁OCH₂C(R₅₁)=, dans laquelle
n et m sont chacun indépendamment choisis parmi 0, 1, 2, 3, 4, 5, et 6
X₃₁ est choisi parmi H, N₃, SO₃W, F, CI, Br, I, OH, OCH₃, OC₂H₅, CH₃, et C₂H₅ ;
X₄₁ représente N ou CH ;
X₅₁ est choisi parmi CH₂, S, O et NH ;
R₅₁ est choisi parmi H, -CONH₂, CH₂CH₂N(CH₃)₂, CH₂CH₂N(CH₂CH₃)₂, CH₂CH₂OR₆₁, CI, F, Br, I, des résidus alkyle possédant entre 1 et 12 atomes de carbone, des résidus alkyloxy possédant entre 1 et 12 atomes de carbone, des résidus alcényle possédant jusqu'à 12 atomes de carbone, des résidus alcynyle possédant jusqu'à 12 atomes de carbone, des résidus aryle, et des résidus hétéroaryle ;
R₆₁ est choisi parmi H, F, CI, Br, I, 2-oxo-1-imidazodinyle, des résidus alkyle possédant entre 1 et 12 atomes de carbone, des résidus alkyloxy possédant entre 1 et 12 atomes de carbone, des résidus alcényle possédant jusqu'à 12 atomes de carbone, des résidus alcynyle possédant jusqu'à 12 atomes de carbone, des résidus aryl (phényl, 5-indanyl, 2,3-dihydro-1H-indèn-5-yl) et des résidus hétéroaryl (4-hydroxy-1,5-naphtyridin-3-yl) ;
R₇₁ est choisi parmi H, F, CI, Br, I, CH₃NHCOCH₂CH(NHR₈₁)CO, R₅₁N=C(NHR₆₁)NHCO-, COCH₃, COR₆₁, PO(OR₅₁)OR₆₁, des résidus alkyle possédant entre 1 et 12 atomes de carbone, des résidus alkyloxy possédant entre 1 et 12 atomes de carbone, des résidus alcényle possédant jusqu'à 12 atomes de carbone, des résidus alcynyle possédant jusqu'à 12 atomes de carbone, des résidus aryle, et des résidus hétéroaryle ;
R₈₁ est choisi parmi H, F, CI, Br, I, CH₃, C₂H₅, CF₃, CH₂CH₂F, CH₂CH₂Cl, CH₂CH₂Br, CH₂CH₂I, CH₂CHF₂, CH₂CF₃, CH₂F, CH₂Cl, CH₂Br, CH₂I, C₃H₇, C₄H₉, C₅H₁₁, CONH₂, et CH₂ OCONH₂ ;
Y₁₁, Y₂₁, Y₃₁ et Y₄₁ sont indépendamment choisis parmi H, OH, OW, OCOW, OCOCH₃, CH₃, C₂H₅, C₃H₇, C₄H₉, SO₃R₆₁, CH₂COOR₈₁, OCH₃, OC₂H₅, CH₃SO₃, NO₂, CN, CF₃, OCF₃, CH=CHCONHCH₂COOW, CH₂(CH₂)ₙNR₅₁R₆₁, CH₂(CH₂)ₙOR₆₁, CH(CONH₂)NHR₆₁, COOR₅₁, F, Br, I, et Cl ;
-W est choisi parmi des résidus alkyle possédant entre 1 et 12 atomes de carbone, des résidus alkyloxy possédant entre 1 et 12 atomes de carbone, des résidus alcynyle possédant jusqu'à 12 atomes de carbone, des résidus aryle, des résidus hétéroaryle, 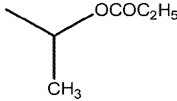 et 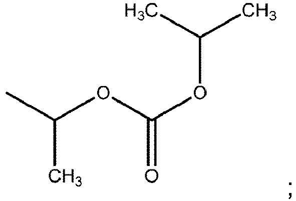
-W₁ représente -RN⁺R₁R₂HA ;
R1 et R2 sont indépendamment choisis parmi des résidus alkyle possédant entre 1 et 12 atomes de carbone, des résidus alkyloxy possédant entre 1 et 12 atomes de carbone, des résidus alcényle possédant jusqu'à 12 atomes de carbone, et des résidus alcynyle possédant jusqu'à 12 atomes de carbone ;
R représente un -(CH2)ₙ₁₋ ramifié ou à chaine linéaire ;
n est choisi parmi 0, 1, 2, 3, 45, 6, 7, et 8 ; et
A⁻ représente un ion négatif choisi parmi le groupe constitué des chlorure, bromure, fluorure, acétate, et citrate.

2. Compose selon la revendication 1, qui est :
acide 6-phénoxyacétacétamidopénicillanique de l'hydrochlorure d'ester de 2-diéthylaminoéthyle ;
acide allylmercaptométhylpénicillinique de l'hydrochlorure d'ester de 2-diéthylaminoéthyle ;
acide 6-[D(-)-α-acétamidophénylacétamidopénicillinique de l'hydrochlorure d'ester de 2-diéthylaminoéthyle ;
D-α-[(imidazolidin-2-on-1-yl)carbonylamino]benzylpénicilline de l'hydrochlorure d'ester de 2-diéthylaminoéthyle ;
acide 6-[3-(2,6-dichlorophényl)-5-méthyl-4-isoazolecarboxamido]pénicillinique de l'hydrochlorure d'ester de 2-diéthylaminoéthyle ;
acide 6R-[2-[3-(méthylsulfonyl)-2-oxo-1-imidazolidinecarboxamido]-2-phénylacétamido]pénicillinique de l'hydrochlorure d'ester de 2-diéthylaminoéthyle
acide 6-(5-méthyl-3-phényl-2-isoxazoline-4-carboxamido)pénicillinique de l'hydrochlorure d'ester de 2-diéthylaminoéthyle ;
acide 6-[3-(o-chlorophényl)-5-méthyl-4-isoxazolecarboxiamido]pénicillinique de l'hydrochlorure d'ester de 2-diéthylaminoéthyle ;
acide 6-D(-)-α-(4-éthyl-2,3-dioxo-1-pipérazinylcarbonylamino)-α-phénylacétamidopénicillinique de l'hydrochlorure d'ester de 2-diéthylaminoéthyle ;
acide 7-(2-thiènylacétamido)céphalosporanique de l'hydrochlorure d'ester de 2-diéthylaminoéthyle ;
acide 7-[(hydroxyphénylacétyl)amino]-3-[[(1-méthyl-1H-tétrazol-5-yl)thio]méthyl]-8-oxo-5-thia-1-azabicyclo[4.2.0]oct-2-ène-2-carboxylique de l'hydrochlorure d'ester de 2-diéthylaminoéthyle ;
acide 3-[[(aminocarbonyl)oxy]méthyl]-7-[[2-furanyl(méthoxyimino)acétyl]amino]-8-oxo-5-thia-1-azabicyclo[4.2.0]oct-2-ène-2-carboxylique de l'hydrochlorure d'ester de 2-diéthylaminoéthyle ;
acide 3-[[(aminocarbonyl)oxy]méthyl]-7-méthoxy-8-oxo-7-[(2-thiènylacétyl)amino]-5-thia-1-azabicyclo[4.2.0]oct-2-ène-2-carboxylique de l'hydrochlorure d'ester de 2-diéthylaminoéthyle ;
acide 7-[[[2-(acétylaminométhyl)phényl]acétyl]amino]-3-[[[1-(éthoxylcarbonylméthyl)-1H-tétrazol-5-yl]thio]méthyl]-8-oxo-5-thia-1-azabicyclo[4.2.0]oct-2-ène-2-carboxylique de l'hydrochlorure d'ester de 2-diéthylaminoéthyle ;
acide 7-[(acétylaminophénylacétyl)amino]-3-chloro-8-oxo-5-thia-1-azabicyclo[4.2.0]oct-2-ène-2-carboxylique de l'hydrochlorure d'ester de 2-diéthylaminoéthyle ;
acide 3-[(acétyloxy)méthyl]-7-[[(2-acétylamino-4-thiazolyl)(méthoxyimino)acétyl]amino]-8-oxo-5-thia-1-azabicyclo[4.2.0]oct-2-ène-2-carboxylique de l'hydrochlorure d'ester de 2-diéthylaminoéthyle ;
acide 7-[[(2-acétylamino-4-thiazolyl)(méthoxyimino)acétyl]amino]-8-oxo-5-thia-1-azabicyclo[4.2.0]oct-2-ène-2-carboxylique de l'hydrochlorure d'ester de 2-diéthylaminoéthyle ;
acide 7-[[[[(4-éthyl-2,3-dioxo-1-pipérazinyl)carbonyl]amino](4-acétoxyphényl)acétyl]amino]-3-[[(1-méthyl-1 H-tétrazol-5-yl)thio]méthyl]-8-oxo-5-thia-1-azabicyclo[4.2.0]oct-2-ène-2-carboxyliqe de l'hydrochlorure d'ester de 2-diéthylaminoéthyle ;
acide 7-[2-(2-acétylamino-4-thiazolyl)-2-((Z)-méthoxyimino)acétamido]-3-(méthoxyméthyl)-8-oxo-5-thia-1-azabicyclo[4.2.0]oct-2-ène-2-carboxylique de l'hydrochlorure d'ester de 2-diéthylaminoéthyle ;
acide 7-[2-(2-acétylamino-4-thiazolyl)-2-((Z)-éthoxycarbonylméthoxy)imino]acétamido]-3-(vinyl)-8-oxo-5-thia-1-azabicyclo[4.2.0]oct-2-ène-2-carboxylique de l'hydrochlorure d'ester de 2-diéthylaminoéthyle.

3. Composition comprenant un ou plusieurs composés selon la revenidcation1 ou la revendication 2.

4. Composé selon la revendication 1 ou la revendication 2 ou la composition selon la revendication 3 pour une utilisation dans le traitement de n'importe quelle condition que l'on peut traiter par des antibiotiques de type béta-lactames chez les humains ou les animaux, dans lequel le compose ou la composition est administré par administration transdermique.

5. Composé selon la revendication 1 ou la revendication 2 ou la composition selon la revendication 3 pour une utilisation dans le traitement de n'importe quelle condition que l'on peut traiter par des antibiotiques de type béta-lactames chez les humains ou les animaux y compris, mais pas seulement, des infections du tractus respiratoire inférieur et supérieur, la détresse respiratoire aigüe, la pneumonie, la méningite, le choc septique, la septicémie, l'otite moyenne et la sinusite, la méningite à méningocoque et la septicémie pneumococcique, l'endocardite streptococcique, la maladie inflammatoire pelvienne, la gonorrhée, la syphilis, la maladie de Lyme, la gangrène gazeuse, le tétanos, la peau et les tissus mous liés, le tractus gastro-intestinal, le tractus urinaire, les os et les joints, ainsi que les septicémies et l'endocardite et les infections intra-abdominales et du tractus biliaire.

6. Composé ou composition selon la revendication 4, dans lequel le composé ou la composition est dans la forme d'une solution, d'un spray, d'une lotion, d'un onguent, d'une émulsion, ou d'un gel.

7. Composé ou composition selon la revendication 4, dans lequel le composé ou la composition est administré de manière transdermique à la peau, au cerveau, aux seins, à la glande de la prostate, au tractus respiratoire supérieur et inférieur, au tractus urinaire, aux os et joints, à la zone génitale, ou à ou proche d'une zone qui subit une inflammation.

8. Système d'application thérapeutique transdermique comprenant un composé selon la revendication 1 ou la revendication 2 ou une composition selon la revendication 3 pour une utilisation dans le traitement de n'importe quelle condition que l'on peut traiter par des antibiotiques de type béta-lactames chez les humains ou les animaux.

9. Système d'application thérapeutique transdermique pour une utilisation selon la revendication 8, **caractérisé en ce que** le système est un pansement ou un patch comprenant une couche matricielle contenant une substance active et une couche de support imperméable.

10. Système d'application thérapeutique transdermique pour une utilisation selon la revendication 8 ou la revendication 9, **caractérisé en ce qu'**il possède un réservoir de substances actives, qui possède un fond perméable faisant face à la peau.

11. Système d'application thérapeutique transdermique pour une utilisation selon les revendications 8 à 10, **caractérisé par** un moyen de commande commandant le taux de libération.
